(19) Europäisches Patentamt / European Patent Office / Office européen des brevets

(11) EP 3 822 260 A1

(12) EUROPEAN PATENT APPLICATION
published in accordance with Art. 153(4) EPC

(43) Date of publication:
**19.05.2021 Bulletin 2021/20**

(21) Application number: **19833589.5**

(22) Date of filing: **02.07.2019**

(51) Int Cl.:
*C07D 305/00* (2006.01) *C08G 59/02* (2006.01)
*C07D 305/06* (2006.01) *C08L 33/08* (2006.01)

(86) International application number:
**PCT/CN2019/094344**

(87) International publication number:
**WO 2020/011062 (16.01.2020 Gazette 2020/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.07.2018 CN 201810765272**
**12.07.2018 CN 201810765950**

(71) Applicants:
- **Changzhou Tronly New Electronic Materials Co., Ltd.**
  **Jiangsu 213011 (CN)**
- **Changzhou Tronly Advanced Electronic Materials Co., Ltd.**
  **Changzhou, Jiangsu 213159 (CN)**

(72) Inventor: **QIAN, Xiaochun**
**Changzhou, Jiangsu 213011 (CN)**

(74) Representative: **Manuel Illescas y Asociados, S.L.**
**C/ Príncipe de Vergara 197, Oficina 1°A**
**28002 Madrid (ES)**

# (54) EPOXY MODIFIED ACRYLIC RESIN AND PREPARATION METHOD THEREFOR, AND ENERGY-CURABLE COMPOSITION CONTAINING EPOXY MODIFIED ACRYLIC RESIN AND APPLICATION

(57) Provided are an epoxy modified acrylic resin and a preparation method therefor, an energy-curable composition containing epoxy modified acrylic resin and application. A branched chain of at least one repeat unit in the acrylic resin is grafted with one or more oxetane groups, and each oxetane group separately has the structure as represented by general formula (I), (II), or (III). After the branched chain of the acrylic resin is covalently grafted with the oxetane group, the epoxy modified acrylic resin is obtained. A coating prepared by using the composition containing the epoxy modified acrylic resin has good flexibility, as well as high adhesion and a great curing film forming speed for a coated substrate. The covalent grafting reaction does not affect the main chain structure of an original acrylic resin, and thus during downstream application, it is not necessary to change original devices and production processes, so that the investment costs are low.

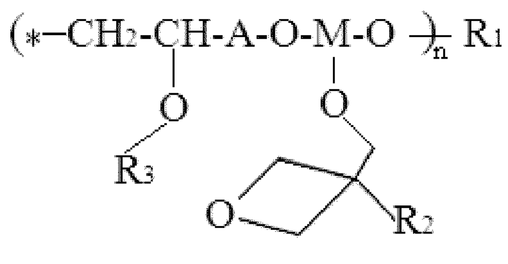
(I),

EP 3 822 260 A1

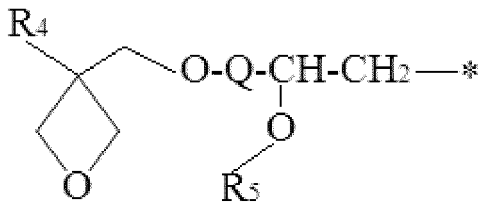

(II),

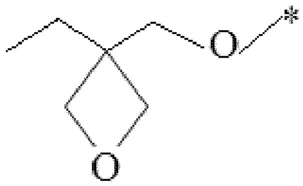

(III).

## Description

TECHNICAL FIELD

The present invention relates to the field of radiation curing, specifically relates to an epoxy-modified acrylic resin and preparation method thereof, an energy-curable composition containing the epoxy-modified acrylic resin and application.

BACKGROUND

Currently, acrylic resins are widely used in paint, ink, and adhesive industries and the like fields, and have been continuously developed and improved. However, with the increase of the amount of acrylic resin, there are more requirements for the performances of acrylic resins, such as no pretreatment on special substrates such as galvanized sheets, magnesium aluminum alloys, and glass, good adhesion provided by paints prepared from acrylic resins, as well as good hardness and scratch resistance of paints when applying acrylic resins in car paints.

It is difficult for ordinary acrylic resins to meet the above requirements. Currently, epoxy resin modified acrylic resins are commonly used in the industry to replace ordinary acrylic resins, in order to improve the weather resistance, chemical resistance, high hardness, high toughness, and the like. There are two methods for modifying the acrylic resin by epoxy resin. One is to use a physical blending method; however, due to the poor compatibility between them, the modification effect of the product obtained by this method is not obvious. The other is to modify by covalent bond grafting. The existing literatures adopt the second method, to prepare an epoxy-modified acrylic resin by introducing an ethylene oxide functional group into the side chain. Although the epoxy-modified acrylic resin has relatively high adhesion, yellowing resistance, as well as good temperature and weather resistances, its hardness and adhesion properties cannot meet the requirements of premium car paint and premium glass baking paint. Thus, its performances need to be further improved.

SUMMARY

A main object of the present invention is to provide an epoxy-modified acrylic resin and preparation method thereof, an energy-curable composition containing epoxy-modified acrylic resin and application, to solve the existing problem of the epoxy-modified acrylic resin such as poor hardness and adhesion during use.

In order to achieve the above object, an epoxy-modified acrylic resin is provided according to one aspect of the present invention, wherein the branched chain of at least one repeat unit in the acrylic resin is grafted with one or more oxetanyl groups, each of the oxetanyl groups has a structure represented by general formula (I), general formula (II), or general formula (III), respectively:

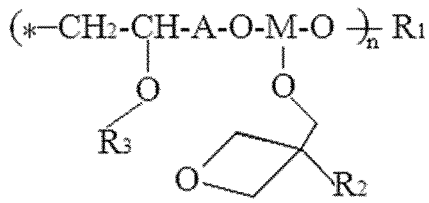

(I),

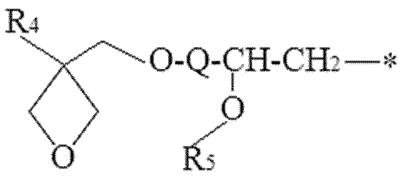

(II),

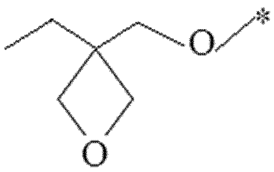

(III),

wherein $R_1$ represents $C_1$-$C_{40}$ linear or branched n-valent alkyl, $C_2$-$C_{30}$ n-valent alkenyl, or $C_6$-$C_{40}$ n-valent aryl, any one of $-CH_2-$ in $R_1$ may be substituted by oxygen atom, ester group, or

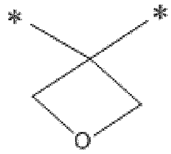

,

any one of hydrogen atoms in $R_1$ may be substituted by alkyl, halogen, or nitro, and n is selected from an integer of 1 to 12;

$R_2$ and $R_4$ independently represent hydrogen, halogen, nitro, $C_1$-$C_{30}$ linear or branched alkyl, $C_3$-$C_{30}$ cycloalkyl or substituted cycloalkyl, $C_2$-$C_{15}$ alkenyl, or $C_6$-$C_{30}$ aryl, respectively, any one of $-CH_2-$ in $R_2$ and $R_4$ may be substituted by oxygen atom or -COO-, and any one of hydrogen atoms in $R_2$ and $R_4$ may be substituted by alkyl, halogen, or nitro;

$R_3$ and $R_5$ independently represent $C_1$-$C_{30}$ linear or branched alkyl, $C_3$-$C_{30}$ cycloalkyl or substituted cycloalkyl, or $C_6$-$C_{30}$ aryl, respectively, any one of $-CH_2-$ in $R_3$ and $R_5$ may be substituted by oxygen atom or -COO-, and any one of hydrogen atoms in $R_3$ and $R_5$ may be substituted by alkyl, halogen, or nitro;

A represents $C_1$-$C_{20}$ linear or branched alkylene, any one of $-CH_2-$ in A may be substituted by oxygen atom or -COO-, and any one of hydrogen atoms in A may be substituted by alkyl, halogen, or nitro;

M represents 3-valent $C_1$-$C_{20}$ linear or branched alkyl, wherein any one of $-CH_2-$ in M may be substituted by oxygen atom, -COO-, or

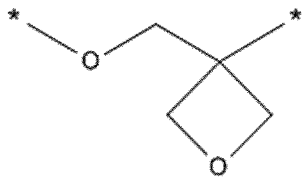

,

and any one of hydrogen atoms in M may be substituted by alkyl, halogen, or nitro;

Q represents $C_1$-$C_{20}$ linear or branched alkylene, wherein any one of $-CH_2-$ in Q may be substituted by oxygen atom, -COO-, or

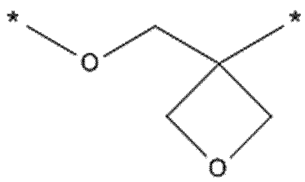

,

and any one of hydrogen atoms in Q may be substituted by alkyl, halogen, or nitro.

In order to achieve the above object, a preparation method of the above epoxy-modified acrylic resin is provided according to one aspect of the present invention, the epoxy-modified acrylic resin is prepared by any one of the following processes:

Process 1: using an acrylic resin comprising the repeat unit represented by formula (IV) as a first starting material,

and using a compound containing oxetanyl and epoxyethyl represented by formula (V) or (VI) as a second starting material, the preparation process including:

subjecting the carboxyl group of the first starting material and the second starting material to an epoxyethyl ring-opening reaction, to obtain a first intermediate product containing hydroxyl group; and reacting the hydroxyl of the first intermediate product with a first end-capping compound, to obtain an epoxy-modified acrylic resin, wherein the first end-capping compound is selected from anhydrides, acyl halides, compounds containing epoxyethyl, compounds containing oxetanyl, or compounds containing a double bond, and the formula (IV), the formula (V), and the formula (VI) are respectively as follows:

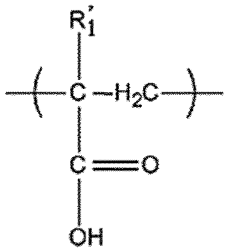

(IV)

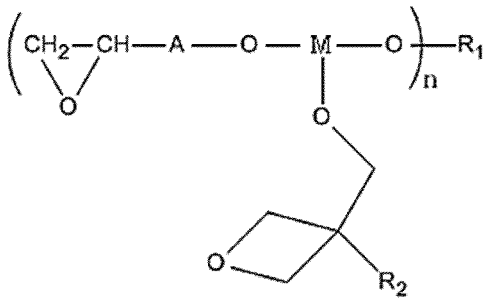

(V)

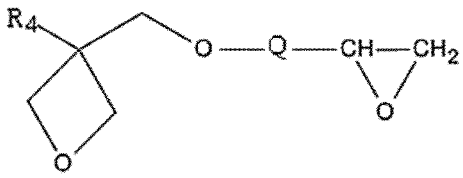

(VI)

wherein $R_1$, $R_2$, $R_4$, A, M, Q, and n have the same meanings as general formula (I) and general formula (II), and $R_1$' is selected from hydrogen or methyl;

Process 2: using an acrylic resin comprising the repeat unit represented by formula (VII) as a third starting material, and using a compound containing oxetanyl and epoxyethyl represented by formula (V) or formula (VI) as a second starting material, the preparation process including:

subjecting the hydroxyl group of the third starting material and the second starting material to an epoxyethyl ring-opening reaction, to obtain a second intermediate product containing hydroxyl group; and reacting the hydroxyl group of the second intermediate product with a second end-capping compound, to obtain an epoxy-modified acrylic resin, wherein the second end-capping compound is selected from anhydrides, acyl halides, compounds containing epoxyethyl, compounds containing oxetanyl, or compounds containing a double bond, the formula (VII) is

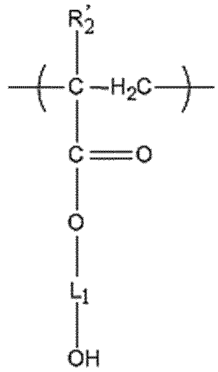

,

$R_2$' is selected from hydrogen or methyl, $L_1$ is selected from $C_2$-$C_{25}$ linear or branched alkylene, and $-CH_2-$ in $L_1$ may be substituted by oxygen atom or ester group; preferably, $L_1$ is selected from $C_2$-$C_{10}$ linear or branched alkylene, and $-CH_2-$ in $L_1$ may be substituted by oxygen atom or ester group;

Process 3: using an olefinic monomer represented by formula (VIII) as a fourth starting material, and using a compound containing oxetanyl and epoxyethyl represented by formula (V) or formula (VI) as a second starting material, the preparation process including:

subjecting the carboxyl group of the fourth starting material and the second starting material to an epoxyethyl ring-opening reaction, to obtain a third intermediate product containing hydroxyl group; reacting the hydroxyl group of the third intermediate product with a third end-capping compound, to obtain a third end-capped product; and copolymerizing the third end-capped product with a first olefinic monomer, to obtain the epoxy-modified acrylic resin; wherein the third end-capping compound is selected from anhydrides, acyl halides, compounds containing epoxyethyl, compounds containing oxetanyl, or compounds containing a double bond, and the formula (VIII) is

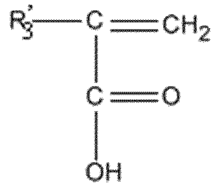

,

wherein $R_3$' is selected from hydrogen or methyl;

Process 4: using an olefinic monomer represented by formula (X) as a fifth starting material, and using a compound containing oxetanyl and epoxyethyl represented by formula (V) or formula (VI) as a second starting material, the preparation process including:

subjecting the hydroxyl group of the fifth starting material and the second starting material to an epoxyethyl ring-opening reaction, to obtain a fourth intermediate product containing hydroxyl group; reacting the hydroxyl group of the fourth intermediate product with a fourth end-capping compound, to obtain a fourth end-capped product; and copolymerizing the fourth end-capped product with a second olefinic monomer, to obtain the epoxy-modified acrylic resin; wherein the fourth end-capping compound is selected from anhydrides, acyl halides, compounds containing epoxyethyl, compounds containing oxetanyl, or compounds containing a double bond, and the formula (X) is

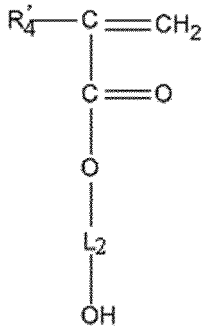

,

wherein $R_4$' is selected from hydrogen or methyl, $L_2$ is selected from $C_2$-$C_{25}$ linear or branched alkylene, and $-CH_2-$ in $L_2$ may be substituted by oxygen atom or ester group; preferably, $L_2$ is selected from $C_2$-$C_{10}$ linear or branched alkylene, and $-CH_2-$ in $L_2$ may be substituted by oxygen atom or ester group;

Process 5: using a compound containing oxetanyl and epoxyethyl represented by formula (V) or formula (VI) as a second starting material, using an acrylic resin comprising the repeat unit represented by formula (VII) as a third starting material, and using a polycarboxylic acid or cyclic anhydride represented by formula (A) or formula (B) as a sixth starting material, the preparation process including:

subjecting the hydroxyl group of the third starting material and the sixth starting material to an esterification reaction, to obtain a first esterified product containing carboxyl group; subjecting the carboxyl group of the first esterified product and the second starting material to an epoxyethyl ring-opening reaction, to obtain a fifth intermediate product containing hydroxyl group; and reacting the hydroxyl group of the fifth intermediate product with a fifth end-capping compound, to obtain the epoxy-modified acrylic resin, wherein the fifth end-capping compound is selected from anhydrides, acyl halides, compounds containing epoxyethyl, compounds containing oxetanyl, or compounds containing a double bond,

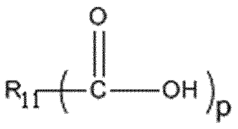 (A), 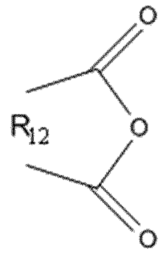 (B),

wherein $R_{11}$ is selected from $C_1$-$C_{20}$ linear or branched alkyl, $C_3$-$C_{20}$ cycloalkyl or substituted cycloalkyl, or $C_6$-$C_{24}$ aryl, and one or more hydrogen atoms in $R_{11}$ each may be independently substituted by a group selected from alkyl, halogen, or nitro; p is an integer of 2 to 6;
$R_{12}$ is selected from $C_1$-$C_{20}$ linear or branched alkyl, $C_3$-$C_{20}$ cycloalkyl or substituted cycloalkyl, or $C_6$-$C_{24}$ aryl, and one or more hydrogen atoms in $R_{12}$ each may be independently substituted by a group selected from carboxyl, alkyl, halogen, or nitro;

Process 6: using an olefinic monomer represented by formula (X) as a fifth starting material, using a polycarboxylic acid or cyclic anhydride represented by formula (A) or formula (B) as a sixth starting material, and using a compound containing oxetanyl and epoxyethyl represented by formula (V) or formula (VI) as a second starting material, the preparation process including:
subjecting the hydroxyl group of the fifth starting material and the sixth starting material to an esterification reaction, to obtain a second esterified product containing carboxyl group; subjecting the carboxyl group of the second esterified product and the second starting material to an epoxyethyl ring-opening reaction, to obtain a sixth intermediate product containing hydroxyl group; reacting the hydroxyl group of the sixth intermediate product with a sixth end-capping compound, to obtain the epoxy-modified acrylic resin, reacting with the sixth end-capping group to obtain a sixth end-capped product, wherein the sixth end-capping compound is selected from anhydrides, acyl halides, compounds containing epoxyethyl, compounds containing oxetanyl, or compounds containing a double bond; and copolymerizing the sixth end-capped product with a third olefinic monomer, to obtain the epoxy-modified acrylic resin;
Process 7: using 3-hydroxymethyl-3-ethyloxetanyl as a seventh starting material, and using methyl methacrylate as an eighth starting material, the preparation process including:

subjecting 3-hydroxymethyl-3-ethyloxetanyl as the seventh starting material and methyl methacrylate as the eighth starting material to a transesterification reaction, to obtain a transesterified product; and copolymerizing the transesterified product with a fourth olefinic monomer;
wherein in Processes 1 to 7, the first olefinic monomer, the second olefinic monomer, the third olefinic monomer, and the fourth olefinic monomer are the same or different.

Another aspect of the subject application also provides an energy-curable composition containing an epoxy-modified acrylic resin, comprising a resin, an initiator, and a curable epoxy-modified acrylic resin, wherein the epoxy-modified acrylic resin is the above epoxy-modified acrylic resin or prepared by the above preparation method.

Further the content of the epoxy-modified acrylic resin is 10-80 wt%; preferably, the content of the epoxy-modified acrylic resin is 30-75 wt%, by the weight percentage of the energy-curable composition containing epoxy-modified acrylic resin.

Still another aspect of the subject application also provides the use of the above energy-curable composition containing the epoxy-modified acrylic resin in the field of energy-curing.

Further, the field of energy-curing is the field of ink, paint, and adhesive.

By applying the technical solution of the present invention, after the branched chain of the acrylic resin is covalently grafted with the oxetanyl group, the epoxy-modified acrylic resin is obtained. The paint film made by the curable epoxy-modified acrylic resin composition containing the same has good flexibility, relatively high adhesion to the coated substrate, and a relatively high curing and film-forming rate. Since the above covalently grafting reaction does not affect the main chain structure of the original acrylic resin, during the downstream applications, it is unnecessary to change original devices and production processes, and it is just needed to add the curing equipment. Thus, the epoxy-modified acrylic having the above structure has low investment costs, and it is easier to be accepted and applied by the market.

DESCRIPTION OF EMBODIMENTS

It should be noted that, in the case of no conflict, the embodiments in the subject application as well as the features therein can be combined with each other. The present invention will be described in detail below with reference to the embodiments.

As described in the background, the existing epoxy-modified acrylic resins have problems such as poor hardness and adhesion during use. To solve the above technical problem, the subject application provides an epoxy-modified acrylic resin, wherein the branched chain of at least one repeat unit in the acrylic resin is grafted with one or more oxetanyl groups, each of the oxetanyl groups has a structure represented by general formula (I), general formula (II), or general formula (III), respectively:

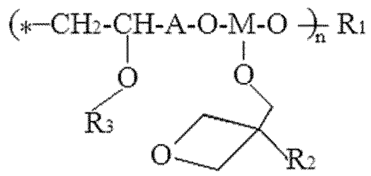

(I),

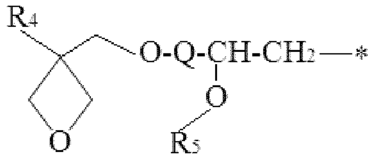

(II),

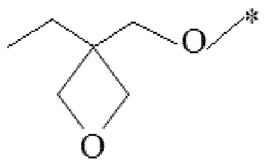

(III),

wherein $R_1$ represents $C_1$-$C_{40}$ linear or branched n-valent alkyl, $C_2$-$C_{30}$ n-valent alkenyl, or $C_6$-$C_{40}$ n-valent aryl, any one of $-CH_2-$ in $R_1$ may be substituted by oxygen atom, ester group, or

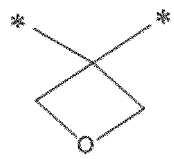

,

any one of hydrogen atoms in $R_1$ may be substituted by alkyl, halogen, or nitro, and n is selected from an integer of 1 to 12;

$R_2$ and $R_4$ independently represent hydrogen, halogen, nitro, $C_1$-$C_{30}$ linear or branched alkyl, $C_3$-$C_{30}$ cycloalkyl or substituted cycloalkyl, $C_2$-$C_{15}$ alkenyl, or $C_6$-$C_{30}$ aryl, respectively, any one of $-CH_2-$ in $R_2$ and $R_4$ may be substituted by oxygen atom or -COO-, and any one of hydrogen atoms in $R_2$ and $R_4$ may be substituted by alkyl, halogen, or nitro;

$R_3$ and $R_5$ independently represent $C_1$-$C_{30}$ linear or branched alkyl, $C_3$-$C_{30}$ cycloalkyl or substituted cycloalkyl, or $C_6$-$C_{30}$ aryl, respectively, any one of $-CH_2-$ in $R_3$ and $R_5$ may be substituted by oxygen atom or -COO-, and any one of hydrogen atoms in $R_3$ and $R_5$ may be substituted by alkyl, halogen, or nitro;

A represents $C_1$-$C_{20}$ linear or branched alkylene, any one of $-CH_2-$ in A may be substituted by oxygen atom or -COO-, and any one of hydrogen atoms in A may be substituted by alkyl, halogen, or nitro;

M represents 3-valent $C_1$-$C_{20}$ linear or branched alkyl, wherein any one of $-CH_2-$ in M may be substituted by oxygen atom, -COO-, or

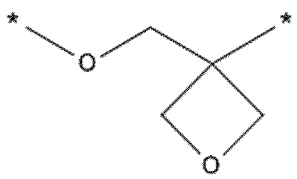

,

and any one of hydrogen atoms in M may be substituted by alkyl, halogen, or nitro;

Q represents $C_1$-$C_{20}$ linear or branched alkylene, wherein any one of $-CH_2-$ in Q may be substituted by oxygen atom, -COO-, or

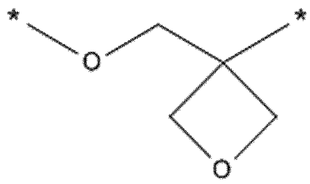

,

and any one of hydrogen atoms in Q may be substituted by alkyl, halogen, or nitro.

After the branched chain of the acrylic resin is covalently grafted with the oxetanyl group, the epoxy-modified acrylic resin is obtained. The paint film made by the curable epoxy-modified acrylic resin composition containing the same has good flexibility, relatively high adhesion to the coated substrate, and a relatively high curing and film-forming rate. Since the above covalently grafting reaction does not affect the main chain structure of the original acrylic resin, during the downstream applications, it is unnecessary to change original devices and production processes, and it is just needed to add the curing equipment. Thus, the epoxy-modified acrylic having the above structure has low investment costs, and it is easier to be accepted and applied by the market.

It should be noted that, "n-valent" in the term "n-valent alkyl" means that there are n substituents in the alkyl. Similarly, "n-valent alkenyl" means that there are n substituents in the alkenyl. Other expressions involving "n-valent group" in this application have the same interpretation.

Similarly, "n" in formula (I) refers to the number of substituents in $R_1$ group. For example, when n is 2, the number of substituents in $R_1$ group is 2.

After being cured, the epoxy-modified acrylic resin composition containing the above structure not only has good flexibility, but also has relatively high adhesion to the coated substrate and a relatively high curing and film-forming rate, while having the advantages such as no change in existing production equipment and the achievement of low VOC or even zero VOC emissions. In a preferred embodiment, $R_3$ and $R_5$ independently represent

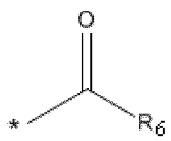

,

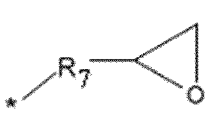

,

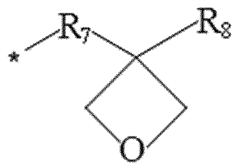

, or

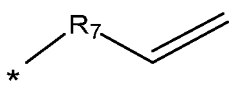

,

respectively,

$R_6$ represents $C_1$-$C_{30}$ linear or branched alkyl, $C_3$-$C_{30}$ cycloalkyl or substituted cycloalkyl, or $C_6$-$C_{30}$ aryl, wherein any one of $-CH_2-$ in $R_6$ may be substituted by oxygen atom or -COO-, and any one of hydrogen atoms in $R_6$ may be substituted by alkyl, halogen, or nitro; $R_7$ represents $C_1$-$C_{20}$ linear or branched alkylene; $R_8$ represents hydrogen, halogen, nitro, $C_1$-$C_{20}$ linear or branched alkyl, $C_3$-$C_{20}$ cycloalkyl or substituted cycloalkyl, $C_2$-$C_{10}$ alkenyl, or $C_6$-$C_{24}$ aryl, wherein any one of $-CH_2-$ in $R_7$ and $R_8$ may be substituted by oxygen atom or -COO-, and one or more hydrogen atoms in $R_7$ and

$R_8$ are each independently substituted by alkyl, halogen, or nitro;
preferably, $R_6$ represents $C_1$-$C_{20}$ linear or branched alkyl, $C_3$-$C_{20}$ cycloalkyl or substituted cycloalkyl, $C_6$-$C_{24}$ aryl, wherein any one of -$CH_2$- in $R_6$ may be substituted by oxygen atom or - COO-, any one of hydrogen atoms in $R_6$ may be substituted by alkyl, halogen, or nitro; $R_7$ represents $C_1$-$C_{10}$ linear or branched alkylene; $R_8$ represents hydrogen, halogen, nitro, $C_1$-$C_{10}$ linear or branched alkyl, $C_3$-$C_{10}$ cycloalkyl or substituted cycloalkyl, $C_2$-$C_{10}$ alkenyl, or $C_6$-$C_{12}$ aryl, wherein any one of -$CH_2$- in $R_7$ and $R_8$ may be substituted by oxygen atom or -COO-, and any one of hydrogen atoms in $R_7$ and $R_8$ may be substituted by alkyl, halogen, or nitro.

In a preferred embodiment, $R_1$ represents $C_1$-$C_{30}$ linear or branched n-valent alkyl, $C_2$-$C_{20}$ n-valent alkenyl, or $C_6$-$C_{30}$ n-valent aryl, any one of -$CH_2$- in $R_1$ may be substituted by oxygen atom, -COO-, or

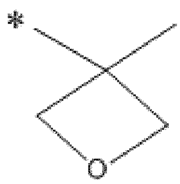

,

and any one of hydrogen atoms in $R_1$ may be substituted by alkyl, halogen, or nitro;
preferably, $R_1$ represents $C_1$-$C_{15}$ linear or branched n-valent alkyl, $C_2$-$C_{15}$ n-valent alkenyl, or $C_6$-$C_{18}$ n-valent aryl, any one of -$CH_2$- in $R_1$ may be substituted by oxygen atom, - COO-, or

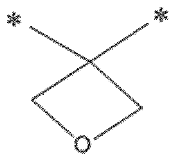

,

and any one of hydrogen atoms in $R_1$ may be substituted by alkyl, halogen, or nitro.

In a preferred embodiment, $R_2$ and $R_4$ independently represent hydrogen, halogen, nitro, $C_1$-$C_{20}$ linear or branched alkyl, $C_3$-$C_{20}$ cycloalkyl or substituted cycloalkyl, $C_2$-$C_{10}$ alkenyl, or $C_6$-$C_{24}$ aryl, respectively, any one of -$CH_2$- in $R_2$ and $R_4$ may be substituted by oxygen atom or - COO-, and any one of hydrogen atoms in $R_2$ and $R_4$ may be substituted by alkyl, halogen, or nitro;
preferably, $R_2$ and $R_4$ independently represent hydrogen, halogen, nitro, $C_1$-$C_{10}$ linear or branched alkyl, $C_3$-$C_{10}$ cycloalkyl or substituted cycloalkyl, $C_2$-$C_{10}$ alkenyl, or $C_6$-$C_{12}$ aryl, respectively, any one of -$CH_2$- in $R_2$ and $R_4$ may be substituted by oxygen atom or -COO-, and any one of hydrogen atoms in $R_2$ and $R_4$ may be substituted by alkyl, halogen, or nitro.

In a preferred embodiment, A represents $C_1$-$C_{15}$ linear or branched alkylene, any one of - $CH_2$- in A may be substituted by oxygen atom or -COO-, and any one of hydrogen atoms in A may be substituted by alkyl, halogen, or nitro.

In a preferred embodiment, M represents 3-valent $C_1$-$C_{15}$ linear or branched alkyl, any one of -$CH_2$- in M may be substituted by oxygen atom, -COO-, or

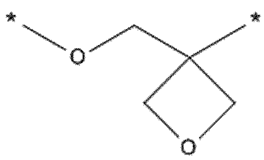

,

and any one of hydrogen atoms in M may be substituted by alkyl, halogen, or nitro.

In a preferred embodiment, Q represents $C_1$-$C_{15}$ linear or branched alkylene, any one of - $CH_2$- in Q may be substituted by oxygen atom, -COO-, or

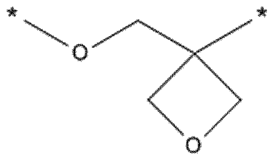

,

or any one of hydrogen atoms in Q may be substituted by alkyl, halogen, or nitro.

In order to reduce the difficulty in synthesizing the epoxy-modified acrylic resin, in a preferred embodiment, n is selected from an integer of 1 to 6.

More preferably, the above epoxy-modified acrylic resin includes, but is not limited to, one or more of the group consisting of:

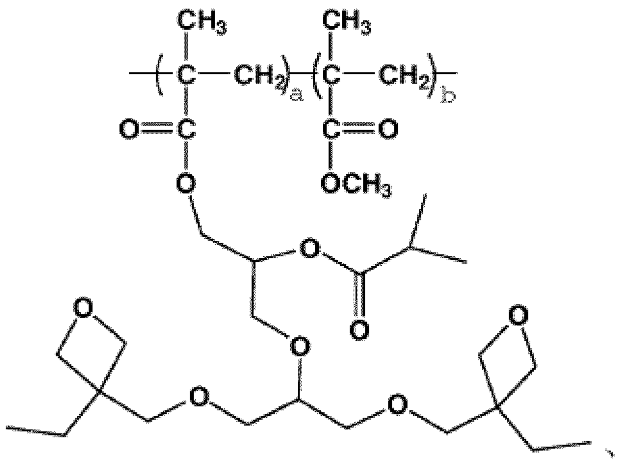

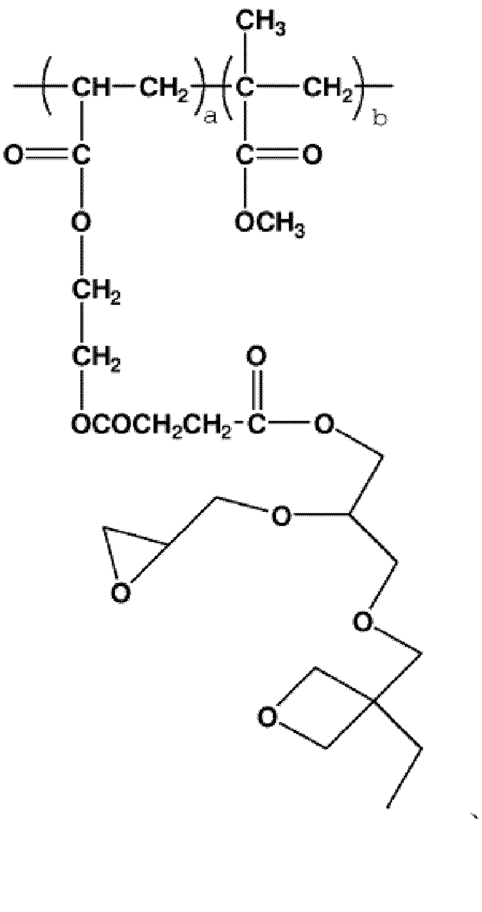

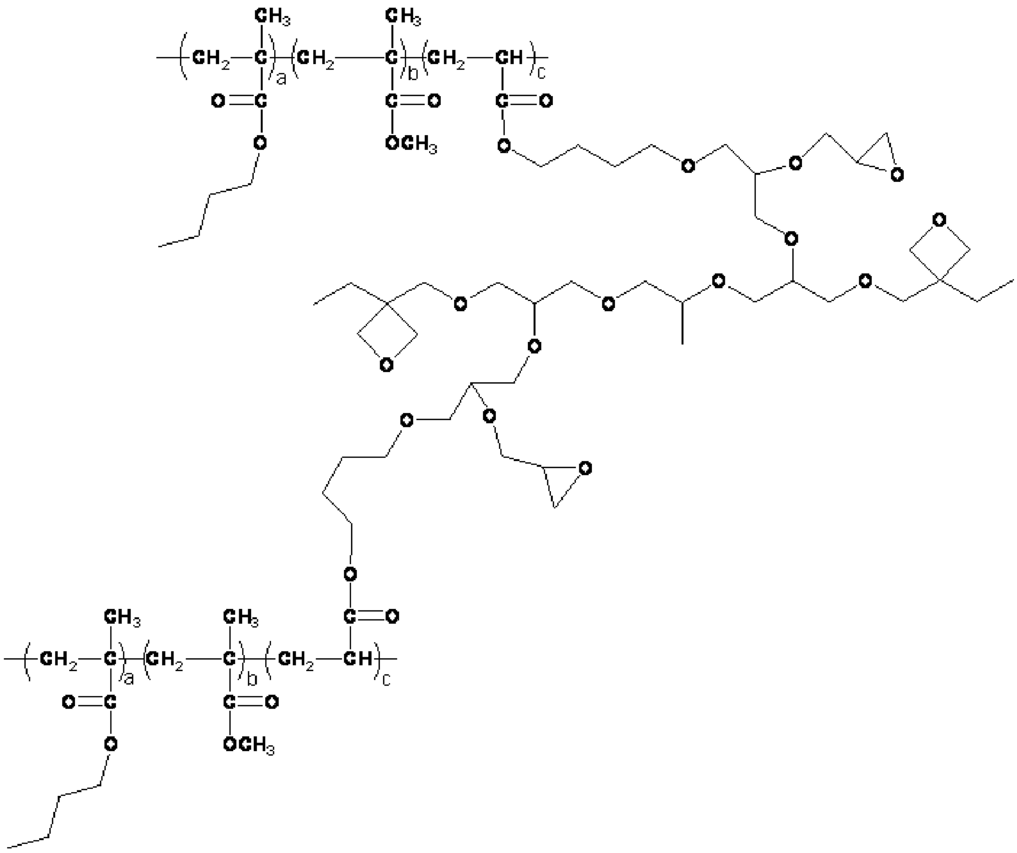

,

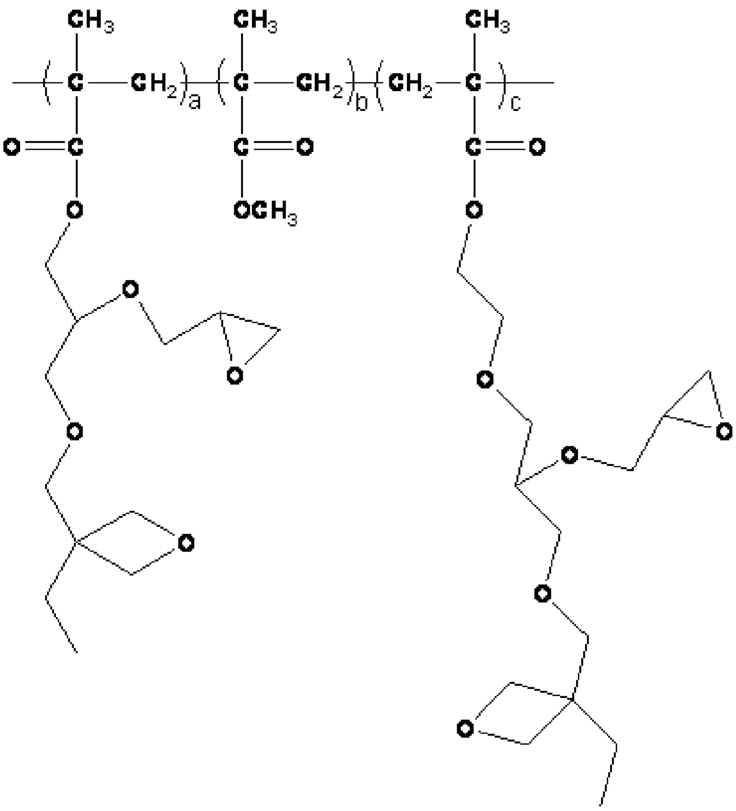

,

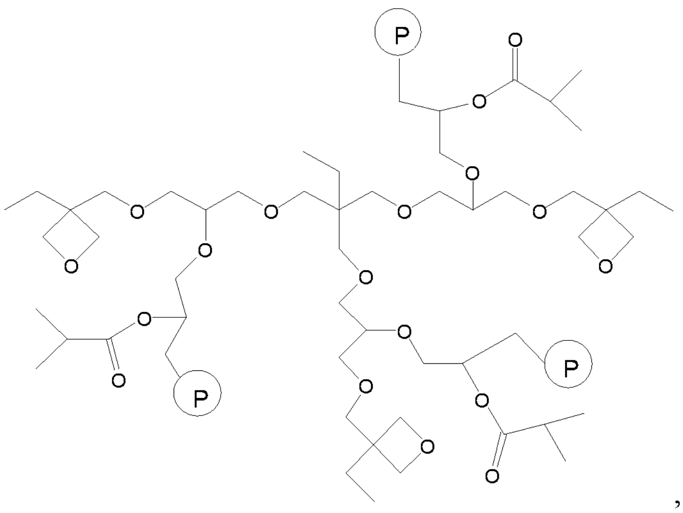

,

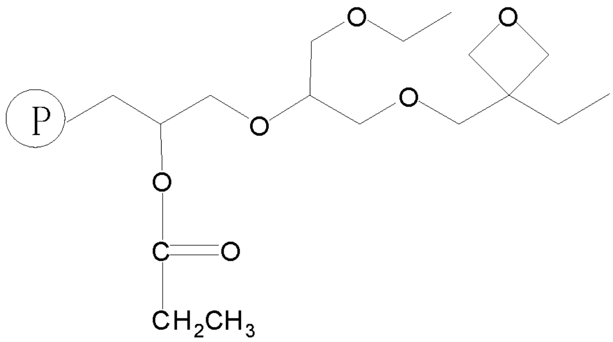

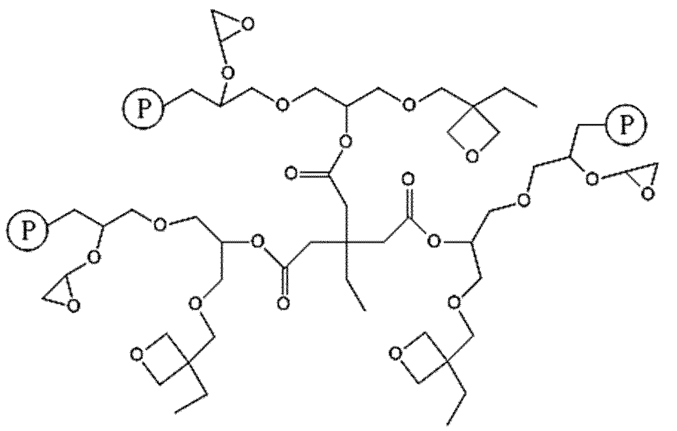

, ,

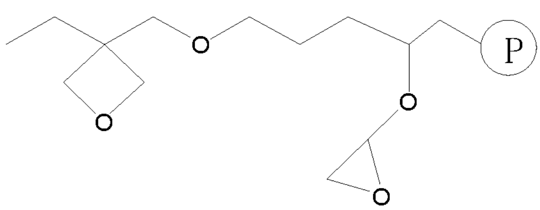

,

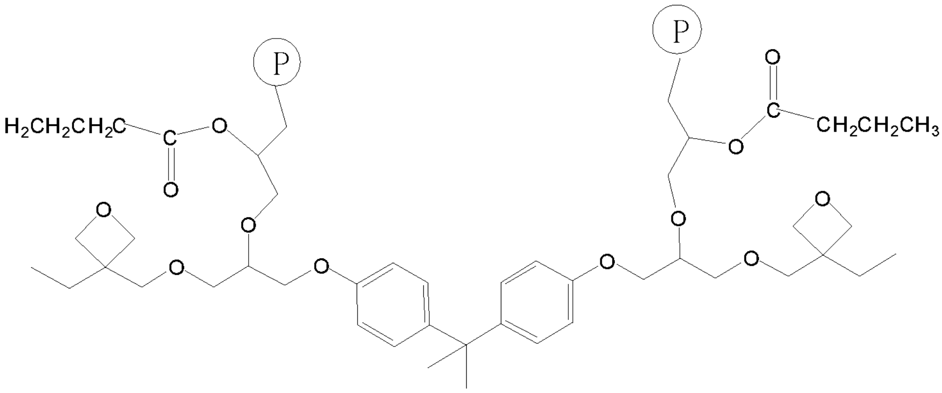

,

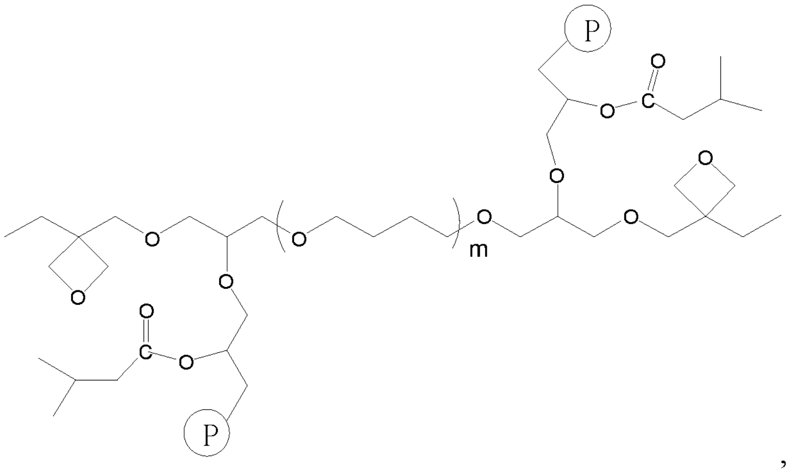

,

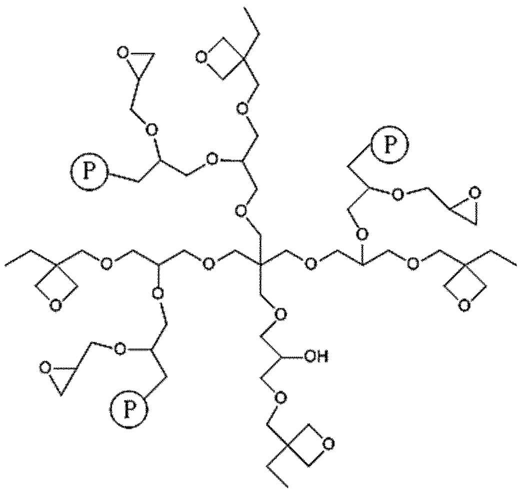

,

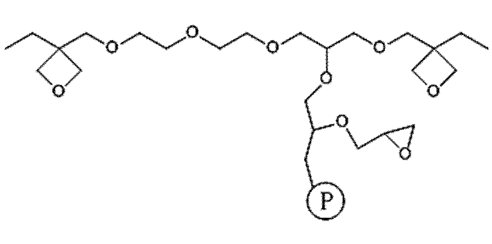

,

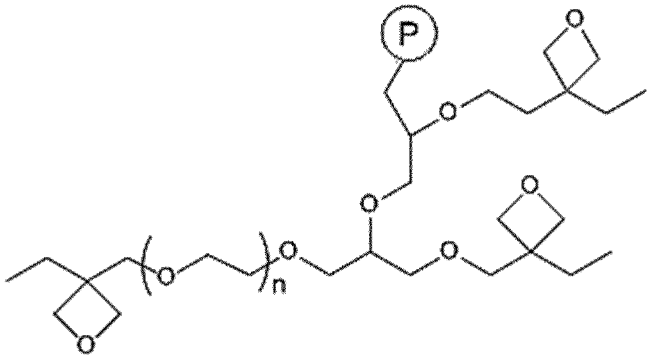

,

and

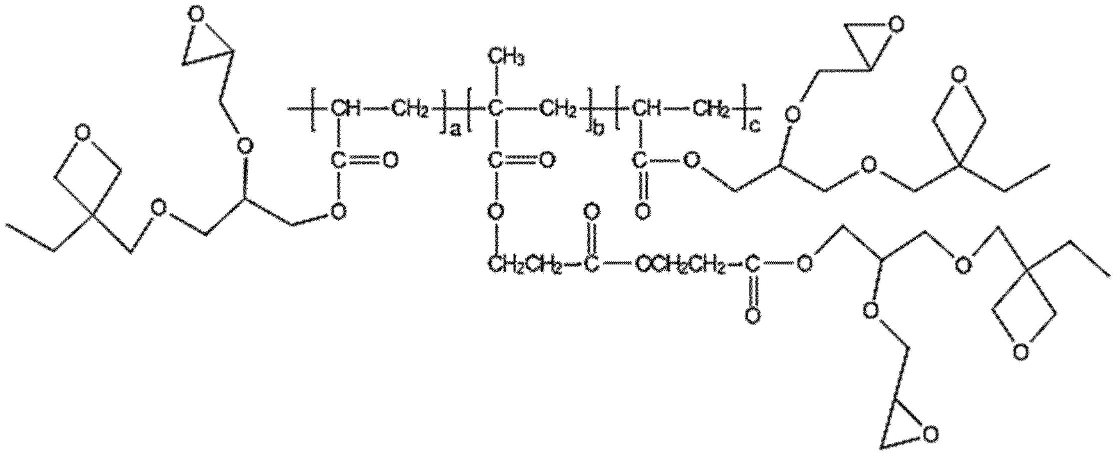

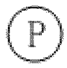

is

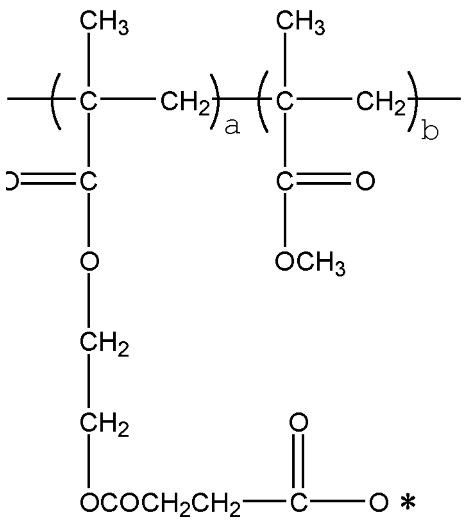

;

m is an integer of 1 to 30, preferably m is an integer of 1 to 10; n is an integer of 1 to 10, preferably n is an integer of 1 to 5; a, b, and c are independently an integer of 3 to 1000, respectively, preferably a, b, and c are independently an

integer of 3 to 200, respectively.

The use of the above epoxy-modified acrylic resin in the preparation of the energy-curable composition facilitates further improving its comprehensive properties such as adhesion to the substrate and hardness.

Another aspect of the subject application also provides a preparation method of the above epoxy-modified acrylic resin, wherein the epoxy-modified acrylic resin is prepared by any one of the following processes:

Process 1: using an acrylic resin comprising the repeat unit represented by formula (IV) as a first starting material, and using a compound containing oxetanyl and epoxyethyl represented by formula (V) or (VI) as a second starting material, the preparation process including:

subjecting the carboxyl group of the first starting material and the second starting material to an epoxyethyl ring-opening reaction, to obtain a first intermediate product containing hydroxyl group; and reacting the hydroxyl of the first intermediate product with a first end-capping compound, to obtain an epoxy-modified acrylic resin, wherein the first end-capping compound is selected from anhydrides, acyl halides, compounds containing epoxyethyl, compounds containing oxetanyl, or compounds containing a double bond, and the formula (IV), the formula (V), and the formula (VI) are respectively as follows:

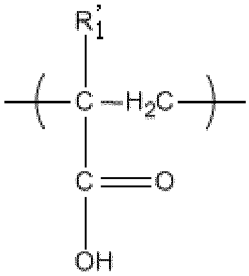

(IV)

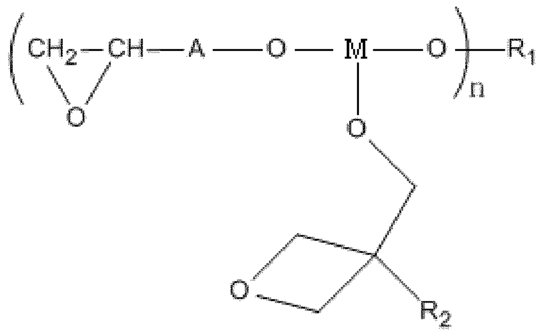

(V)

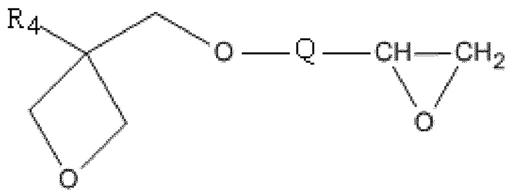

(VI)

wherein $R_1$, $R_2$, $R_4$, A, M, Q, and n have the same meanings as general formula (I) and general formula (II), and $R_1$' is selected from hydrogen or methyl;

Process 2: using an acrylic resin comprising the repeat unit represented by formula (VII) as a third starting material, and using a compound containing oxetanyl and epoxyethyl represented by formula (V) or formula (VI) as a second starting material, the preparation process including:

subjecting the hydroxyl group of the third starting material and the second starting material to an epoxyethyl ring-opening reaction, to obtain a second intermediate product containing hydroxyl group; and reacting the hydroxyl group of the second intermediate product with a second end-capping compound, to obtain an epoxy-modified acrylic resin, wherein the second end-capping compound is selected from anhydrides, acyl halides, compounds containing epoxyethyl, compounds containing oxetanyl, or compounds containing a double bond, the formula (VII) is

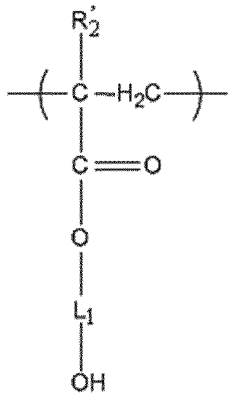

,

$R_2$' is selected from hydrogen or methyl, $L_1$ is selected from $C_2$-$C_{25}$ linear or branched alkylene, and $-CH_2-$ in $L_1$ may be substituted by oxygen atom or ester group; preferably, $L_1$ is selected from $C_2$-$C_{10}$ linear or branched alkylene, and $-CH_2-$ in $L_1$ may be substituted by oxygen atom or ester group;

Process 3: using an olefinic monomer represented by formula (VIII) as a fourth starting material, and using a compound containing oxetanyl and epoxyethyl represented by formula (V) or formula (VI) as a second starting material, the preparation process including:

subjecting the carboxyl group of the fourth starting material and the second starting material to an epoxyethyl ring-opening reaction, to obtain a third intermediate product containing hydroxyl group; and reacting the hydroxyl group of the third intermediate product with a third end-capping compound, to obtain a third end-capped product; and copolymerizing the third end-capped product with a first olefinic monomer, to obtain the epoxy-modified acrylic resin; wherein the third end-capping compound is selected from anhydrides, acyl halides, compounds containing epoxyethyl, compounds containing oxetanyl, or compounds containing a double bond, the formula (VIII) is

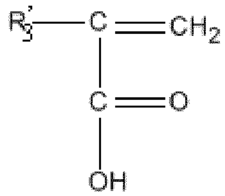

,

wherein $R_3$' is selected from hydrogen or methyl;

Process 4: using an olefinic monomer represented by formula (X) as a fifth starting material, and using a compound containing oxetanyl and epoxyethyl represented by formula (V) or formula (VI) as a second starting material, the preparation process including:

subjecting the hydroxyl group of the fifth starting material and the second starting material to an epoxyethyl ring-opening reaction, to obtain a fourth intermediate product containing hydroxyl group; reacting the hydroxyl group of the fourth intermediate product with a fourth end-capping compound, to obtain a fourth end-capped product; and copolymerizing the fourth end-capped product with a second olefinic monomer, to obtain the epoxy-modified acrylic resin; wherein the fourth end-capping compound is selected from anhydrides, acyl halides, compounds containing epoxyethyl, compounds containing oxetanyl, or compounds containing a double bond, the formula (X) is

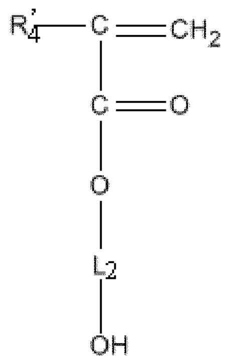

,

wherein $R_4$' is selected from hydrogen or methyl, $L_2$ is selected from $C_2$-$C_{25}$ linear or branched alkylene, and $-CH_2-$ in $L_2$ may be substituted by oxygen atom or ester group; preferably, $L_2$ is selected from $C_2$-$C_{10}$ linear or branched alkylene, and $-CH_2-$ in $L_2$ can be substituted by oxygen atom or ester group;

Process 5: using a compound containing oxetanyl and epoxyethyl represented by formula (V) or formula (VI) as a second starting material, using an acrylic resin comprising the repeat unit represented by formula (VII) as a third starting material, and using a polycarboxylic acid represented by formula (A) or a cyclic anhydride represented by formula (B) as a sixth starting material, the preparation process including:

subjecting the hydroxyl group of the third starting material and the sixth starting material to an esterification reaction, to obtain a first esterified product containing carboxyl group; subjecting the carboxyl group of the first esterified product and the second starting material to an epoxyethyl ring-opening reaction, to obtain a fifth intermediate product containing hydroxyl group; and reacting the hydroxyl group of the fifth intermediate product with a fifth end-capping compound, to obtain the epoxy-modified acrylic resin, wherein the fifth end-capping compound is selected from anhydrides, acyl halides, compounds containing epoxyethyl, compounds containing oxetanyl, or compounds containing a double bond, wherein the formula (A) is

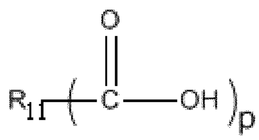

,

the formula (B) is

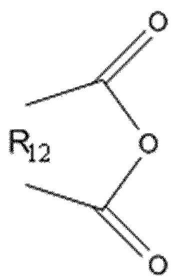

,

wherein $R_{11}$ is selected from $C_1$-$C_{20}$ linear or branched alkyl, $C_3$-$C_{20}$ cycloalkyl or substituted cycloalkyl, or $C_6$-$C_{24}$ aryl, and one or more hydrogen atoms in $R_{11}$ each may be independently substituted by a group selected from alkyl, halogen, or nitro; p is an integer of 2 to 6;

$R_{12}$ is selected from $C_1$-$C_{20}$ linear or branched alkyl, $C_3$-$C_{20}$ cycloalkyl or substituted cycloalkyl, or $C_6$-$C_{24}$ aryl, and one or more hydrogen atoms in $R_{12}$ each may be independently substituted by a group selected from carboxyl, alkyl, halogen, or nitro;

Process 6: using an olefinic monomer represented by formula (X) as a fifth starting material, using a polycarboxylic acid or cyclic anhydride represented by formula (A) or formula (B) as a sixth starting material, and using a compound containing oxetanyl and epoxyethyl represented by formula (V) or formula (VI) as a second starting material, the preparation process including: subjecting the hydroxyl group of the fifth starting material and the sixth starting material to an esterification reaction, to obtain a second esterified product containing carboxyl group; subjecting the carboxyl

group of the second esterified product and the second starting material to an epoxyethyl ring-opening reaction, to obtain a sixth intermediate product containing hydroxyl group; reacting the hydroxyl group of the sixth intermediate product with a sixth end-capping compound, to obtain the epoxy-modified acrylic resin, reacting with the sixth end-capping group to obtain a sixth end-capped product, wherein the sixth end-capping compound is selected from anhydrides, acyl halides, compounds containing epoxyethyl, compounds containing oxetanyl, or compounds containing a double bond; and copolymerizing the sixth end-capped product with a third olefinic monomer, to obtain the epoxy-modified acrylic resin;

Process 7: using 3-hydroxymethyl-3-ethyloxetanyl as a seventh starting material, and using methyl methacrylate as an eighth starting material, the preparation process including:

subjecting 3-hydroxymethyl-3-ethyloxetanyl as the seventh starting material and methyl methacrylate as the eighth starting material to a transesterification reaction, to obtain a transesterified product; and copolymerizing the transesterified product with a fourth olefinic monomer;

wherein in Processes 1 to 7, the first olefinic monomer, the second olefinic monomer, the third olefinic monomer, and the fourth olefinic monomer are the same or different.

In the chemical grafting method, the acrylic resin is formed by the copolymerization of acrylate or methacrylate with other olefinic monomers, wherein at least one of the acrylic monomers and other olefinic monomers is a hydroxyl-containing or carboxyl-containing functional monomer. For the hydroxyl-containing functional monomer, hydroxyethyl acrylate, hydroxybutyl acrylate, hydroxyethyl methacrylate, hydroxybutyl methacrylate, hydroxypropyl acrylate, hydroxypropyl methacrylate, 4-hydroxybutyl acrylate, allyl alcohol, cinnamyl alcohol, citronellol, and 2-hexen-l-ol can be listed; and for the carboxyl-containing functional monomer, acrylic acid, methacrylic acid, butenoic acid, crotonic acid, itaconic acid, maleic acid, maleic anhydride, fumaric acid, 2-carboxyethyl (methyl)acrylate, 3-carboxypropyl (methyl)acrylate, and 5-carboxypentyl (methyl)acrylate can be listed. For other olefinic monomers, the group consisting of methyl acrylate, methyl methacrylate, ethyl acrylate, ethyl methacrylate, propyl acrylate, propyl methacrylate, isopropyl acrylate, isopropyl methacrylate, butyl acrylate, butyl methacrylate, isobutyl acrylate, isobutyl methacrylate, hexyl acrylate, hexyl methacrylate, acrylamide, glycidyl acrylate, dimethylaminopropyl acrylate, tetrahydrofuryl acrylate, fluoroalkyl acrylate, acrylonitrile, or styrene can be listed, or they can also be olefinic monomers grafted with oxetanyl groups prepared according to the present invention.

In a preferred embodiment, the second starting material (the compound containing oxetanyl and epoxyethyl) is selected from one or more of the group consisting of:

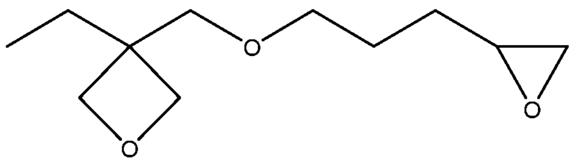
,
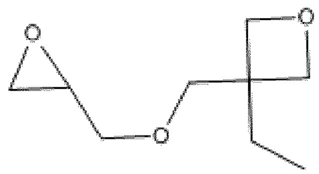
,

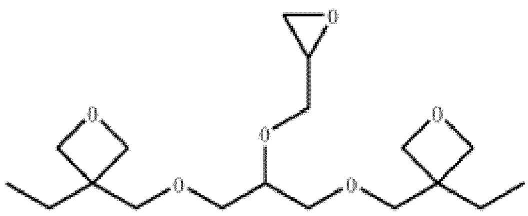
,
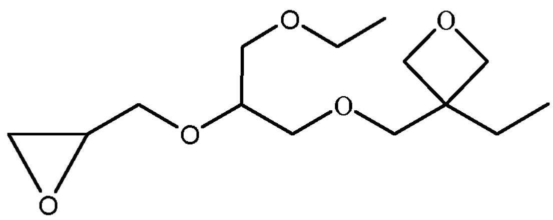
,

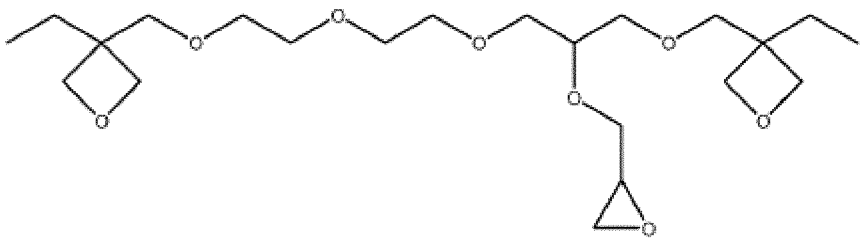
.

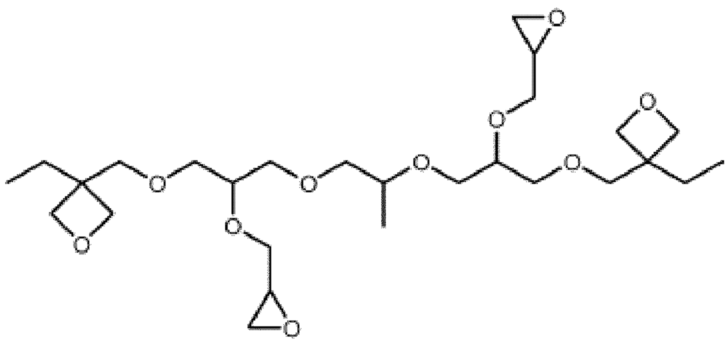

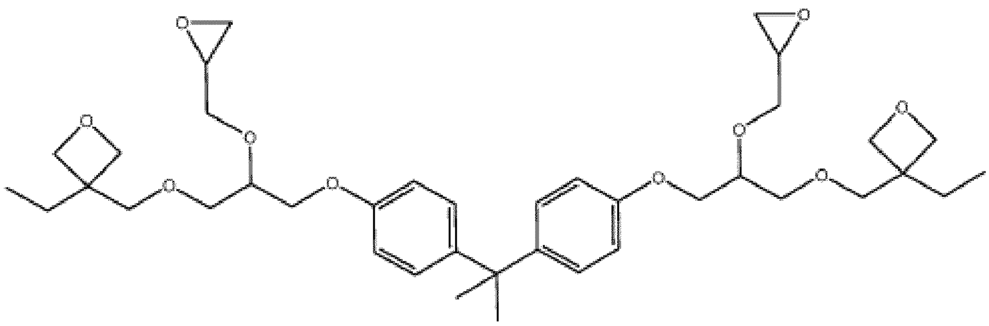

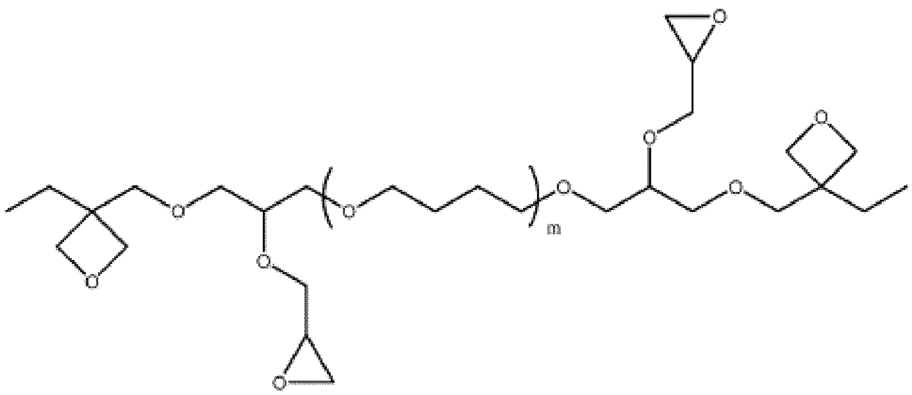

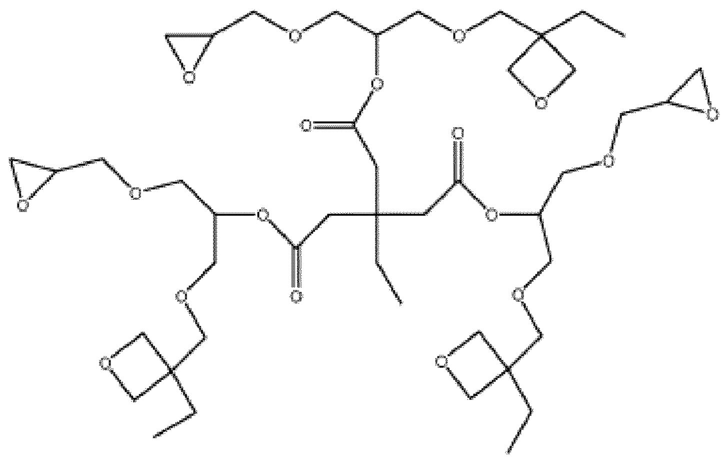

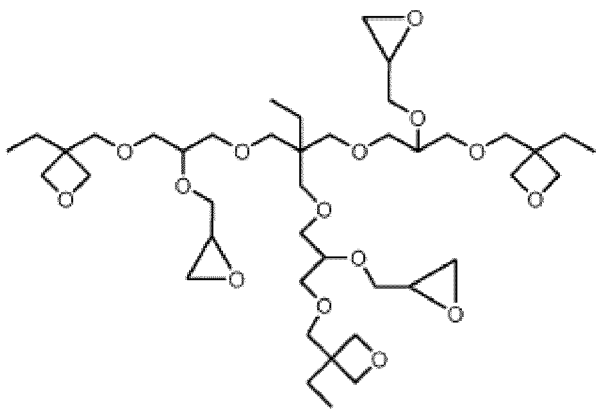

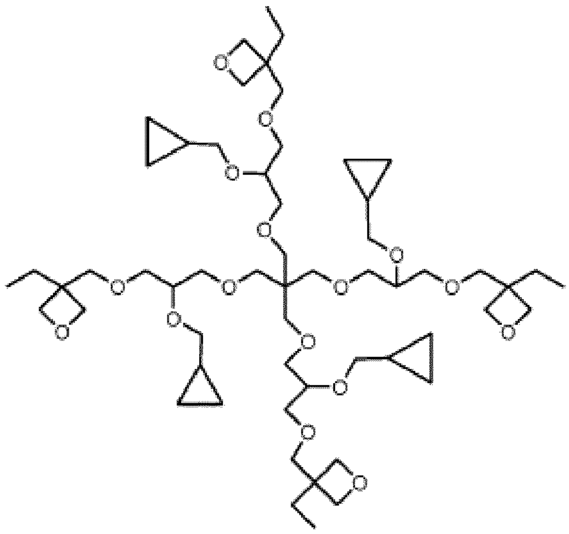

,

and

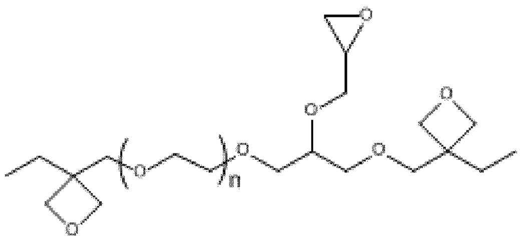

,

wherein m is an integer of 1 to 30, preferably m is an integer of 1 to 10; n is an integer of 1 to 10, preferably n is an integer of 1 to 5.

In a preferred embodiment, the first end-capping compound, the second end-capping compound, the third end-capping compound, the fourth end-capping compound, the fifth end-capping compound, the sixth end-capping compound are independently selected from compounds represented by formula (C) to formula (G), respectively.

An acyl halide:

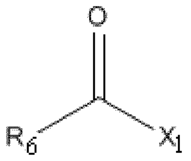

(C),

a compound containing epoxyethyl:

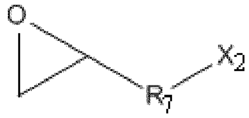

(D),

a compound containing oxetanyl:

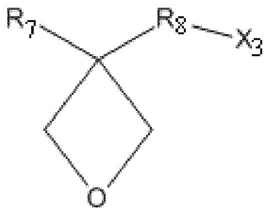

(E),

a compound containing a double bond:

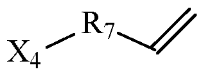

(F),

and an anhydride:

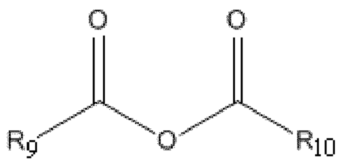

(G),

wherein $R_6$, $R_7$, and $R_8$ have the same meanings as above, $R_9$ and $R_{10}$ are independently selected from $C_1$-$C_6$ linear or branched alkyl, respectively, and $X_1$, $X_2$, $X_3$, and $X_4$ are independently selected from halogen atom, respectively.

Exemplarily, for $R_9$ and $R_{10}$ in anhydride (G), methyl, ethyl, n-propyl, isopropyl, n-butyl, isobutyl, tert-butyl, n-pentyl, isopentyl, neopentyl, tert-pentyl, n-hexyl, and isohexyl can be listed.

Exemplarily, for the acyl halide (C), acetyl chloride, propionyl chloride, butyryl chloride, isobutyryl chloride, n-valeryl chloride, isovaleryl chloride, trimethylacetyl chloride, tert-butylacetyl chloride, benzoyl chloride, cyclohexanecarbonyl chloride, methacryloyl chloride, phthaloyl chloride, o-chloroterephthaloyl chloride, biphenyldicarbonyl chloride, propionyl bromide, crotonoyl bromide, 2-bromocapryloyl bromide, p-bromophenylacetyl bromide, iodomethyl pivalate, and acryloyl iodide can be listed.

Exemplarily, for the compound containing epoxyethyl (D), epichlorohydrin, epoxychloroethane, epoxychlorobutane, 2-((2-chloroethoxy)methyl)oxirane, 2-((2-(chloromethoxy)ethoxy)methyl)oxirane, epoxybromopropane, [(1,1,2,2-tetrafluoroethoxy)methyl]oxirane, and 2-trimethyloxirane can be listed.

Exemplarily, for the compound containing oxetanyl (E), 2-methyl-2-iodomethyloxetane, 3,3-diiodomethyl-1-oxetane, 3-bromomethyl-3-methyl-1-oxetane, 3-(3-bromophenyl)oxetane, 3-bromophenyl-3-methyloxetane, 3-chloromethyl-3-methyloxetane, 3,3-bis(chloromethyl)oxetane, 3-fluoromethyl-3-chloromethyloxetane, 3-chloromethyl-3-iodomethyl-1-oxetane, 3-bromomethyl-3-chloromethyl-1-oxetane, 3-iodomethyloxetane, 3-iodooxetane, 3-bromomethyloxetane, 3-chlorooxetane, 3-chloromethyloxetane, and 3-iodomethyl-3-methyloxetane can be listed.

Exemplarily, for the compound containing a double bond (F), 3-chloropropene, 3-bromopropene, 3-chloromethoxy-1-propene, vinyl 2-chloroacetate, 6-chloro-3-methyl-1-hexene, 3-chloro-1-butene, or 3-bromomethoxy-1-propene can be listed.

In a preferred embodiment, in the polycarboxylic acid represented by formula (A), $R_{11}$ represents $C_1$-$C_{10}$ linear or branched alkyl, $C_3$-$C_{10}$ cycloalkyl or substituted cycloalkyl, or $C_6$-$C_{12}$ aryl, and one or more hydrogen atoms in $R_{11}$ each may be independently substituted by a group selected from alkyl, halogen, or nitro.

In a preferred embodiment, in the cyclic anhydride represented by formula (B), $R_{12}$ is selected from $C_1$-$C_{10}$ linear or branched alkyl, $C_3$-$C_{15}$ cycloalkyl or substituted cycloalkyl, $C_6$-$C_{18}$ aryl, and one or more hydrogen atoms in $R_{12}$ each may be independently substituted by a group selected from carboxyl, alkyl, halogen, or nitro.

Exemplarily, for polycarboxylic acid, oxalic acid, malonic acid, adipic acid, sebacic acid, fumaric acid, isophthalic acid, 1,4-cyclohexanedicarboxylic acid, 1,3-cyclohexanedicarboxylic acid, terephthalic acid, trimellitic acid, 1,2,4-cyclohexanetricarboxylic acid, cyclohexanetetracarboxylic acid, cyclobutanetetracarboxylic acid, 1,2,3,4-butanetetracarboxylic acid, and 1,4,5,8-naphthalenetetracarboxylic acid can be listed.

Exemplarily, for cyclic anhydride, cyclopentane-1,2-dicarboxylic anhydride, maleic anhydride, succinic anhydride, 2-(2-oct-3-enyl)succinic anhydride, glutaric anhydride, citraconic anhydride, itaconic anhydride, phthalic anhydride, cis-cyclohexane-1,2-dicarboxylic anhydride, norbomenedioic anhydride, 3-vinyl phthalic anhydride, 4-vinyl phthalic anhydride, dimethyltetrahydrophthalic anhydride, 1,2,3,6-tetrahydrophthalic anhydride, n-dodecylsuccinic anhydride, dodecenylsuccinic anhydride, hexahydrophthalic anhydride, trimellitic anhydride, 2,3,3',4'-diphenyl ether tetracarboxylic dianhydride, methylnorbornene-2,3-dicarboxylic anhydride, 5-norbornene-2,3-dicarboxylic anhydride, benzophenonetetracarboxylic dianhydride, and cyclopentane tetracarboxylic dianhydride can be listed.

Another aspect of the subject application also provides an energy-curable composition containing an epoxy-modified acrylic resin, comprising a resin, an initiator, and a curable epoxy-modified acrylic resin, wherein the epoxy-modified acrylic resin is the above epoxy-modified acrylic resin or prepared by the above method.

After the branched chain of the acrylic resin in the epoxy-modified acrylic resin is covalently grafted with oxetanyl, the paint film made by the curable epoxy-modified acrylic resin composition containing the same has good flexibility, relatively high adhesion to the coated substrate, and a relatively high curing and film-forming rate. In addition, since it does not affect the main chain structure of the original acrylic resin, during the downstream applications, as compared to the original acrylic resin, it is unnecessary to change original devices and production processes, and it is just needed to add the curing equipment. Thus, it has low investment costs, and it is easier to be accepted and applied by the market. Moreover, the above epoxy-modified acrylic resin has good compatibility with the resin and initiator. Thus, the diluting solvent or active monomer may have reduced amounts or not be used during its use, improving the environmental conditions during the production and use of the paint, ink, and adhesive and achieving low VOC or even zero VOC emissions. In summary, the energy-curable composition containing the epoxy-modified acrylic resin as provided in the subject application not only has good flexibility, but also has relatively high adhesion to the coated substrate and a relatively high curing and film-forming rate, while it has the advantages such as no change in existing production equipment and the achievement of low VOC or even zero VOC emissions.

In the above energy-curable composition containing epoxy-modified acrylic resin, the resin is one or more of (modified) alkyd resin, (modified) phenolic resin, (modified) amino resin, (modified) polyester resin, (modified) acrylic resin, (modified) polyurethane resin, (modified) polyurea resin, (modified) fluorocarbon resin, (modified) silicone resin, (modified) chlorinated polyolefin resin.

The above modified resin refers to a modified resin obtained by modifying an existing resin (such as alkyd resin and phenolic resin) through a chemical or physical method (such as grafting or blending) in the prior art. The term "(modified) alkyd resin" represents an alkyd resin or modified alkyd resin, and similar terms are interpreted in the same way.

In a preferred embodiment, by the weight percentage of the energy-curable composition containing the epoxy-modified acrylic resin, the content of the epoxy-modified acrylic resin is 10-80 wt%. The amount of the epoxy-modified acrylic resin includes but is not limited to the above range, while limiting the amount within the above range facilitates further improving the comprehensive properties of the coating formed after the energy-curing of the energy-curable composition containing epoxy-modified acrylic resin. Preferably, the content of the epoxy-modified acrylic resin is 30-75 wt%.

For the above energy-curable composition containing the epoxy-modified acrylic resin, the resin composition can be cured by at least one of light, heat, or electron radiation. Preferably, the composition is cured by UV light. The UV light source may be configured to be a light source or radiation source emitting light in the ultraviolet region (i.e., between 10 nm and 420nm), which, for example, may be selected from fluorescent lamps, fluorescent black-light lamps, short-wave ultraviolet lamps, lasers, ultraviolet lasers, ultraviolet gas lasers, high-power gas lasers (e.g., nitrogen lasers or excimer lasers), ultraviolet laser diodes, ultraviolet solid-state lasers, electron beams, illuminators, monochromatic light sources, light emitting diodes (LED), LED arrays, ultraviolet LED, gas discharge lamps, argon and deuterium lamps, Hg-Cd lamps, arc lamps, electric arc lamps, flash lamps, Xe or halogen lamps, or any other suitable light sources.

In a preferred embodiment, the above energy-curable composition containing the epoxy-modified acrylic resin

also comprises an auxiliary agent. The above auxiliary agent includes, but is not limited to, one or more of flame retardant, nucleating agent, coupling agent, filler, plasticizer, impact modifier, lubricant, antibacterial agent, mold release agent, heat stabilizer, antioxidant, light stabilizer, compatibilizer, colorant, stabilizer, separant, antistatic agent, pigment, dye, and fire retardant.

The above initiator is a cationic initiator. Preferably, the cationic initiator is selected from one or more of a diazonium salt, an onium salt, and an organometallic complex capable of forming a strong acid when an external energy source is applied. More preferably, the cationic initiator includes, but is not limited to, one or more of the group consisting of diazonium fluoroborate, pyrazole diazonium inner salt, triptycene diazonium salt, diazoaminobenzene, triarylsulfonium hexafluorophosphate, triarylsulfonium antimonate, 4,4'-dimethyldiphenyliodonium hexafluorophosphate, 4,4'-dimethyldiphenyliodonium hexafluorophosphate, 10-(4-biphenylyl)-2-isopropylthioxanthenone-10-sulfonium hexafluorophosphate, 4-octyloxydiphenyliodonium hexafluoroantimonate, bis(4-tert-butylbenzene)iodonium hexafluorophosphate, diphenyl-(4-phenylthio)phenylsulfonium hexafluorophosphate, bis(4-diphenylthiophenyl)sulfide dihexafluoroantimonate, 4-isobutylphenyl-4'-methylphenyliodonium hexafluorophosphate, and 6-cumylferrocene hexafluorophosphate.

The above energy-curable epoxy-modified acrylic resin composition can be applied in the field of energy-curing. The coating formed by the composition containing the above energy-curable epoxy-modified acrylic resin not only has good flexibility, but also has relatively high adhesion to the coated substrate and a relatively high curing and film-forming rate, while has the advantages such as no change in existing production equipment and the achievement of low VOC or even zero VOC emissions.

Preferably, the field of energy-curing is the fields of ink, paint, and adhesive. Exemplarily, letterpress ink, gravure ink, planographic ink, and screen ink can be listed for the ink; building paint, anticorrosive paint, automotive paint, antifogging paint, antirust paint, waterproofing paint, moisturizing paint, elastic paint can be listed for the paint; and solvent adhesive, emulsion adhesive, reactive (heat-curable, UV-curable, moisture-curable) adhesive, hot-melt adhesive, rewetting adhesive, pressure-sensitive adhesive can be listed for the adhesive.

The subject application is further described in detail below with reference to specific examples, which cannot be understood as limiting the claimed scope in the subject application.

**(I) Preparation of epoxy-modified acrylic resin**

**Preparations of starting materials 1 to 12:**

Starting material 1:

Reaction: 3-hydroxymethyl-3-ethyloxetane (58 g, 0.5 mol), epichlorohydrin (46 g, 0.5 mol), and sodium hydroxide (20 g, 0.5 mol) were sequentially added to a three-necked flask, and reacted at 40 ° C for 12h. 3-Hydroxymethyl-3-ethyloxetanyl was traced by GC until it was completely reacted. After the completion of the reaction, the product was washed with water, extracted, and distilled under reduced pressure, to obtain 86 g of starting material 1.

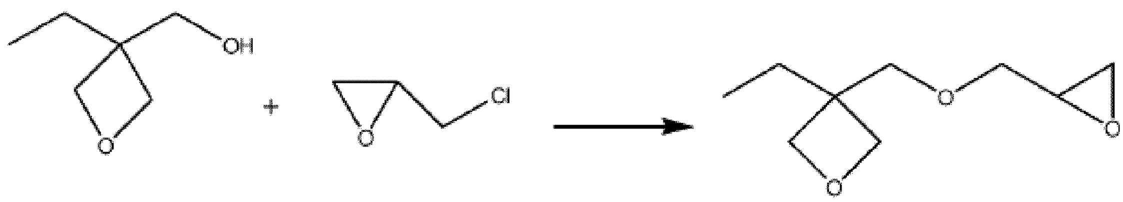

Starting material 2:

Reaction 1: 3-hydroxymethyl-3-ethyloxetanyl(58 g, 0.5 mol), sodium hydroxide (4 g, 0.1 mol), and toluene (100g) were sequentially added to a three-necked flask, and heated to 80 °C under stirring. The starting material 1 (86 g, 0.5 mol) was dropwise added over 1.5 h. The reaction was continued under stirring, and when 3-hydroxymethyl-3-ethyloxetanyl was traced by GC until its content remained unchanged, the heating was stopped. The reaction system was adjusted to neutral pH, filtered, washed with water, extracted, and distilled under reduced pressure, to obtain 130 g of compound 1 as light yellow thick liquid.

Reaction 2: compound 1 (144 g, 0.5 mol), epichlorohydrin (46 g, 0.5 mol), and sodium hydroxide (20 g, 0.5 mol) were sequentially added to a three-necked flask, and reacted at 40 °C for 12h. Compound 1 was traced by GC until it was completely reacted. After the completion of the reaction, the product was washed with water, extracted, and distilled under reduced pressure, to obtain 155 g of starting material 2.

The synthesis route is as follows:

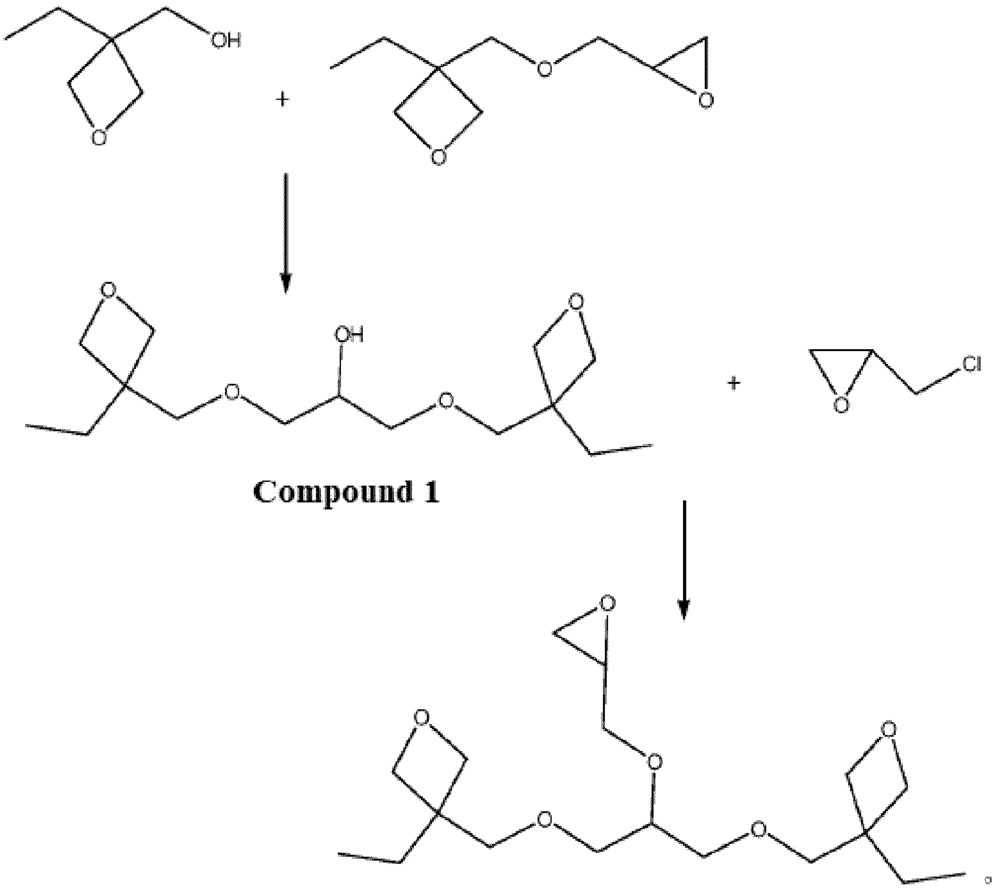

Compound 1

In accordance with the preparation methods for starting materials 1 and 2, starting materials 3 to 12 were prepared.

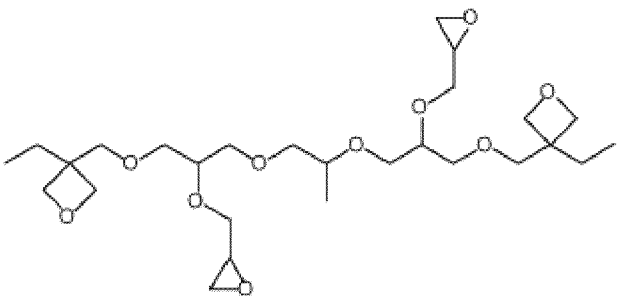

Starting material 3;

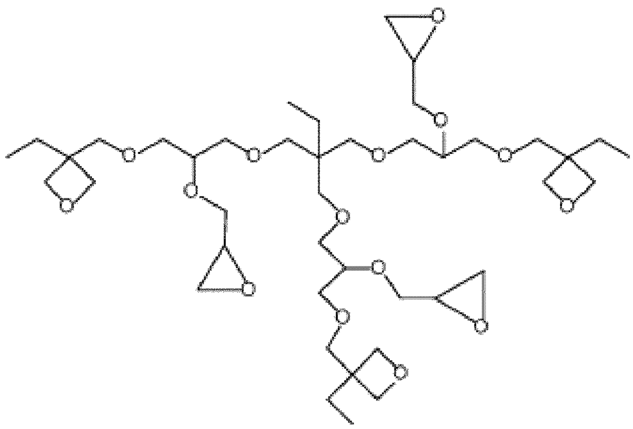

Starting material 4;

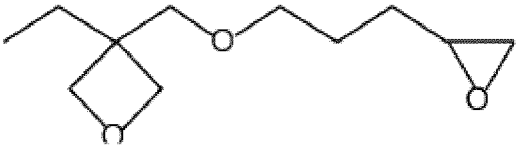

Starting material 5;

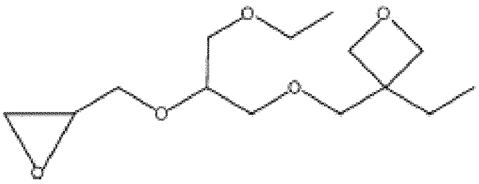

Starting material 6;

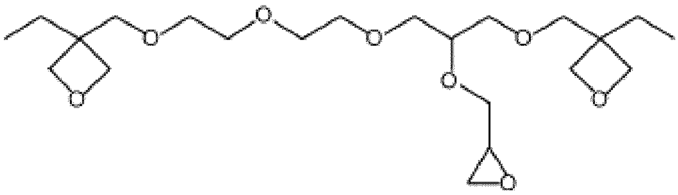

Starting material 7;

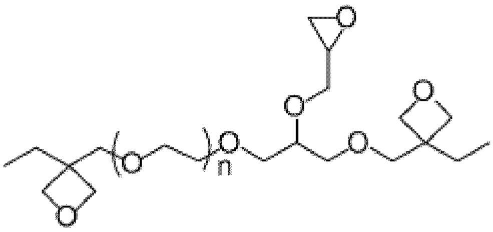

Starting material 8;

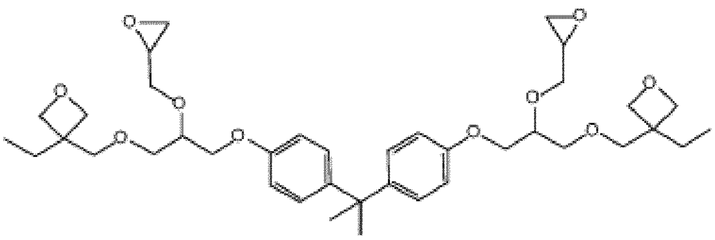

Starting material 9;

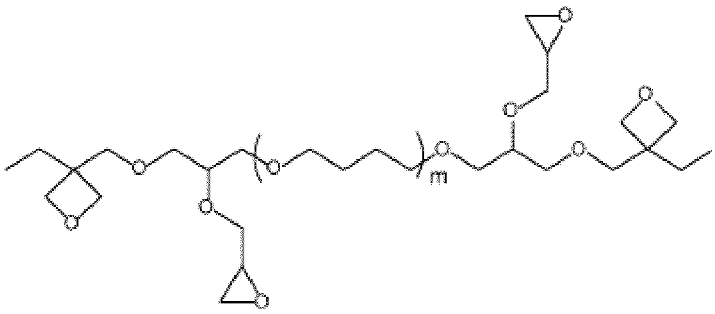

Starting material 10;

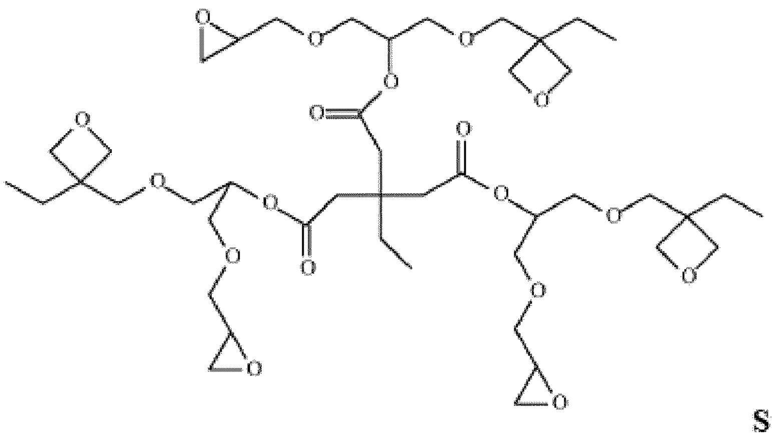

Starting material 11;

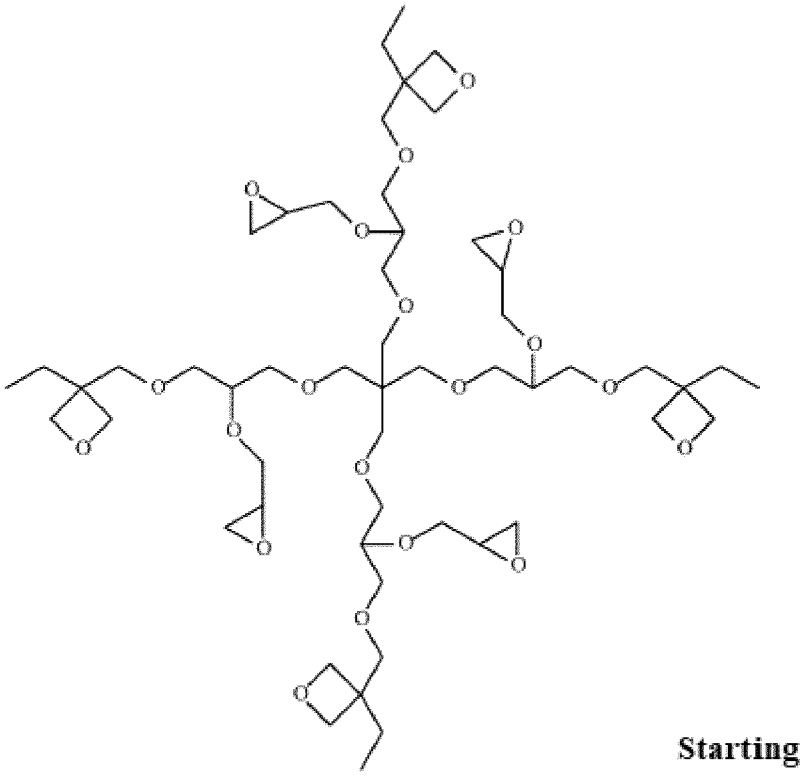

Starting material 12.

**Preparation of epoxy-modified acrylic resin:**

Example 1

Reaction 1: preparation of acrylic resin: the copolymerization reaction using methacrylic acid monomer (MAA) and methyl methacrylate monomer (MMA) as starting materials was conducted as follows: MAA (43 g, 0.5 mol) and propylene glycol methyl ether acetate (200 mL) were added to a reaction flask and heated to 60 °C, and then a mixed solution of MMA (50 g, 0.5 mol) and azodiisobutyronitrile (2.5 g, 3 wt%) was slowly dropwise added to the above reaction flask over a feeding time of 0.5 h. After the reactants were completely added, the reaction was continued for 6 h, to obtain a white polymeric precipitate. The above precipitate was filtered and dried, to obtain 85 g of MAA-MMA acrylic resin.

Reaction 2: the MAA-MMA acrylic resin (100 g, containing 0.5 mol of carboxyl group) was added to a three-necked flask, followed by toluene (500 mL), and heated at 70 ° C for dissolving. After dissolving, triphenylphosphine (8 g) and starting material 2 (170 g, 0.50 mol) were added. After 8 h of reaction, the product was extracted, and distillated under reduced pressure, to obtain 250 g of epoxy-modified acrylic resin.

Reaction 3: the above epoxy-modified acrylic resin (100 g, containing 0.2 mol of hydroxyl group), isobutyryl chloride (21 g, 0.2 mol), toluene (200 mL), and sodium hydroxide (10 g) were sequentially added to a three-necked flask,

and reacted at 40 ° C for 12 h. After the completion of the reaction, the product was washed with water, extracted, and distillated under reduced pressure, to obtain 110 g of epoxy-modified acrylic resin 1 having a weight-average molecular weight of 5×$10^4$, a molecular weight distribution index of 1.35, an acid value of 0.4 mgKOH/g, and an epoxy equivalent of 263 g/mol.

The reaction process is as follows:

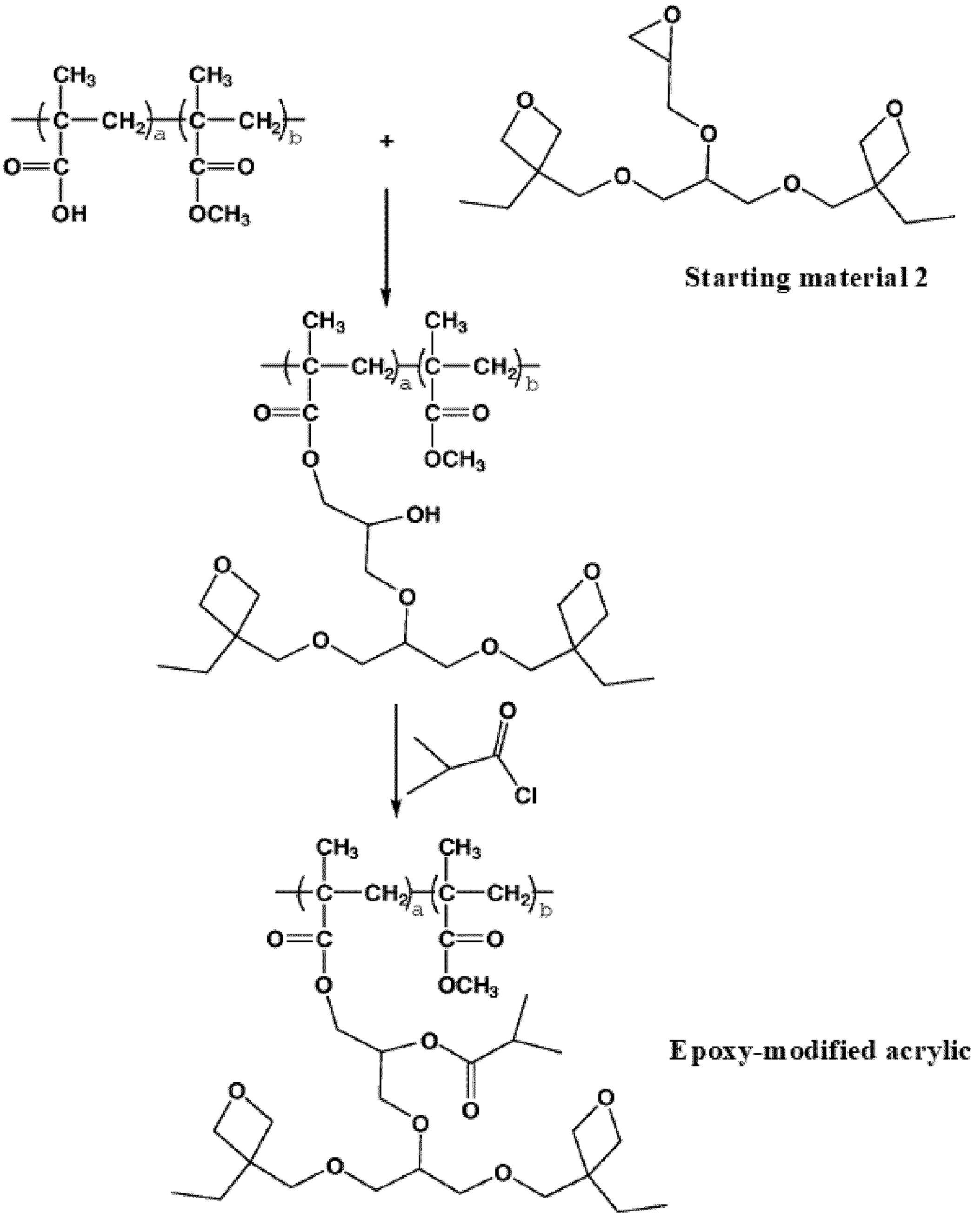

Starting material 2

Epoxy-modified acrylic resin 1

Example 1-1

Reaction 1: methacrylic acid monomer (43 g, 0.5 mol) was added to a three-necked flask, followed by toluene (200 mL), and heated at 70 ° C for dissolving. After dissolving triphenylphosphine (6.5 g) and starting material 2 (170 g, 0.50 mol) were added. After 8 h of reaction, the product was extracted, and distillated under reduced pressure, to obtain 200 g of intermediate 1.

Reaction 2: the intermediate 1 (85 g, 0.2 mol), isobutyryl chloride (21 g, 0.2 mol), toluene (200 mL), and sodium hydroxide (10 g) were sequentially added to a three-necked flask and reacted at 40 ° C for 12 h. After the completion of the reaction, the product was washed with water, extracted, and distillated under reduced pressure, to obtain 95 g of

intermediate 2.

Reaction 3: preparation of acrylic resin: the intermediate 2 (245 g, 0.5 mol) and propylene glycol methyl ether acetate (500 mL) were added to a reaction flask and pre-heated to 60 °C. Then MMA (50 g, 0.5 mol) and azodiisobutyronitrile (9 g, 3 wt%) were mixed and slowly dropwise added to the reaction flask over a feeding time of about 0.5 h. After the reactants were completely added, the reaction was continued for 6 h, to produce a white polymeric precipitate. The precipitate was filtered and dried, to obtain 275 g of epoxy-modified acrylic resin 1-1 with the same structure as the epoxy-modified acrylic resin 1, having a weight-average molecular weight of about $2\times10^4$, a molecular weight distribution of 1.50, an acid value of 0.4 mgKOH/g, and an epoxy equivalent of 255 g/mol.

The reaction process is as follows:

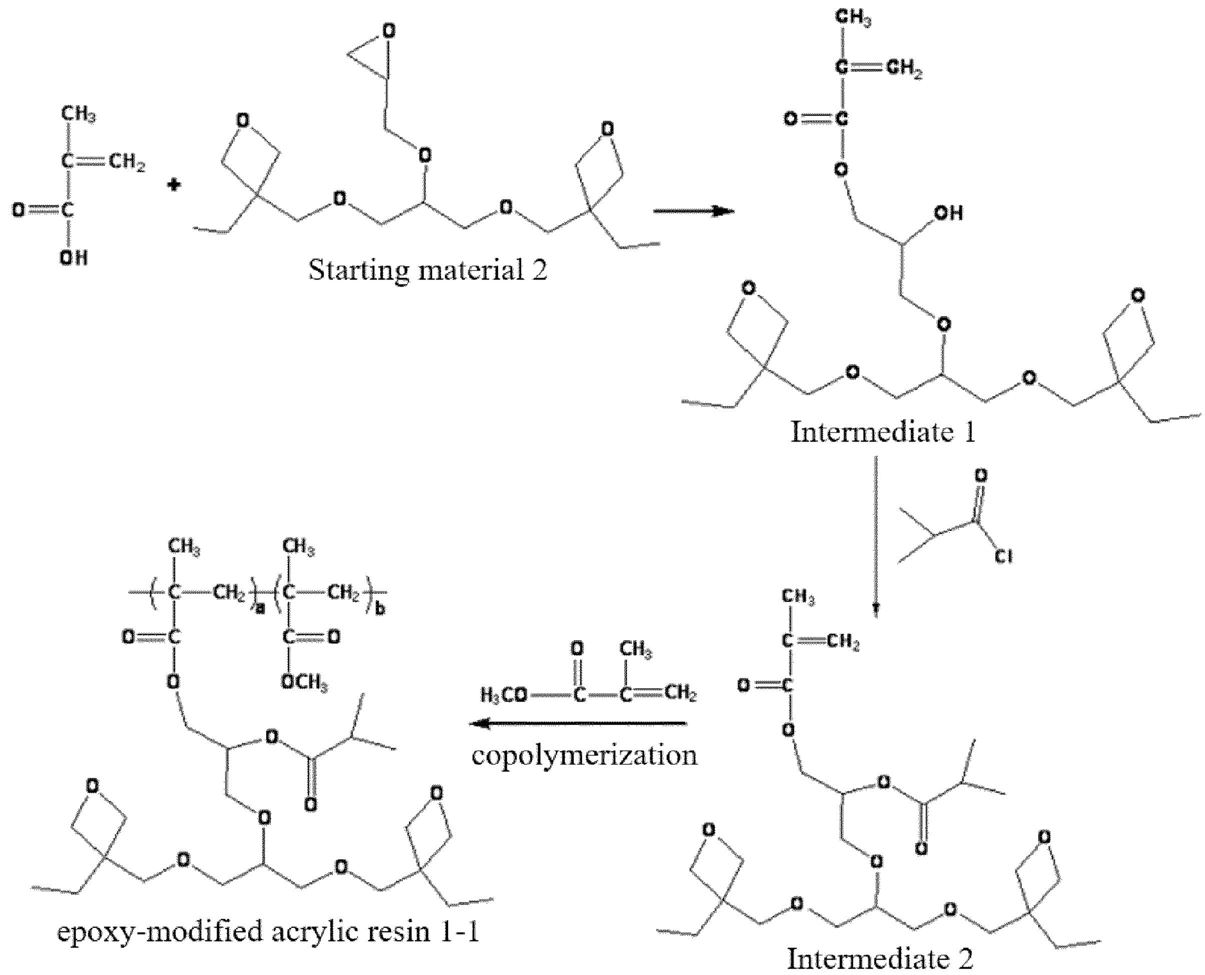


Example 2

Reaction 1: preparation of acrylic resin: the copolymerization reaction using hydroxyethyl acrylate monomer (HEA) and methyl methacrylate monomer (MMA) as starting materials: HEA (53 g, 0.5 mol) and propylene glycol methyl ether acetate (200 mL) were added to a reaction flask and heated to 60 ° C. A mixed solution of MMA (50 g, 0.5 mol) and azodiisobutyronitrile (3 g, 3 wt%) was slowly dropwise added to the reaction flask over a feeding time of 0.5 h. After the reactants were completely added, the reaction was continued for 6 h, to produce a white polymeric precipitate. The above precipitate was filtered and dried, to obtain 85 g of HEA-MMA acrylic resin.

Reaction 2: the HEA-MMA acrylic resin (100 g, containing 0.5 mol of hydroxyl group), succinic acid (59 g, 0.5 mol), acidic cationic exchange resin (5 g), and toluene (300 mL) were added to a three-necked flask, uniformly mixed, and heated for reaction. The reaction temperature was controlled at 120 °C. The reaction was completed until the amount of water to be fractionated approached the theoretical amount. The above reaction system was then washed with saturated brine, concentrated under reduced pressure, and bubbled, to obtain 130 g of intermediate 3.

Reaction 3: the intermediate 3 (100 g, containing 0.3 mol of carboxyl group) was added to a three-necked flask,

followed by toluene (200 mL), and heated at 70 ° C for dissolving. After dissolving, triphenylphosphine (4.5 g) and starting material 1 (52 g, 0.30 mol) were added. After 8 h of reaction, the product was extracted, and distillated under reduced pressure, to obtain 130 g of epoxy-modified acrylic resin.

Reaction 4: the epoxy-modified acrylic resin (100 g, 0.2 mol of hydroxyl group), epichlorohydrin (18.5 g, 0.2 mol), toluene (200 mL), and sodium hydroxide (10 g) were sequentially added to a three-necked flask and reacted at 40 ° C for 12 h. After the completion of the reaction, the product was washed with water, extracted, and distillated under reduced pressure, to obtain 110 g of epoxy-modified acrylic resin 2 having a weight-average molecular weight of $1.5\times10^5$, a molecular weight distribution index of 1.40, an acid value of 0.3 mgKOH/g, and an epoxy equivalent of 304 g/mol.

The reaction process is as follows:

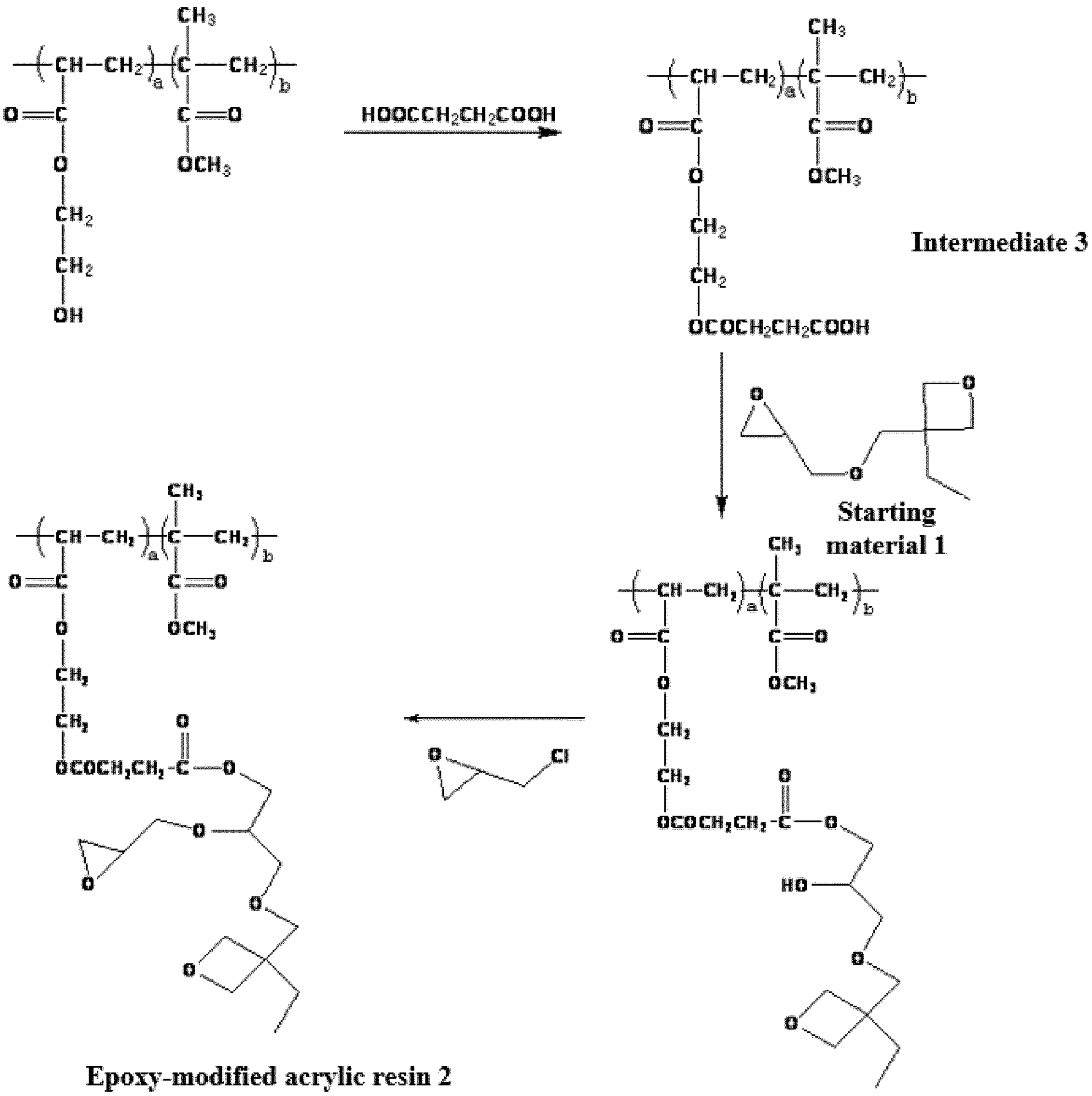


Example 2-1

The hydroxyethyl acrylate monomer (HEA) was used instead of HEA-MMA acrylic resin to complete the same reaction process as in steps 2 to 4 in Example 2, to obtain epoxy-grafted hydroxyethyl acrylate monomer, which was polymerized as follows: (92 g, 0.2 mol) epoxy-grafted hydroxyethyl acrylate monomer and propylene glycol methyl ether acetate (500 mL) were added to a reaction flask and pre-heated to 60 °C, then MMA (20 g, 0.2 mol) and AIBN (3.5 g, 3 wt%) were mixed and slowly dropwise added to the reaction flask over a feeding time of about 0.5 h. After the reactants were completely added, the reaction was continued for 6 h, to produce a white polymeric precipitate. The precipitate was filtered and dried, to obtain 100 g of epoxy-modified acrylic resin 2-1 with the same structure general formula as

the epoxy-modified acrylic resin 2, having a weight-average molecular weight of about $3.5\times10^4$, a molecular weight distribution of 1.55, an acid value of 0.3 mgKOH/g, and an epoxy equivalent of 298 g/mol.

Example 3

Reaction 1: preparation of acrylic resin: hydroxyethyl acrylate monomer (HEA) and methyl methacrylate monomer (MMA) were copolymerized as follows: HEA (53 g, 0.5 mol) and propylene glycol methyl ether acetate (200 mL) were added to a reaction flask and pre-heated to 60 °C, and then MMA (50 g, 0.5 mol) and azodiisobutyronitrile (3 g, 3 wt%) was mixed and slowly dropwise added to the reaction flask over a feeding time of 0.5 h. After the reactants were completely added, the reaction was continued for 6 h, to obtain a white polymeric precipitate. The above precipitate was filtered and dried, to obtain 85 g of HEA-MMA acrylic resin.

Reaction 2: the HEA-MMA acrylic resin (86 g, containing 0.2 mol of hydroxyl group), succinic anhydride (20 g, 0.2 mol), pyridine (3 g), and toluene (200 mL) were added to a three-necked flask, uniformly mixed, and heated for reaction. The reaction temperature was controlled at 120 ° C. The reaction was completed until the amount of water to be fractionated approached the theoretical amount. The above reaction system was then washed with saturated brine, concentrated under reduced pressure, and bubbled, to obtain 85 g of intermediate 4.

Reaction 3: the intermediate 4 (105 g, containing 0.2 mol of carboxyl group) was added to a three-necked flask, followed by toluene (200 mL), and heated at 70 ° C for dissolving. After dissolving triphenylphosphine (5 g) and starting material 2 (68 g, 0.20 mol) were added. After 8 h of reaction, the product was extracted, and distilled under reduced pressure, to obtain 150 g of epoxy-modified acrylic resin.

Reaction 4: the epoxy-modified acrylic resin (140 g, containing 0.2 mol of hydroxyl group), 3-(bromomethyl)oxetane (30 g, 0.2 mol), toluene (200 mL), and sodium hydroxide (5 g) were sequentially added to a three-necked flask, and reacted at 40 ° C for 12 h. After the completion of the reaction, the product was washed with water, extracted, and distilled under reduced pressure, to obtain 150 g of epoxy-modified acrylic resin 3 having a weight-average molecular weight of $8\times10^4$, a molecular weight distribution index of 2.05, an acid value of 0.6 mgKOH/g, and an epoxy equivalent of 352 g/mol.

The reaction process is as follows:

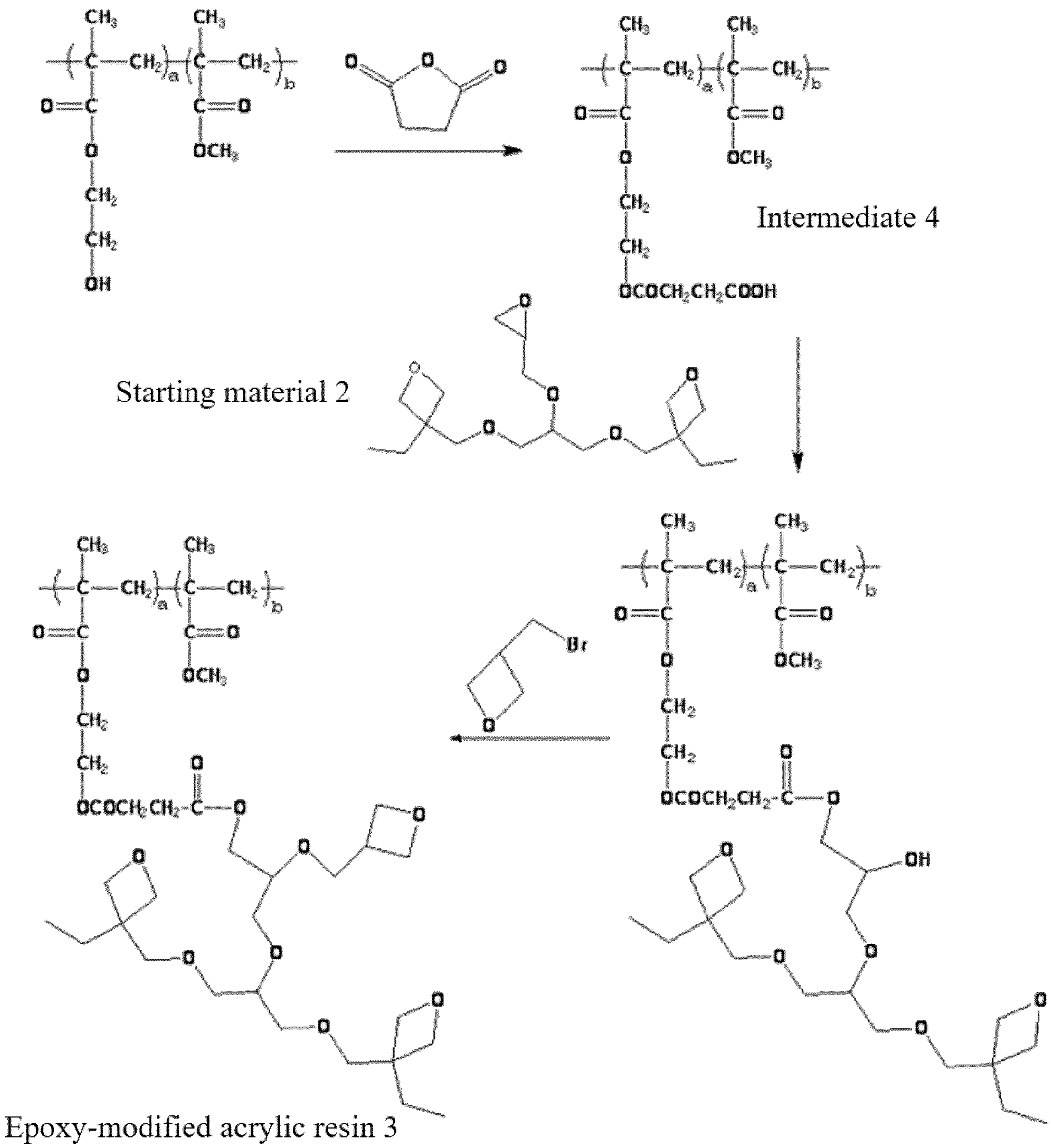


Example 3-1

The hydroxyethyl acrylate monomer (HEA) was used instead of HEA-MMA acrylic resin to complete the same reaction process as in steps 2 to 4 in Example 3, to obtain epoxy-grafted hydroxyethyl acrylate monomer, which was polymerized as follows: (135 g, 0.2 mol) epoxy-grafted hydroxyethyl acrylate monomer and propylene glycol methyl ether acetate (500 mL) were added to a reaction flask and pre-heated to 60 °C, then MMA (20 g, 0.2 mol) and AIBN (4.5g, 3 wt%) were mixed and slowly dropwise added to the reaction flask over a feeding time of about 0.5 h. After the reactants were completely added, the reaction was continued for 6 h, to produce a white polymeric precipitate. The precipitate was filtered and dried, to obtain 135 g of epoxy-modified acrylic resin 3-1 with the same structure general formula as the epoxy-modified acrylic resin 3, having a weight-average molecular weight of about $1.5\times10^4$, a molecular weight distribution of 2.20, an acid value of 0.6 mgKOH/g, and an epoxy equivalent of 347 g/mol.

Example 4

Reaction 1: 4-hydroxybutyl acrylate monomer (58 g, 0.4 mol) was added to a three-necked flask, followed by toluene (800 mL), and heated at 70 ° C for dissolving. After dissolving, sodium hydroxide (8.5 g) and starting material 3 (225 g, 0.20 mol) were added to the above three-necked flask. After 8 h of reaction, the product was extracted, and distilled under reduced pressure, to obtain 260 g of intermediate 5.

Reaction 2: the intermediate 5 (200 g, 0.15 mol), epichlorohydrin (28 g, 0.3 mol), toluene (500 mL), and sodium hydroxide (10 g) were sequentially added to a three-necked flask and reacted at 40 °C for 12 h. After the completion of the reaction, the product was washed with water, extracted, and distilled under reduced pressure, to obtain 200 g of intermediate 6.

Reaction 3: preparation of acrylic resin: the intermediate 6 (260 g, 0.2 mol) and propylene glycol methyl ether acetate (500 mL) were added to a reaction flask and heated to 60 ° C. Then MMA (20 g, 0.2 mol), butyl acrylate monomer (BA) (26 g, 0.2 mol), and azodiisobutyronitrile (9 g, 3 wt%) were mixed and slowly dropwise added to the reaction flask over a feeding time of 0.5 h. After the reactants were completely added, the reaction was continued for 6 h, to obtain a white polymeric precipitate. The above precipitate was filtered and dried to obtain 275 g of epoxy-modified acrylic resin 4 having a weight-average molecular weight of $1\times10^4$, a molecular weight distribution index of 2.00, an acid value of 0.8 mgKOH/g, and an epoxy equivalent of 381 g/mol.

The reaction process is as follows:

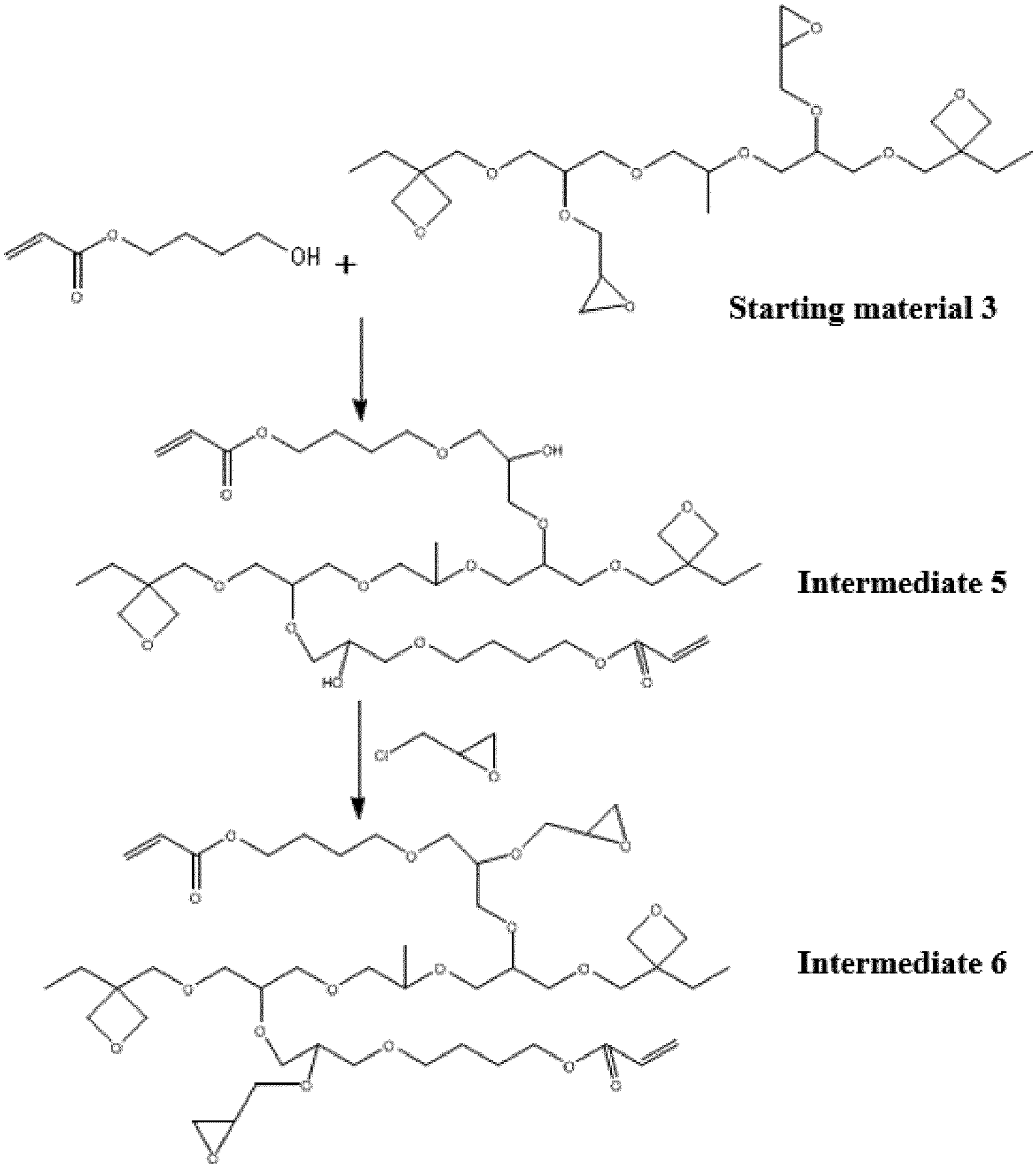

Starting material 3

Intermediate 5

Intermediate 6

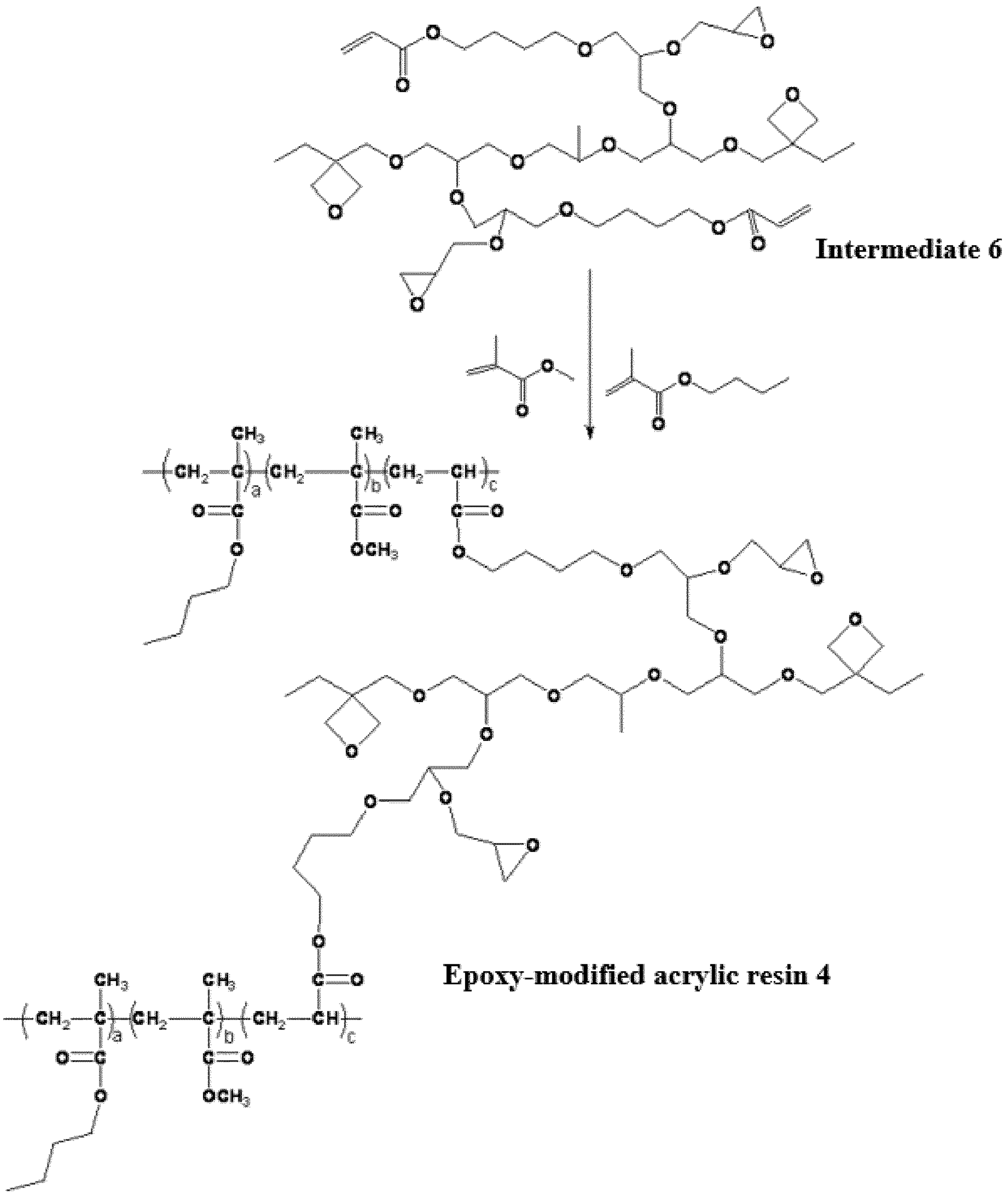

Intermediate 6

Epoxy-modified acrylic resin 4

Example 4-1

Reaction 1: preparation of acrylic resin: 4-hydroxybutyl acrylate monomer, methyl methacrylate monomer (MMA), and butyl acrylate monomer (BA) were copolymerized as follows: 4-hydroxybutyl acrylate (72 g, 0.5 mol) and propylene glycol methyl ether acetate (500 mL) were added to a reaction flask and pre-heated to 60 °C, and then MMA (50 g, 0.5 mol), BA (64 g, 0.5 mol), and AIBN (5.6 g, 3 wt%) were mixed and slowly dropwise added to the reaction flask over a feeding time of about 0.5 h. After the reactants were completely added, the reaction was continued for 4 h, to produce a white polymeric precipitate. The precipitate was filtered and dried, to obtain 150g of acrylic resin.

The acrylic resin prepared as above was used instead of 4-hydroxybutyl acrylate monomer to complete the same reaction process as in steps 1 to 2 in Example 4, to obtain epoxy-modified acrylic resin 4-1 with the same structure general formula as the epoxy-modified acrylic resin 4, having a weight-average molecular weight of about $0.8\times10^4$, a molecular weight distribution of 2.00, an acid value of 0.8 mgKOH/g, and an epoxy equivalent of 377 g/mol.

Example 5

Reaction 1: methacrylic acid monomer (43 g, 0.5 mol) was added to a three-necked flask, followed by toluene (200 mL), and heated at 70 ° C for dissolving. After dissolving, triphenylphosphine (4 g) and starting material 1 (86 g, 0.50 mol) were added. After 8 h of reaction, the product was extracted, and distillated under reduced pressure, to obtain 110 g of intermediate 7.

Reaction 2: the intermediate 7 (52 g, 0.2 mol), epichlorohydrin (18 g, 0.2 mol), toluene (150 mL), and sodium hydroxide (5 g) were sequentially added to a three-necked flask, and reacted at 40 °C for 12 h. After the completion of the reaction, the product was washed with water, extracted, and distillated under reduced pressure, to obtain 55 g of intermediate 8.

Reaction 3: hydroxyethyl acrylate (HEA) monomer (65 g, 0.5 mol) was added to a three-necked flask, followed by toluene (200 mL), and heated at 70 ° C for dissolving. After dissolving sodium hydroxide (4 g), starting material 2 (186 g, 0.50 mol) was added. After 8 h of reaction, the product was extracted, and distillated under reduced pressure, to obtain 140 g of intermediate 9.

Reaction 4: the intermediate 9 (60 g, 0.2 mol), epichlorohydrin (18 g, 0.2 mol), toluene (150 mL), and sodium hydroxide (5 g) were sequentially added to a three-necked flask, and reacted at 40 °C for 12 h. After the completion of the reaction, the product was washed with water, extracted, and distillated under reduced pressure, to obtain 65 g of intermediate 10.

Reaction 5: preparation of acrylic resin: the intermediate 8 (63 g, 0.2 mol) and propylene glycol methyl ether acetate (500 mL) were added and pre-heated to 60 ° C. MMA (20 g, 0.2mol), the intermediate 10 (72 g, 0.2 mol), and azodiisobutyronitrile (4.5 g, 3 wt%) were mixed to a reaction flask and slowly dropwise added to the reaction flask over a feeding time of about 0.5 h. After the reactants were completely added, the reaction was continued for 6 h, to produce a white polymeric precipitate. The above precipitate was filtered and dried to obtain 145 g of epoxy-modified acrylic resin 5 having a weight-average molecular weight of $5 \times 10^4$, a molecular weight distribution index of 1.40, an acid value of 0.3 mgKOH/g, and an epoxy equivalent of 205 g/mol.

The reaction process is as follows:

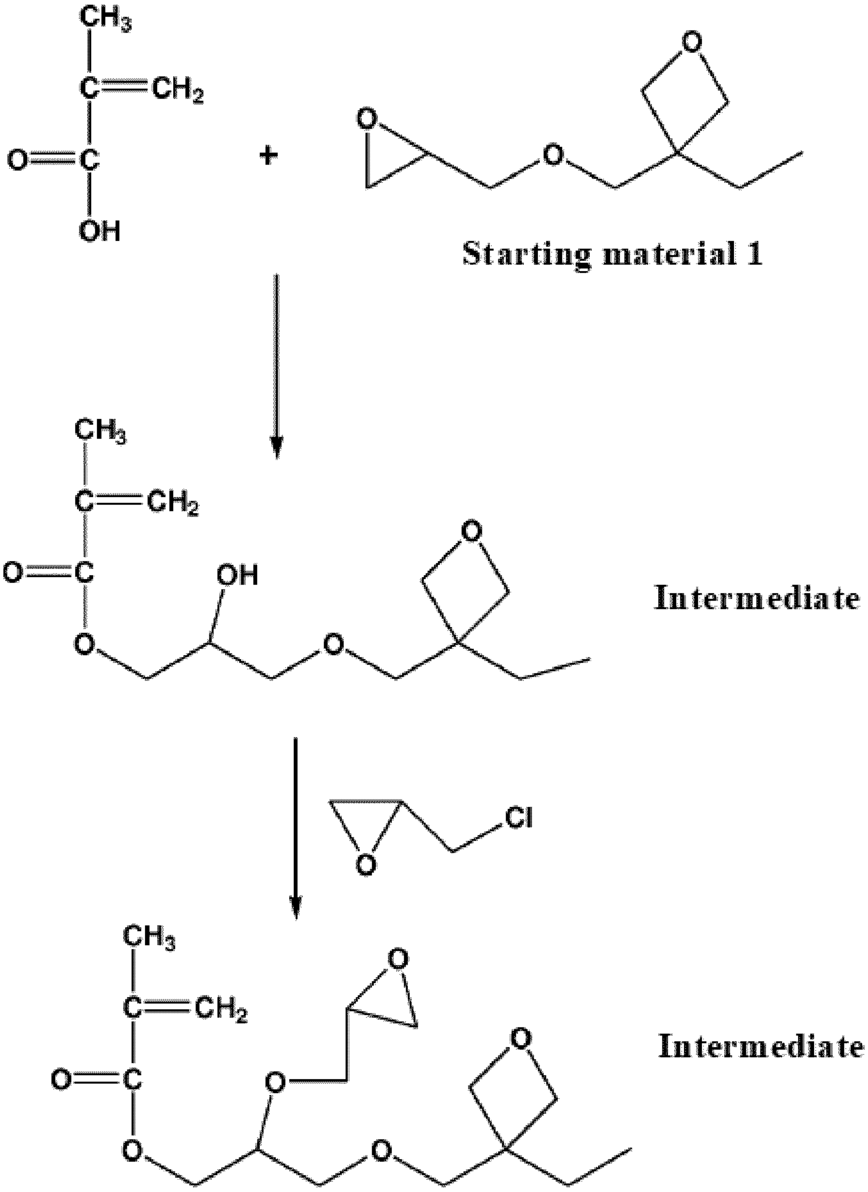

Starting material 1

Intermediate 7

Intermediate 8

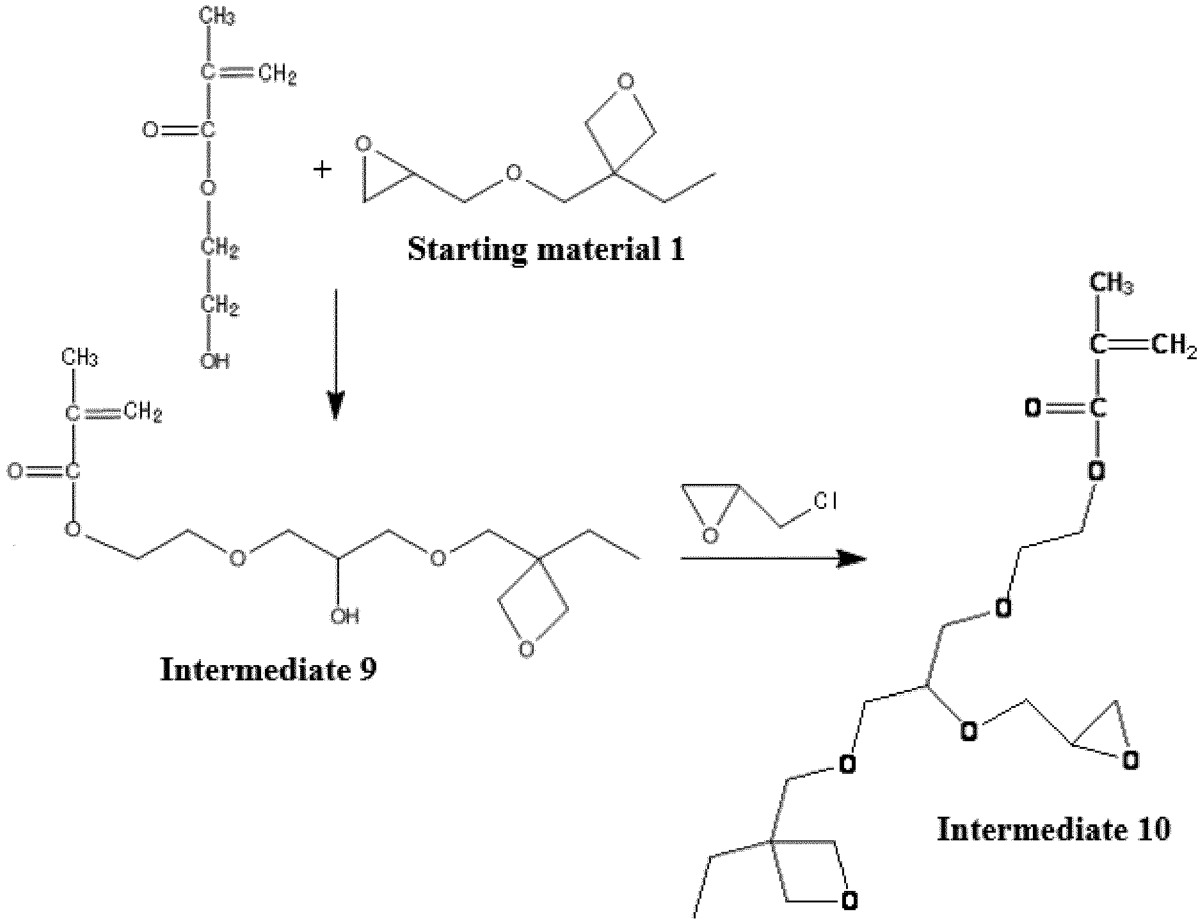

Starting material 1

Intermediate 9

Intermediate 10

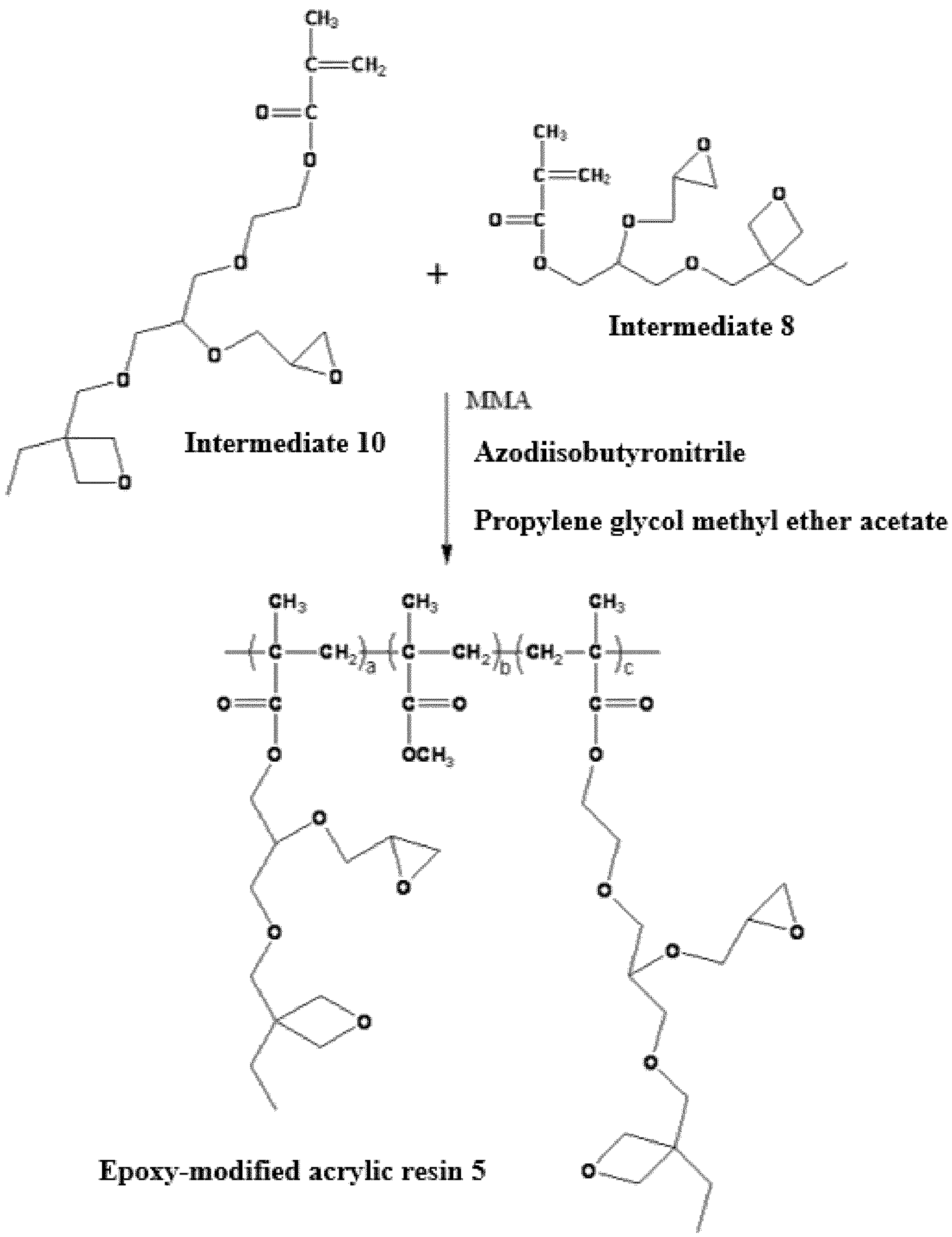


Example 6

In accordance with the above examples, the epoxy-modified acrylic resins 6 to 14 were prepared. Table 1 shows the corresponding relationship between starting materials and products. The epoxy-modified acrylic resins 6 to 14 have the following structure formulae, wherein

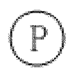

represents

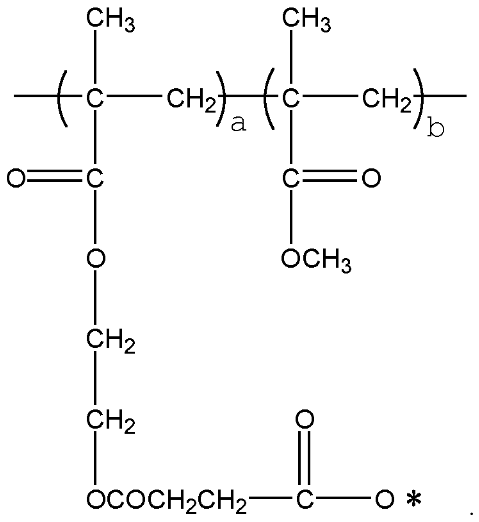

Table 1

| Starting material | Product | Weight-average molecular weight | Molecular weight distribution index | Acid value (mgKOH/g) |
|---|---|---|---|---|
| Starting material 4 | Epoxy-modified acrylic resin 6 | $1.5\times10^4$ | 1.20 | 0.5 |
| Starting material 6 | Epoxy-modified acrylic resin 7 | $5\times10^4$ | 1.45 | 0.4 |
| Starting material 11 | Epoxy-modified acrylic resin 8 | $1\times10^4$ | 1.20 | 0.5 |
| Starting material 5 | Epoxy-modified acrylic resin 9 | $5\times10^4$ | 1.45 | 0.3 |
| Starting material 9 | Epoxy-modified acrylic resin 10 | $3\times10^4$ | 1.30 | 0.4 |
| Starting material 10 | Epoxy-modified acrylic resin 11 | $2\times10^4$ | 1.25 | 0.3 |
| Starting material 12 | Epoxy-modified acrylic resin 12 | $1.5\times10^4$ | 1.30 | 0.7 |
| Starting material 7 | Epoxy-modified acrylic resin 13 | $4\times10^4$ | 1.40 | 0.2 |
| Starting material 8 | Epoxy-modified acrylic resin 14 | $2\times10^4$ | 1.35 | 0.3 |

The prepared products have the following structure formulae:

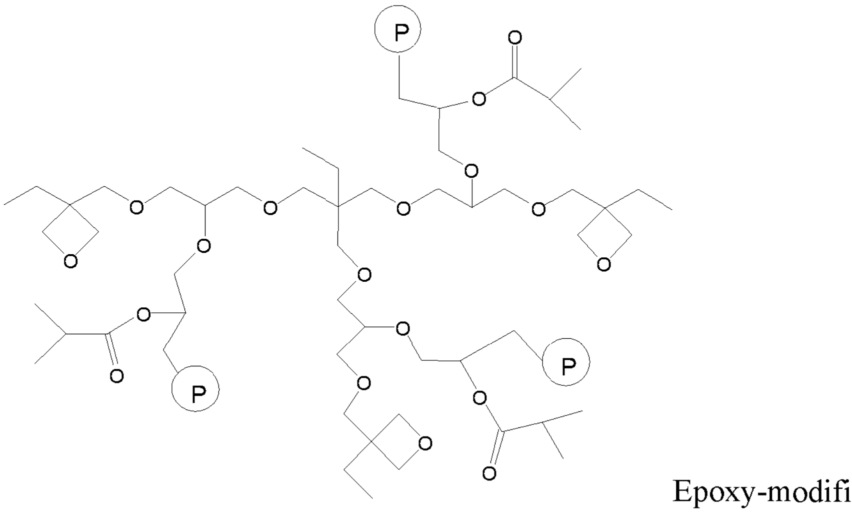

Epoxy-modified acrylic resin 6;

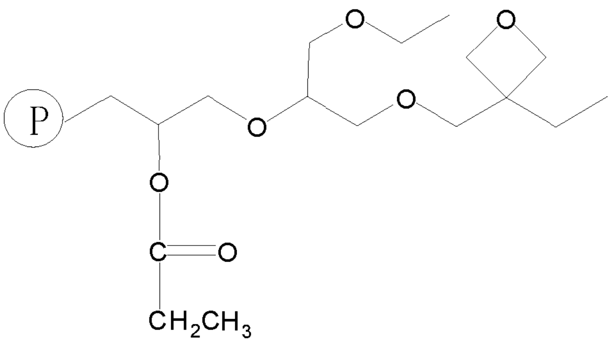

Epoxy-modified acrylic resin 7;

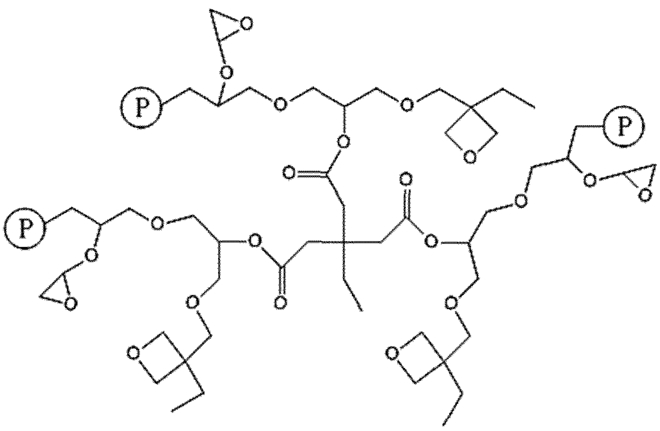

Epoxy-modified acrylic resin 8;

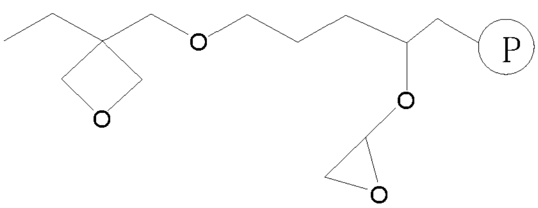

Epoxy-modified acrylic resin 9;

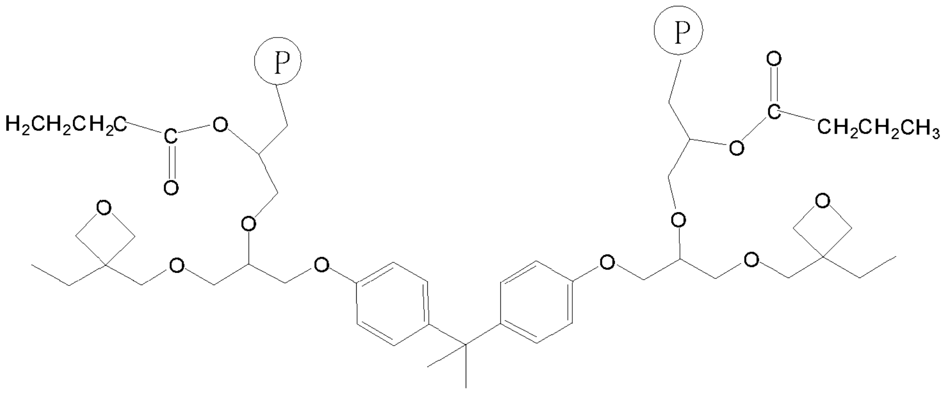

(Epoxy-modified acrylic resin 10);

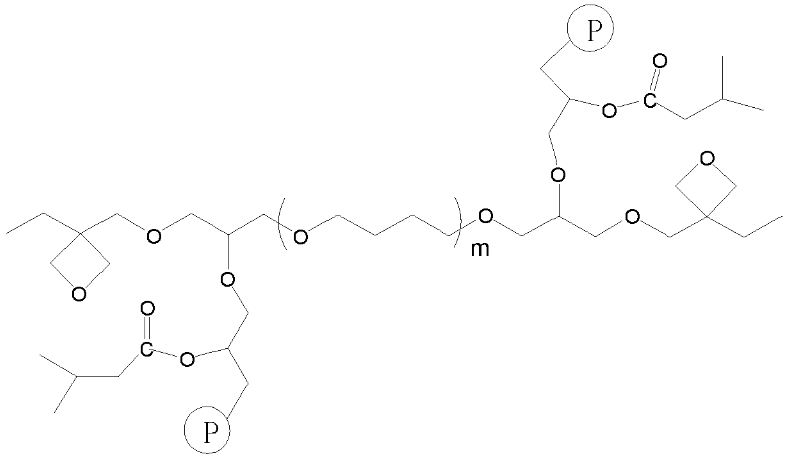

Epoxy-modified acrylic resin 11;

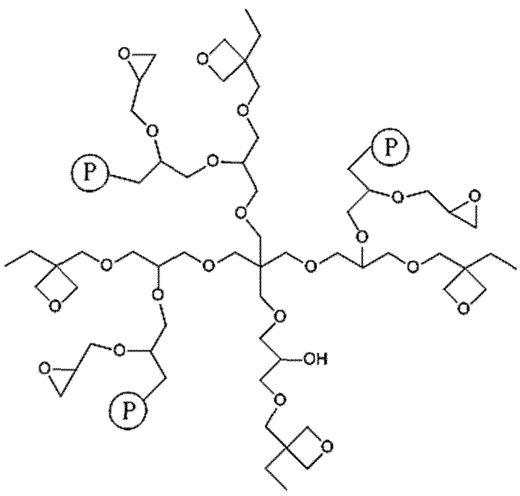

Epoxy-modified acrylic resin 12;

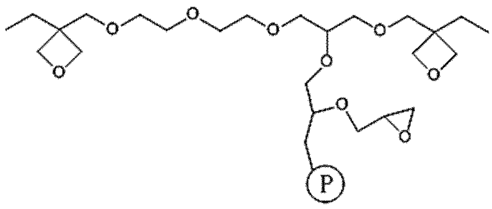

Epoxy-modified acrylic resin 13;

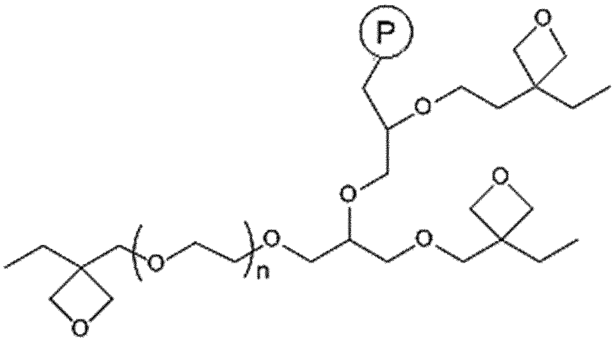

Epoxy-modified acrylic resin 14.

Example 7

Reaction 1: preparation of acrylic resin: in accordance with the reaction process of Reaction 1 in Example 4-1, acrylic acid monomer (AA), methacrylic acid monomer (MAA), and hydroxyethyl acrylate monomer (HEA) were copolymerized, to prepare AA-MAA-HEA acrylic resin.

Reactions 2 to 4: in accordance with Reactions 2 to 4 in Example 2, the epoxy-modified acrylic resin 15 was prepared, having a weight-average molecular weight of $3\times10^4$, a molecular weight distribution index of 1.40, and an acid value of 0.3 mgKOH/g.

The reaction process is as follows:

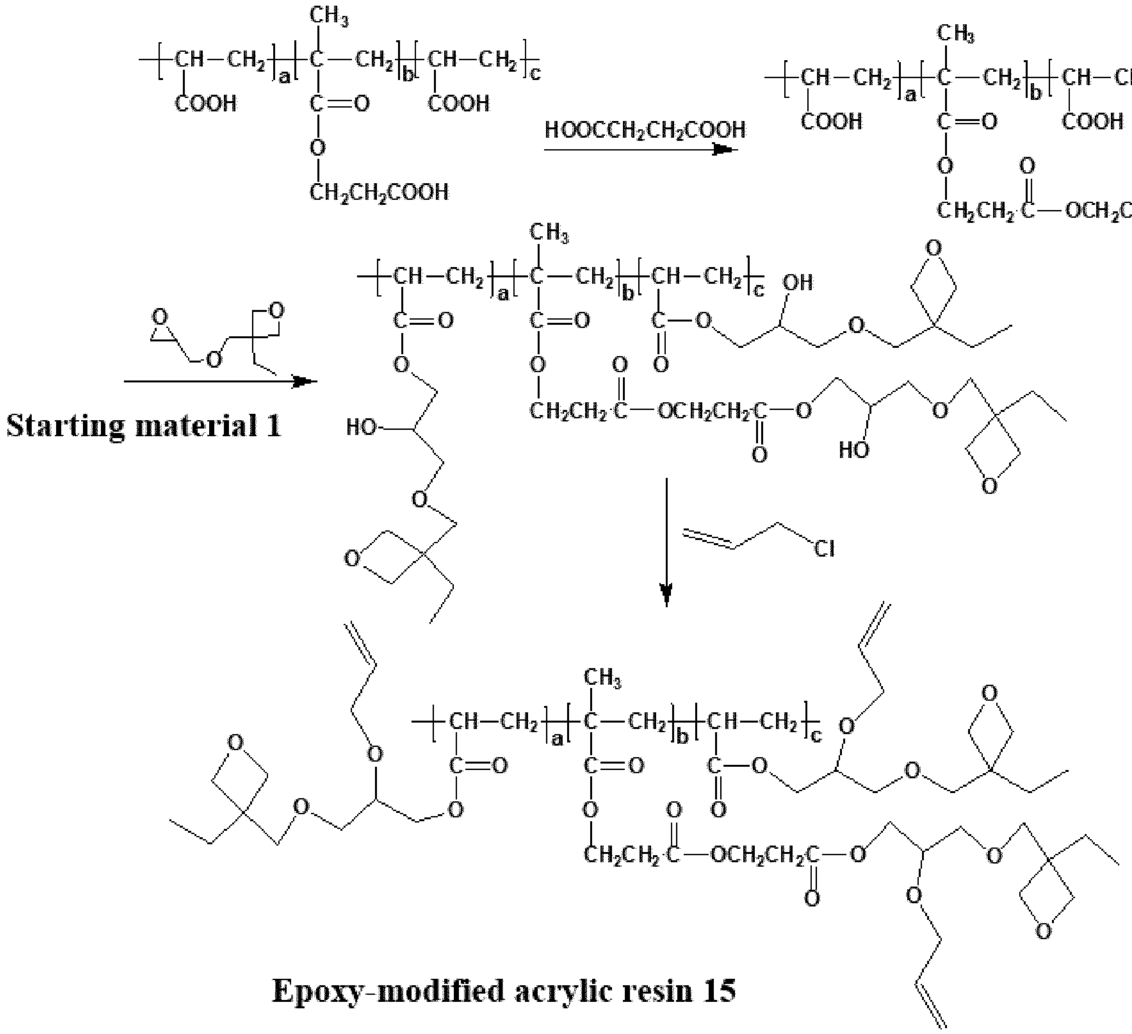


Starting material 1

Epoxy-modified acrylic resin 15

Example 8

Reaction 1: 3-hydroxymethyl-3-ethyloxetane (58 g, 0.5 mol), methyl methacrylate (50 g, 0.5 mol), and toluene (200 mL) were added to a four-necked flask equipped with a stirring device, a thermometer, a rectification column, and a water trap, heated under reflux to remove the moisture in the system. After cooling to approximately 60 °C, tetraethyl titanate (2.5 g) was added, and heated under reflux for reaction. The reflux ratio was adjusted to take out the methanol generated by the reaction. When the temperature at the top of the rectification column was raised to 110 ° C, the reaction was stopped. After cooling to 70 °C, water (10 g) was added and stirred for 1h and filtered while being hot. The filtrate was distillated under reduced pressure, to obtain 75 g of intermediate 11.

Reaction 2: preparation of acrylic resin: the intermediate 11 (92 g, 0.5 mol) and propylene glycol methyl ether acetate (200 mL) were added to a reaction flask and pre-heated to 60 °C. Then MMA (50 g, 0.5 mol) and azodiisobutyronitrile (5 g, 3 wt%) were mixed and slowly dropwise added to the reaction flask over a feeding time of 0.5 h. After the reactants were completely added, the reaction was continued for 6 h, to obtain a white polymeric precipitate. The above precipitate was filtered and dried to obtain 130 g of epoxy-modified acrylic resin 16 having a weight-average molecular weight of $3.5\times10^4$, a molecular weight distribution index of 1.15, an acid value of 0.2 mgKOH/g, and an epoxy equivalent of 300 g/mol.

The reaction process is as follows:

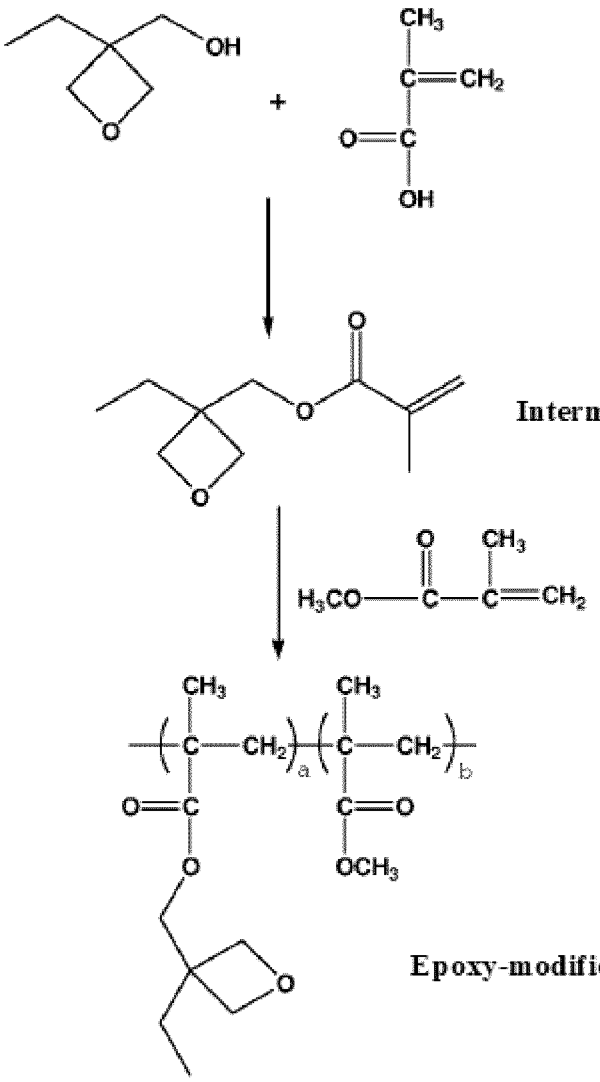

Intermediate 11

Epoxy-modified acrylic resin 16

**(II) Preparation and performance test for the composition containing epoxy-modified acrylic resin**

In order to better illustrate the application properties of the epoxy-modified acrylic resin prepared in this application, following compositions were prepared and their performances were tested in this application, as follows:

Example 9

The energy-curable composition containing epoxy-modified acrylic resin of the present invention comprised: 75 parts by weight of epoxy-modified acrylic resin 4 (having a molecular weight of $1\times10^4$, a viscosity of 2000 cps, and an acid value of 0.8 mgKOH/g), 25 parts by weight of polyester resin (manufactured by SK, Product No.: ES900), 4 parts by weight of auxiliary agent (leveling agent, manufactured by BYK, Product No.: BYK-333), and 6 parts by weight of photoinitiator (triarylsulfonium salt, manufactured by CUREASE, Product No.: Easepi6976).

Example 10

The energy-curable composition containing epoxy-modified acrylic resin of the present invention comprised: 30 parts by weight of epoxy-modified acrylic resin 4 (having a molecular weight of $1\times10^4$, a viscosity of 2000 cps, and an acid value of 0.8 mgKOH/g), 60 parts by weight of polyester resin (manufactured by SK, Product No.: ES900), 4 parts by weight of auxiliary agent (leveling agent, manufactured by BYK, Product No.: BYK-333), and 6 parts by weight of photoinitiator (triarylsulfonium salt, manufactured by CUREASE, Product No.: Easepi6976).

Example 11

The energy-curable composition containing epoxy-modified acrylic resin of the present invention comprised: 60 parts by weight of epoxy-modified acrylic resin 4 (having a molecular weight of $1\times10^4$, a viscosity of 2000 cps, and an acid value of 0.8 mgKOH/g), 30 parts by weight of polyester resin (manufactured by SK, Product No.: ES900), 4 parts by weight of auxiliary agent (leveling agent, manufactured by BYK, Product No.: BYK-333), and 6 parts by weight of photoinitiator (triarylsulfonium salt, manufactured by CUREASE, Product No.: Easepi6976).

Example 12

The energy-curable composition containing epoxy-modified acrylic resin of the present invention comprised: 60 parts by weight of epoxy-modified acrylic resin 4 (having a molecular weight of $1\times10^4$, a viscosity of 2000 cps, and an acid value of 0.8 mgKOH/g), 30 parts by weight of alkyd resin (manufactured by ChangZhou FangXin Chemical Material Co., Ltd., Product No.: FX-1270B), 4 parts by weight of auxiliary agent (leveling agent, manufactured by BYK, Product No.: BYK-333), and 6 parts by weight of photoinitiator (triarylsulfonium salt, manufactured by CUREASE, Product No.: Easepi6976).

Example 13

The energy-curable composition containing epoxy-modified acrylic resin of the present invention comprised: 60 parts by weight of epoxy-modified acrylic resin 4 (having a molecular weight of $1\times10^4$, a viscosity of 2000 cps, and an acid value of 0.8 mgKOH/g), 30 parts by weight of polyester resin (manufactured by SK, Product No.: ES900), 4 parts by weight of auxiliary agent (leveling agent, manufactured by BYK, Product No.: BYK-333), and 6 parts by weight of thermal initiator (manufactured by Tronly, Product No.: TR-TAG-50101).

Example 14

The energy-curable composition containing epoxy-modified acrylic resin of the present invention comprised: 60 parts by weight of epoxy-modified acrylic resin 4 (having a molecular weight of $1\times10^4$, a viscosity of 2000 cps, and an acid value of 0.8 mgKOH/g), 30 parts by weight of alkyd resin (manufactured by ChangZhou FangXin Chemical Material Co., Ltd., Product No.: FX-1270B), 4 parts by weight of auxiliary agent (leveling agent, manufactured by BYK, Product No.: BYK-333), and 6 parts by weight of photoinitiator (triarylsulfonium salt, manufactured by CUREASE, Product No.: Easepi6976).

Example 15

The energy-curable composition containing epoxy-modified acrylic resin of the present invention comprised: 60 parts by weight of epoxy-modified acrylic resin 1 (having a molecular weight of $5\times10^4$, a viscosity of 3000 cps, and an acid value of 0.4 mgKOH/g), 30 parts by weight of alkyd resin (manufactured by ChangZhou FangXin Chemical Material Co., Ltd., Product No.: FX-1270B), 4 parts by weight of auxiliary agent (leveling agent, manufactured by BYK, Product No.: BYK-333), and 6 parts by weight of photoinitiator (triarylsulfonium salt, manufactured by CUREASE, Product No.: Easepi6976).

Example 16

The energy-curable composition containing epoxy-modified acrylic resin of the present invention comprised: 60 parts by weight of epoxy-modified acrylic resin 16 (having a molecular weight of $3.5\times10^4$, a viscosity of 2000 cps, and an acid value of 0.2 mgKOH/g), 30 parts by weight of alkyd resin (manufactured by ChangZhou FangXin Chemical Material Co., Ltd., Product No.: FX-1270B), 4 parts by weight of auxiliary agent (leveling agent, manufactured by BYK, Product No.: BYK-333), and 6 parts by weight of photoinitiator (triarylsulfonium salt, manufactured by CUREASE, Product No.: Easepi6976).

Comparative Example 1

The comparative composition comprised: 60 parts by weight of acrylic resin (prepared in accordance with Reaction 1 in Example 1, having a molecular weight of $4.8\times10^4$, a viscosity of 3500 cps, and an acid value of 5 mgKOH/g), 30 parts by weight of polyester resin (manufactured by SK, Product No.: ES900), 15 parts by weight of diluting agent (butanone), and 4 parts by weight of auxiliary agent (leveling agent, manufactured by BYK, Product No.: BYK-333).

Comparative Example 2

The comparative composition comprised: 60 parts by weight of acrylic resin (prepared in accordance with Reaction 1 in Example 1, having a molecular weight of $4.8\times10^4$, a viscosity of 3500 cps, and an acid value of 5 mgKOH/g), 30 parts by weight of alkyd resin (manufactured by ChangZhou FangXin Chemical Material Co., Ltd., Product No.: FX-1270B), 15 parts by weight of diluting agent (butanone), and 4 parts by weight of auxiliary agent (leveling agent, manufactured by BYK, Product No.: BYK-333).

Table 2 shows the contents of the main components in the above Examples 9 to 16 as well Comparative Examples 1 and 2 (parts by weight).

Table 2

| Components | Example | | | | | | | | Comparative Example | |
|---|---|---|---|---|---|---|---|---|---|---|
| | 9 | 10 | 11 | 12 | 13 | 14 | 15 | 16 | 1 | 2 |
| Epoxy-modified acrylic resin 1 | 0 | 0 | 0 | 0 | 0 | 0 | 60 | 0 | 0 | 0 |
| Epoxy-modified acrylic resin 4 | 75 | 30 | 60 | 60 | 60 | 60 | 0 | 0 | 0 | 0 |
| Epoxy-modified acrylic resin 16 | 0 | 0 | 0 | 0 | 0 | 0 | 0 | 60 | 0 | 0 |
| Polyester resin | 25 | 60 | 30 | 0 | 30 | 0 | 0 | 0 | 30 | 0 |
| Acrylic resin | 0 | 0 | 0 | 0 | 0 | 0 | 0 | 0 | 60 | 60 |
| Alkyd resin | 0 | 0 | 0 | 30 | 0 | 30 | 30 | 30 | 0 | 30 |
| Auxiliary agent | 4 | 4 | 4 | 4 | 4 | 4 | 4 | 4 | 4 | 4 |
| Photo initiator | 6 | 6 | 6 | 6 | 0 | 6 | 6 | 6 | 0 | 0 |
| Thermal initiator | 0 | 0 | 0 | 0 | 6 | 0 | 0 | 0 | 0 | 0 |
| Solvent | 0 | 0 | 0 | 0 | 0 | 0 | 0 | 0 | 15 | 15 |

**Performance test**

After the above compositions containing epoxy-modified acrylic resin were formed into films, the performance evaluation test was performed as follows:
Sample preparation method: the above energy-curable compositions containing epoxy-modified acrylic resin were dispersed at high speed for 30 min to be uniformly mixed.

The tinplate was used as the substrate material, sanded with sand paper to remove the oil stains thereon, and washed with deionized water. After drying off, it was spray painted with a coating thickness of $25\pm5$ μm, and tested for their performances after complete curing.

Test method:

(1) Hardness test: in accordance with China National Standard GB/T6739-86, a set of drawing pencils with a hardness of 6B to 6H was prepared, and the pencil hardness of the paint film was manually measured. The paint film plate was horizontally placed on the table top. A pencil was held by hand at an angle of 45°, pressed hard by about 1 cm on the paint film surface at a uniform speed, and a scratch was left on the paint film. The scratching was repeated by 5 times for the pencil of the same hardness. If there were two or more scratches that do not reach the substrate of the sample plate, a pencil with a next higher hardness was used instead, until the paint film was found to be scratched to have two or more scratches. The pencil hardness of the paint film was represented by the next pencil hardness lower than this hardness. The test results are shown in Table 3.
(2) Adhesion test: in accordance with China National Standard GB/T9286-1998, 6 parallel cut marks were cut on the paint film by a knife through the entire depth of the film; then the same 6 cut marks perpendicular to the former were cut, to form many small squares. A translucent pressure-sensitive adhesive tape with a width of 25 mm was then pasted on the entire squares of the cut marks. The tape was yanked, and compared with the standard to determine the level of the paint film adhesion. The test results were shown in Table 3. A smaller adhesion level indicated a better adhesion.
(3) Flexibility test: in accordance with China National Standard GB1731, the composition was applied on PET. After

being cured, with the paint film facing up, the test sample was tightly pressed on the shaft rod of the required diameter and bent around the rod. After bending, two thumbs should be symmetrical to the center line of the shaft rod. Whether the paint film had damage phenomena such as textures, cracks, and peeling was observed visually or by 4X magnifier. The flexibility of the paint film was represented by the minimal diameter of the shaft rod that did not result in the paint film damage when the sample plate was bent on the shaft rods of different diameters. A smaller diameter of the shaft rod indicated a better flexibility. The test results were shown in Table 3.

(4) Curing time test:

Curing operations for the examples: the composition was spray painted on the tinplate to prepare a sample with a thickness of 200 μm, and the sample was irradiated with ultraviolet light at room temperature. The starting time was the time when the sample was irradiated with the light, the reaction completion time was the time when the surface of the sample was completely cured, and the difference therebetween was the time required for the complete curing of the sample. The ultraviolet wavelength was 365 nm, and the irradiation intensity was 20 mw/cm$^2$. When the surface of the cured film was touched by a finger and no fingerprint trace was left on the surface, the film was considered to be completely cured. When the sample was cured by thermal radiation, the temperature was 120°C. When the sample was cured by electron beam radiation, the electron beam energy was lower than 300 KeV.

Curing operations for the comparative examples: the composition was spray painted on the tinplate to prepare a sample with a thickness of 200 μm. It was placed in a conventional laboratory environment and naturally dried. For the curing standard, when the surface of the cured film was touched by a finger and no fingerprint trace was left on the surface, the film was considered to be completely cured. The test results were shown in Table 3.

(5) VOC test: 0.2 g of sample was weighed and applied on a weighed aluminum plate, and weighted; the aluminum plate applied with the sample was cured, and the cured sample was cooled at room temperature for 15 min, and weighted; the cured and cooled sample was placed in a ventilated oven at 110 °C and dried for 1 h, then placed in a desiccator to be cooled to room temperature, and weighted. The test results were shown in Table 3.

$$\text{Processing volatiles} = 100[(B-C)/(B-A)];\ \text{Potential volatiles} = 100[(C-D)/(B-A)];$$

$$\text{Total volatiles\%} = \text{processing volatiles\%} + \text{potential volatiles\%},$$

Where:

A -- weight of aluminum plate, g;
B -- weight of sample and aluminum plate, g;
C -- weight of sample and aluminum plate after being cured, g;
D -- weight of sample and aluminum plate after being cured by heating, g.

Table 3

| Item | Example | | | | | | | | Comparative Example | |
|---|---|---|---|---|---|---|---|---|---|---|
| | 9 | 10 | 11 | 12 | 13 | 14 | 15 | 16 | 1 | 2 |
| Adhesion | Level 0 | Level 2 | Level 0 | Level 0 | Level 0 | Level 0 | Level 0 | Level 0 | Level 4 | Level 4 |
| Hardness | 4 H | 3 H | 4 H | 4 H | 4 H | 4 H | 4 H | 4 H | 2 H | 2 H |
| Flexibility (mm) | 1.0 | 2.0 | 1.5 | 1.5 | 1.5 | 1.5 | 1.0 | 1.0 | 2.5 | 2.5 |
| Curing time | 5min | 15min | 5min | 5min | 5min | 5min | 5min | 5min | 48h | 48h |
| VOC emission (%) | 0 | 0 | 0 | 0 | 0 | 0 | 0 | 0 | 18 | 18 |

It can be seen from Table 3 that, the above energy-curable composition containing the epoxy-modified acrylic resin of the present invention has better adhesion, high hardness, excellent flexibility, and high curing rate. Moreover, since the epoxy-modified acrylic resin used in the present invention has good compatibility with other components in the formulation system, solvents and active monomers may be eliminated during use, the real zero VOC emission can be achieved and it has broad commercial application prospects.

**(III) Application of the energy-curable composition containing the epoxy-modified acrylic resin in the fields of ink, paint, and adhesive**

Example 17 <Energy-curable paint>

The acrylic resin (prepared in accordance with Reaction 1 in Example 1, having a molecular weight of $4.8\times10^4$, a viscosity of 3500 cps, and an acid value of 5 mgKOH/g), epoxy-modified acrylic resin 1, and photoinitiator (triarylsulfonium salt, manufactured by CUREASE, Product No.: Easepi6976) were added to a stirring tank, stirred at room temperature at high speed until they were uniformly mixed. Then titanium pigment was added and stirred for 4 h, followed by the polymeric dispersant (BYK110 dispersant, Germany), stirred for 2 h, uniformly mixed, and filtered, to prepare the energy-curable paint.

Table 4 shows the contents of the components in the above example and comparative example (parts by weight).

Table 4

| Component | Example | Comparative Example |
|---|---|---|
| Epoxy-modified acrylic resin 1 | 40 | 0 |
| Acrylic resin | 20 | 60 |
| Polymeric dispersant | 5 | 5 |
| Photoinitiator | 10 | 10 |
| Titanium pigment | 20 | 20 |
| Solvent | 0 | 15 |

The energy-curable paints prepared in the above example and comparative example were uniformly applied on the surfaces of the metal substrates (cold-rolled steel sheet, galvanized sheet, or silicon steel sheet). Then the metal substrates applied with the paints were placed in the Dymax curing device and cured using medium-pressure mercury lamp for 60 s (3.3 $J/cm^2$UVA). After being cured, they were tested for their performances using the same test method as above. The test results were shown in Table 5.

Table 5

| Sample | Adhesion | Hardness | VOC emission (%) |
|---|---|---|---|
| Example | Level 0 | 4 H | 0 |
| Comparative Example | Level 4 | 2 H | 18 |

It can be seen from Table 5 above that, the energy-curable paint provided by the present invention has high hardness, good adhesion, and no VOC emission.

Example 18 <Energy-curable ink>

Under the condition of no light, polyester resin (manufactured by SK, Product No.: ES900), epoxy-modified acrylic resin 2 or acrylic resin (purchased from BASF Co. Ltd., Product No.: Joncryl 678), and photoinitiator (triarylsulfonium salt, manufactured by CUREASE, Product No.: Easepi6976) were added to a stirring tank respectively, stirred at room temperature at high speed until they were uniformly mixed. Then the filler (Celite) was added and stirred for 4 h, followed by the auxiliary agent (BYK381, Germany), and stirred for 2 h. After being grinded evenly, the insoluble matters were filtered off using a PTFE filter with a pore size of 0.45 μm, to prepare the energy-curable ink.

Table 6 shows the contents of the components in the above example and comparative example (parts by weight).

Table 6

| Component | Example | Comparative Example |
|---|---|---|
| Epoxy-modified acrylic resin 2 | 40 | 0 |
| Acrylic resin | 0 | 40 |

(continued)

| Component | Example | Comparative Example |
|---|---|---|
| Polyester resin | 20 | 20 |
| Photoinitiator | 10 | 10 |
| Auxiliary agent | 5 | 5 |
| Filler | 20 | 20 |
| Solvent | 0 | 15 |

The energy-curable inks prepared in the above example and comparative example were uniformly applied on PET. Then they were placed in the Dymax curing device, and cured using medium-pressure mercury lamp for 60 s (3.3 $J/cm^2$UVA). After being cured, they were tested for their performances using the same test method as above. The test results were shown in Table 7.

Table 7

| Sample | Adhesion | Flexibility (mm) | VOC emission (%) |
|---|---|---|---|
| Example | Level 0 | 2.0 | 0 |
| Comparative Example | Level 4 | 2.5 | 18 |

It can be seen from Table 7 above that, the energy-curable ink provided by the present invention has good adhesion, good flexibility, and no VOC emission.

Example 19 <Energy-curable adhesive>

Under the condition of no light, phenolic resin (purchased from DKSH Company, Switzerland, Product No.: 2402), epoxy-modified acrylic resin 3 or acrylic resin (purchased from BASF Co. Ltd., Product No.: Joncryl 678), and photoinitiator (triarylsulfonium salt, manufactured by CUREASE, Product No.: Easepi6976) were added to a stirring tank respectively, stirred at room temperature at high speed until they were uniformly mixed. Then the filler (talc powder) was added and stirred for 4 h, followed by the auxiliary agent (silane coupling agent), and stirred for 2 h. After being grinded evenly, the product was deformed, to prepare the energy-curable adhesive.

Table 8 shows the contents of the components in the above example and comparative example (parts by weight).

Table 8

| Component | Example | Comparative Example |
|---|---|---|
| Epoxy-modified acrylic resin 3 | 40 | 0 |
| Acrylic resin | 0 | 40 |
| Phenolic resin | 20 | 20 |
| Photoinitiator | 10 | 10 |
| Silane coupling agent | 5 | 5 |
| Filler | 20 | 20 |
| Solvent | 0 | 15 |

The energy-curable adhesives prepared in the above example and comparative example were uniformly applied on PET. Then they were placed in the Dymax curing device and cured using medium-pressure mercury lamp for 60 s (3.3 $J/cm^2$UVA). After being cured, they were tested for their performances, wherein the test method for the adhesion was the same as above, and the tensile shear strength was measured in accordance with GB/T 7124-2008. The test results were shown in Table 9.

Table 9

| Sample | Adhesion | Tensile shear strength (MPa) | VOC emission (%) |
|---|---|---|---|
| Example | Level 0 | 4.18 | 0 |
| Comparative Example | Level 4 | 3.91 | 18 |

It can be seen from Table 9 above that, the energy-curable adhesive provided by the present invention has good adhesion, strong tensile shear force, and no VOC emission.

The above descriptions are merely preferred embodiments of the present invention and are not intended to limit the present invention. For those skilled in the art, the present invention may have various modifications and changes. Any modification, equivalent replacement, and improvement made within the spirit and principle of the present invention shall be included within the scope of the present invention.

## Claims

1. An epoxy-modified acrylic resin, **characterized in that** the branched chain of at least one repeat unit in the acrylic resin is grafted with one or more oxetanyl groups, each of the oxetanyl groups has a structure represented by general formula (I), general formula (II), or general formula (III), respectively:

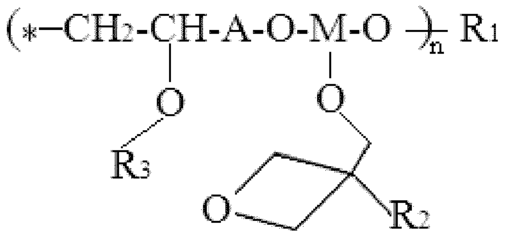

(I),

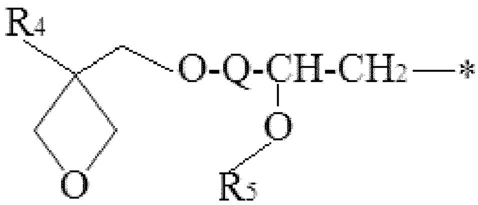

(II),

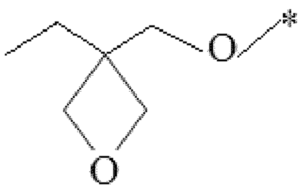

(III),

wherein $R_1$ represents $C_1$-$C_{40}$ linear or branched n-valent alkyl, $C_2$-$C_{30}$ n-valent alkenyl, or $C_6$-$C_{40}$ n-valent aryl, any one of $-CH_2-$ in $R_1$ may be substituted by oxygen atom, ester group, or

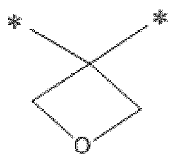

,

any one of hydrogen atoms in $R_1$ may be substituted by alkyl, halogen, or nitro, and n is selected from an integer of 1 to 12;

$R_2$ and $R_4$ independently represent hydrogen, halogen, nitro, $C_1$-$C_{30}$ linear or branched alkyl, $C_3$-$C_{30}$ cycloalkyl or substituted cycloalkyl, $C_2$-$C_{15}$ alkenyl, or $C_6$-$C_{30}$ aryl, respectively, any one of - $CH_2$- in $R_2$ and $R_4$ may be substituted by oxygen atom or -COO-, and any one of hydrogen atoms in $R_2$ and $R_4$ may be substituted by alkyl, halogen, or nitro;

$R_3$ and $R_5$ independently represent $C_1$-$C_{30}$ linear or branched alkyl, $C_3$-$C_{30}$ cycloalkyl or substituted cycloalkyl, $C_6$-$C_{30}$ aryl, respectively, any one of -$CH_2$- in $R_3$ and $R_5$ may be substituted by oxygen atom or -COO-, and any one of hydrogen atoms in $R_3$ and $R_5$ may be substituted by alkyl, halogen, or nitro;

A represents $C_1$-$C_{20}$ linear or branched alkylene, any one of -$CH_2$- in A may be substituted by oxygen atom or -COO-, and any one of hydrogen atoms in A may be substituted by alkyl, halogen, or nitro;

M represents 3-valent $C_1$-$C_{20}$ linear or branched alkyl, wherein any one of -$CH_2$- in M may be substituted by oxygen atom, -COO-, or

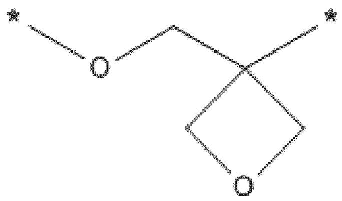

,

and any one of hydrogen atoms in M may be substituted by alkyl, halogen, or nitro;

Q represents $C_1$-$C_{20}$ linear or branched alkylene, wherein any one of -$CH_2$- in Q may be substituted by oxygen atom, -COO-, or

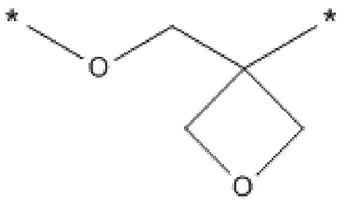

,

and any one of hydrogen atoms in Q may be substituted by alkyl, halogen, or nitro.

2. The epoxy-modified acrylic resin according to claim 1, **characterized in that** $R_3$ and $R_5$ independently represent

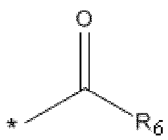 , 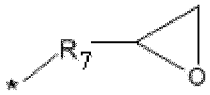 , 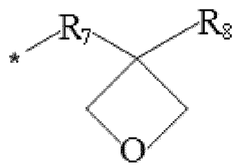 ,

or

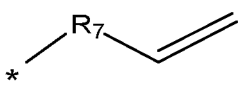

,

respectively, $R_6$ represents $C_1$-$C_{30}$ linear or branched alkyl, $C_3$-$C_{30}$ cycloalkyl or substituted cycloalkyl, or $C_6$-$C_{30}$ aryl, wherein any one of -$CH_2$- in $R_6$ may be substituted by oxygen atom or -COO-, and any one of hydrogen atoms in $R_6$ may be substituted by alkyl, halogen, or nitro; $R_7$ represents $C_1$-$C_{20}$ linear or branched alkylene; $R_8$ represents hydrogen, halogen, nitro, $C_1$-$C_{20}$ linear or branched alkyl, $C_3$-$C_{20}$ cycloalkyl or substituted cycloalkyl, $C_2$-$C_{10}$ alkenyl, or $C_6$-$C_{24}$ aryl, wherein any one of -$CH_2$- in $R_7$ and $R_8$ may be substituted by oxygen atom or -COO-, and one or more hydrogen atoms in $R_7$ and $R_8$ are each independently substituted by alkyl, halogen, or nitro; preferably, $R_6$

represents $C_1$-$C_{20}$ linear or branched alkyl, $C_3$-$C_{20}$ cycloalkyl or substituted cycloalkyl, or $C_6$-$C_{24}$ aryl, wherein any one of -$CH_2$- in $R_6$ may be substituted by oxygen atom or -COO-, and any one of hydrogen atoms in $R_6$ may be substituted by alkyl, halogen, or nitro; $R_7$ represents $C_1$-$C_{10}$ linear or branched alkylene; $R_8$ represents hydrogen, halogen, nitro, $C_1$-$C_{10}$ linear or branched alkyl, $C_3$-$C_{10}$ cycloalkyl or substituted cycloalkyl, $C_2$-$C_{10}$ alkenyl, or $C_6$-$C_{12}$ aryl, wherein any one of -$CH_2$- in $R_7$ and $R_8$ may be substituted by oxygen atom or -COO-, and any one of hydrogen atoms in $R_7$ and $R_8$ may be substituted by alkyl, halogen, or nitro.

3. The epoxy-modified acrylic resin according to claim 1, **characterized in that** $R_1$ represents $C_1$-$C_{30}$ linear or branched n-valent alkyl, $C_2$-$C_{20}$ n-valent alkenyl, or $C_6$-$C_{30}$ n-valent aryl, any one of -$CH_2$- in $R_1$ may be substituted by oxygen atom, -COO-, or

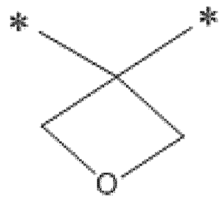

,

and any one of hydrogen atoms in $R_1$ may be substituted by alkyl, halogen, or nitro;
preferably, $R_1$ represents $C_1$-$C_{15}$ linear or branched n-valent alkyl, $C_2$-$C_{15}$ n-valent alkenyl, or $C_6$-$C_{18}$ n-valent aryl, any one of -$CH_2$- in $R_1$ may be substituted by oxygen atom, -COO-, or

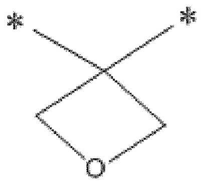

,

and any one of hydrogen atoms in $R_1$ may be substituted by alkyl, halogen, or nitro.

4. The epoxy-modified acrylic resin according to claim 1, **characterized in that** $R_2$ and $R_4$ independently represent hydrogen, halogen, nitro, $C_1$-$C_{20}$ linear or branched alkyl, $C_3$-$C_{20}$ cycloalkyl or substituted cycloalkyl, $C_2$-$C_{10}$ alkenyl, or $C_6$-$C_{24}$ aryl, respectively, any one of - $CH_2$- in $R_2$ and $R_4$ may be substituted by oxygen atom or -COO-, and any one of hydrogen atoms in $R_2$ and $R_4$ may be substituted by alkyl, halogen, or nitro;
preferably, $R_2$ and $R_4$ independently represent hydrogen, halogen, nitro, $C_1$-$C_{10}$ linear or branched alkyl, $C_3$-$C_{10}$ cycloalkyl or substituted cycloalkyl, $C_2$-$C_{10}$ alkenyl, or $C_6$-$C_{12}$ aryl, respectively, any one of -$CH_2$- in $R_2$ and $R_4$ may be substituted by oxygen atom or -COO-, and any one of hydrogen atoms in $R_2$ and $R_4$ may be substituted by alkyl, halogen, or nitro.

5. The epoxy-modified acrylic resin according to claim 1, **characterized in that** A represents $C_1$-$C_{15}$ linear or branched alkylene, any one of -$CH_2$- in A may be substituted by oxygen atom or - COO-, and any one of hydrogen atoms in A may be substituted by alkyl, halogen, or nitro.

6. The epoxy-modified acrylic resin according to claim 1, **characterized in that** M represents 3-valent $C_1$-$C_{15}$ linear or branched alkyl, any one of -$CH_2$- in M may be substituted by oxygen atom, -COO-, or

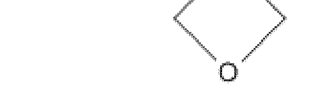

,

and any one of hydrogen atoms in M may be substituted by alkyl, halogen, or nitro.

**7.** The epoxy-modified acrylic resin according to claim 1, **characterized in that** Q represents $C_1$-$C_{15}$ linear or branched alkylene, any one of $-CH_2-$ in Q may be substituted by oxygen atom, - COO-, or

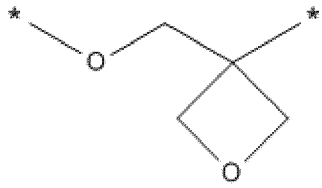

,

or any one of hydrogen atoms in Q may be substituted by alkyl, halogen, or nitro.

**8.** The epoxy-modified acrylic resin according to claim 1, **characterized in that** n is selected from an integer of 1 to 6.

**9.** The epoxy-modified acrylic resin according to claim 1, **characterized in that** the epoxy-modified acrylic resin is selected from one or more of the group consisting of:

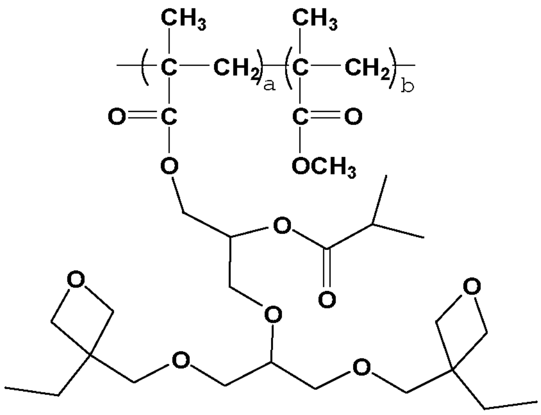

,

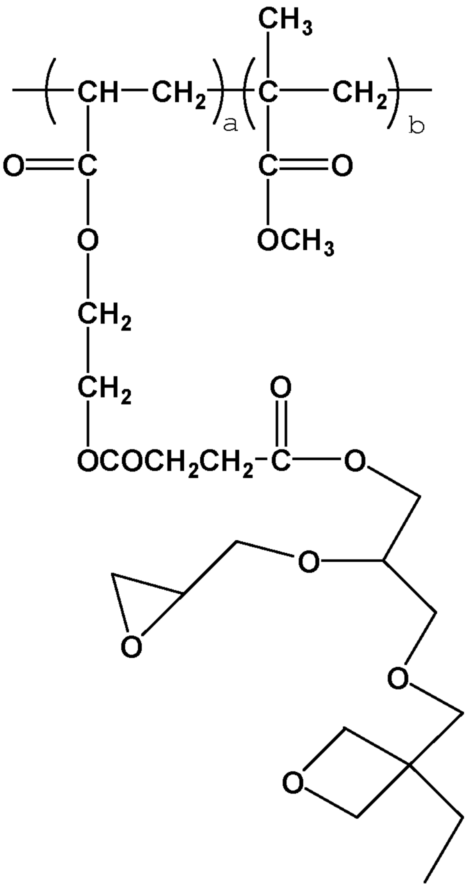

,

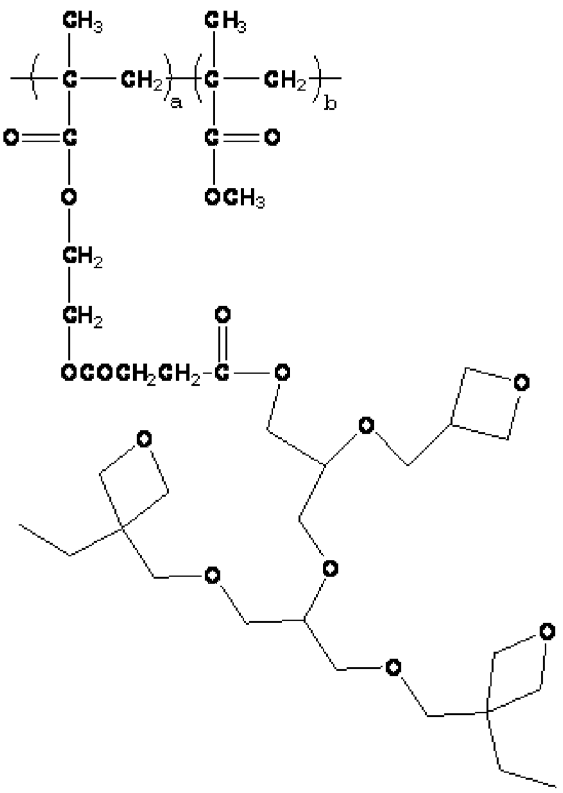

,

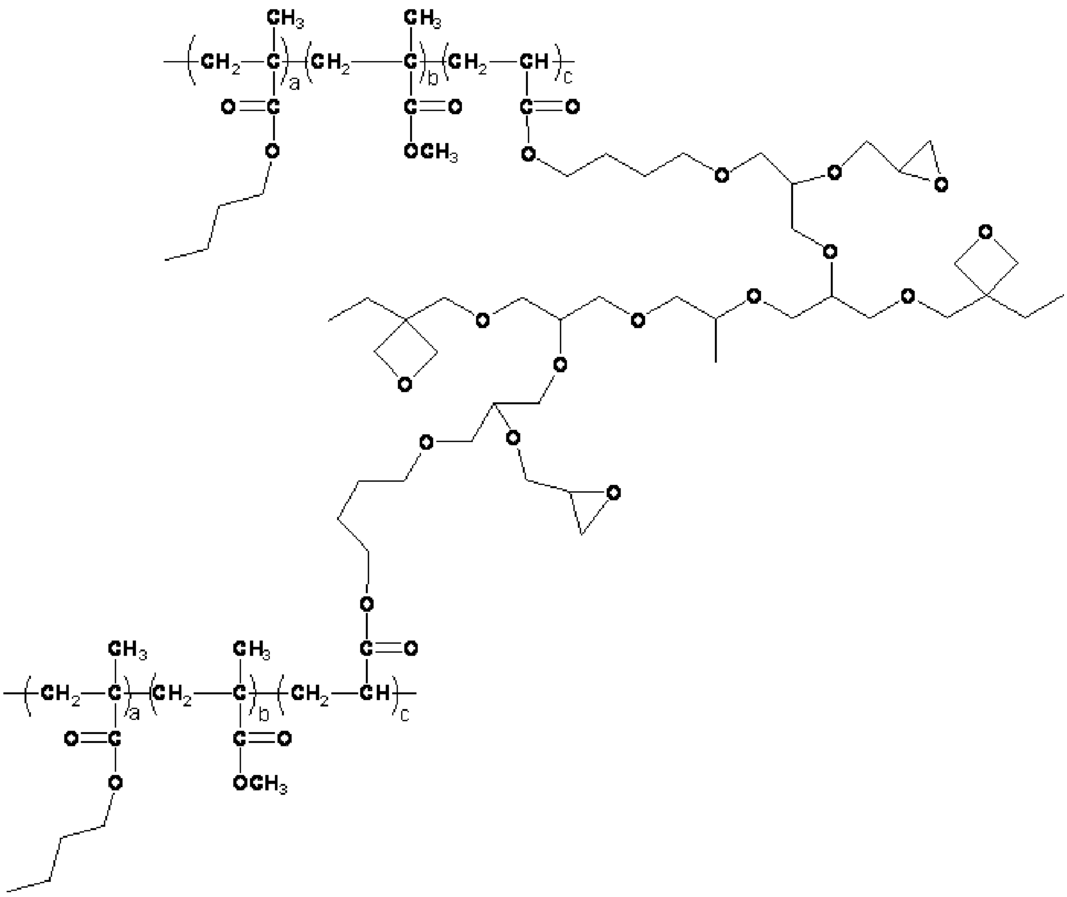

,

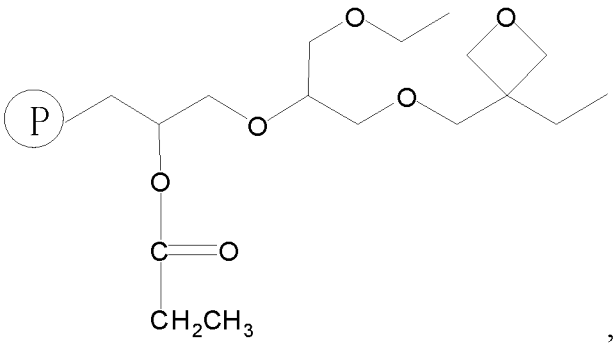

,

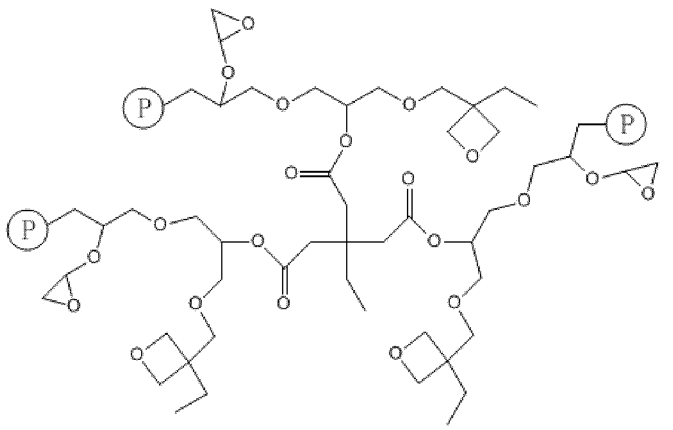

.

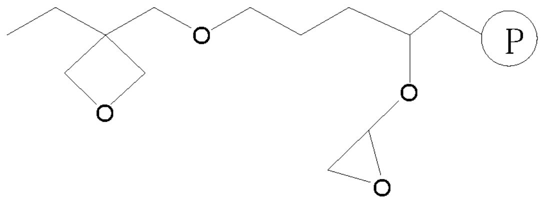

,

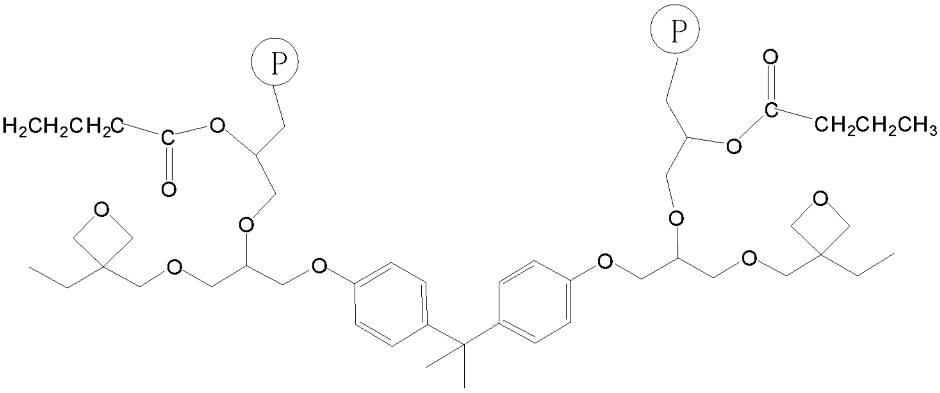

,

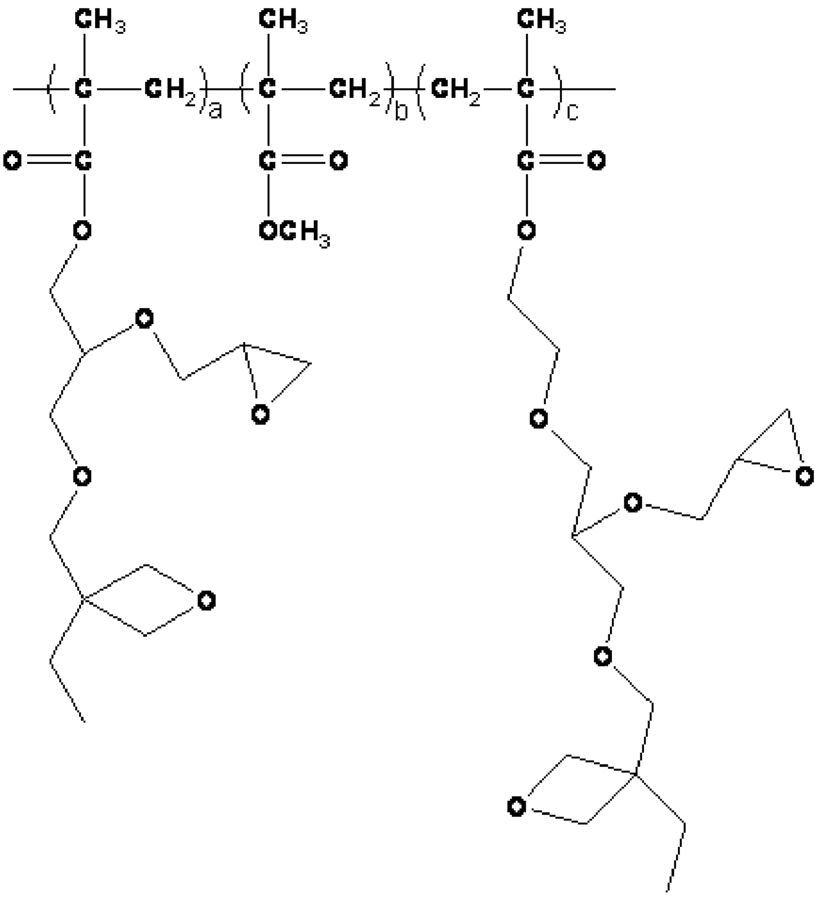

,

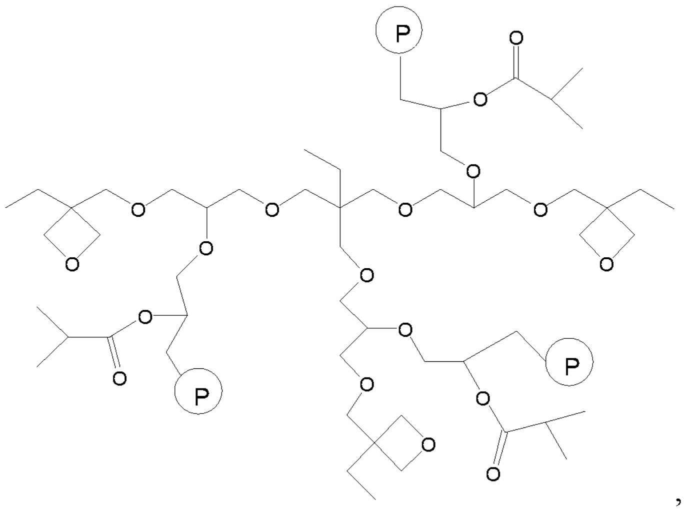

,

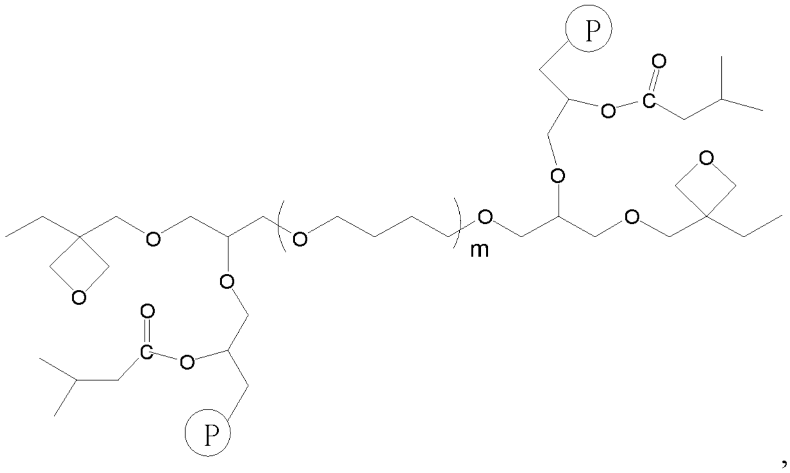

,

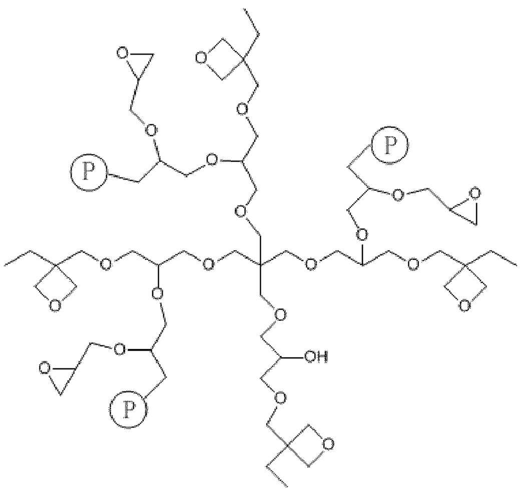

,

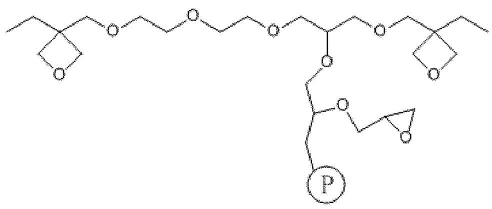

,

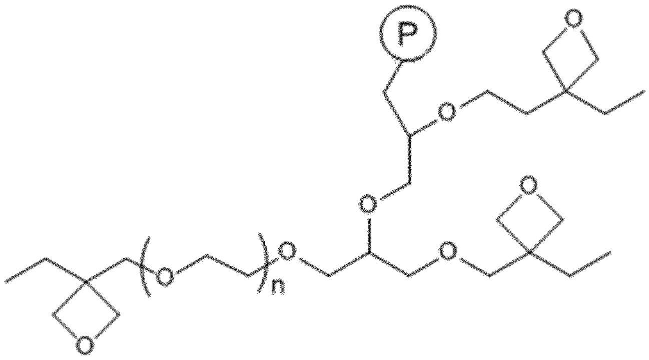

,

and

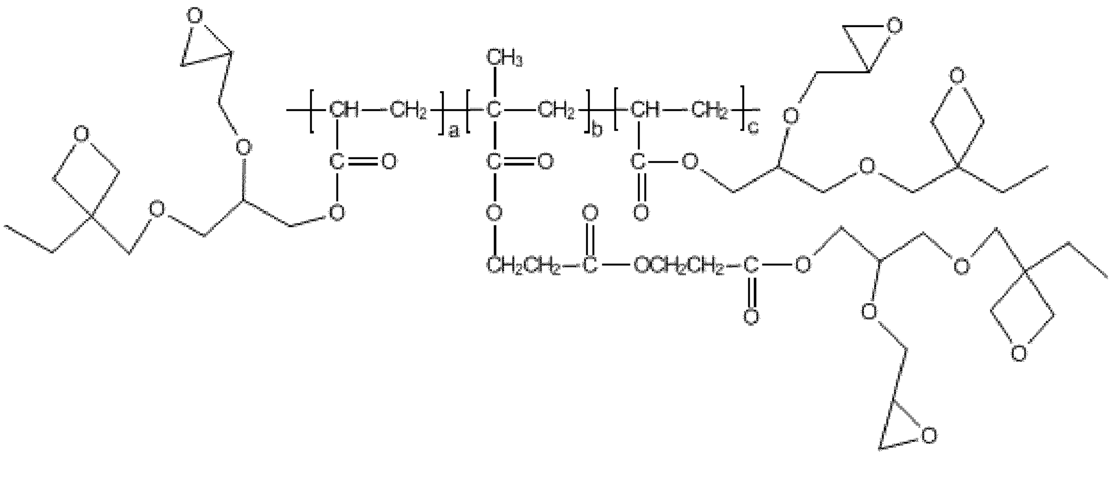

;

P is

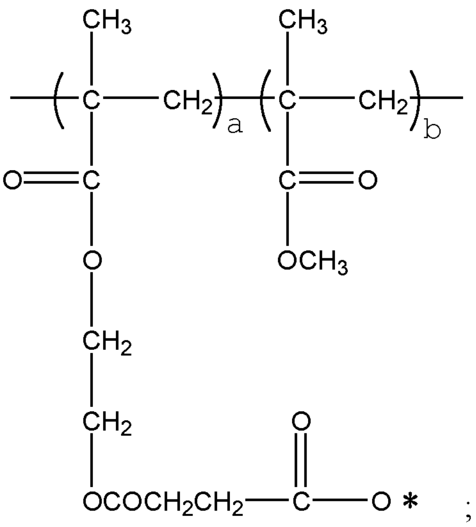

m is an integer of 1 to 30, preferably m is an integer of 1 to 10; n is an integer of 1 to 10, preferably n is an integer of 1 to 5; a, b, and c are independently an integer of 3 to 1000 respectively, preferably a, b, and c are independently an integer of 3 to 200 respectively.

**10.** A preparation method of the epoxy-modified acrylic resin according to anyone of claims 1 to 9, **characterized in that** the epoxy-modified acrylic resin is prepared by any one of the following processes:

Process 1: using an acrylic resin comprising the repeat unit represented by formula (IV) as a first starting material, and using a compound containing oxetanyl and epoxyethyl represented by formula (V) or (VI) as a second starting material, the preparation process including:
subjecting the carboxyl group of the first starting material and the second starting material to an epoxyethyl ring-opening reaction, to obtain a first intermediate product containing a hydroxyl group; and reacting the hydroxyl group of the first intermediate product with a first end-capping compound, to obtain the epoxy-modified acrylic resin, wherein the first end-capping compound is selected from anhydrides, acyl halides, compounds containing epoxyethyl, compounds containing oxetanyl, or compounds containing a double bond, and the formula (IV), the formula (V), and the formula (VI) are respectively as follows:

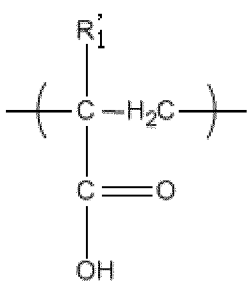

(IV)

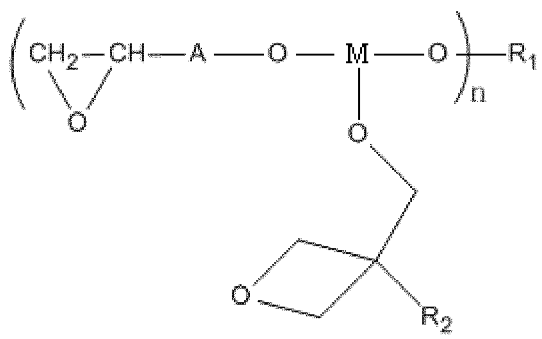

(V)

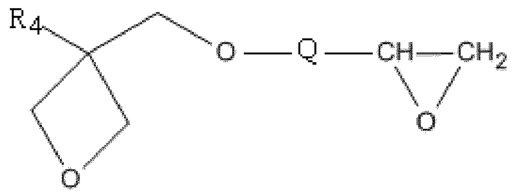

(VI)

wherein $R_1$, $R_2$, $R_4$, A, M, Q, and n have the same meanings as in general formula (I) and general formula (II), and $R_1$' is selected from hydrogen or methyl;

Process 2: using an acrylic resin comprising the repeat unit represented by formula (VII) as a third starting material, and using a compound containing oxetanyl and epoxyethyl represented by formula (V) or formula (VI) as a second starting material, the preparation process including:

subjecting the hydroxyl group of the third starting material and the second starting material to an epoxyethyl ring-opening reaction, to obtain a second intermediate product containing hydroxyl group; and reacting the hydroxyl group of the second intermediate product with a second end-capping compound, to obtain the epoxy-modified acrylic resin, wherein the second end-capping compound is selected from anhydrides, acyl halides, compounds containing epoxyethyl, compounds containing oxetanyl, or compounds containing a double bond, the formula (VII) is

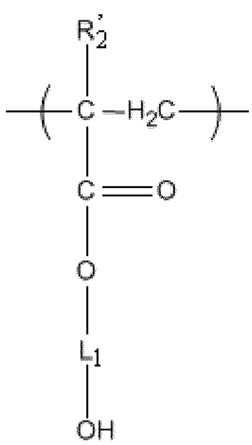

,

$R_2$' is selected from hydrogen or methyl, $L_1$ is selected from $C_2$-$C_{25}$ linear or branched alkylene, and -$CH_2$- in $L_1$ may be substituted by oxygen atom or ester group; preferably, $L_1$ is selected from $C_2$-$C_{10}$ linear or branched alkylene, and -$CH_2$- in $L_1$ may be substituted by oxygen atom or ester group;

Process 3: using an olefinic monomer represented by formula (VIII) as a fourth starting material, and using a compound containing oxetanyl and epoxyethyl represented by formula (V) or formula (VI) as a second starting material, the preparation process including:

subjecting the carboxyl group of the fourth starting material and the second starting material to an epoxyethyl ring-opening reaction, to obtain a third intermediate product containing a hydroxyl group; and reacting the hydroxyl group of the third intermediate product with a third end-capping compound, to obtain a third end-capped product; and copolymerizing the third end-capped product with a first olefinic monomer, to obtain the epoxy-modified acrylic resin; wherein the third end-capping compound is selected from anhydrides, acyl halides, compounds containing epoxyethyl, compounds containing oxetanyl, or compounds containing a double bond, the formula (VIII) is

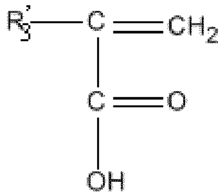

, '

wherein $R_3$' is selected from hydrogen or methyl;

Process 4: using an olefinic monomer represented by formula (X) as a fifth starting material, and using a compound containing oxetanyl and epoxyethyl represented by formula (V) or formula (VI) as a second starting material, the preparation process including:

subjecting the hydroxyl group of the fifth starting material and the second starting material to an epoxyethyl ring-opening reaction, to obtain a fourth intermediate product containing hydroxyl group; reacting the hydroxyl group of the fourth intermediate product with a fourth end-capping compound, to obtain a fourth end-capped product; and copolymerizing the fourth end-capped product with a second olefinic monomer, to obtain the epoxy-modified acrylic resin; wherein the fourth end-capping compound is selected from anhydrides, acyl halides, compounds containing epoxyethyl, compounds containing oxetanyl, or compounds containing a double bond, the formula (X) is

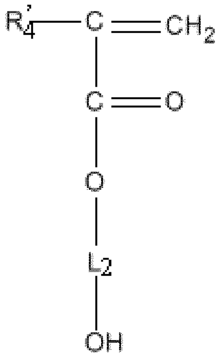

,

wherein $R_4$' is selected from hydrogen or methyl, $L_2$ is selected from $C_2$-$C_{25}$ linear or branched alkylene, and $-CH_2-$ in $L_2$ may be substituted by oxygen atom or ester group; preferably, $L_2$ is selected from $C_2$-$C_{10}$ linear or branched alkylene, and - $CH_2$- in $L_2$ may be substituted by oxygen atom or ester group;

Process 5: using a compound containing oxetanyl and epoxyethyl represented by formula (V) or formula (VI) as a second starting material, using an acrylic resin comprising the repeat unit represented by formula (VII) as a third starting material, and using a polycarboxylic acid or cyclic anhydride represented by formula (A) or formula (B) as a sixth starting material, the preparation process including:

subjecting the hydroxyl group of the third starting material and the sixth starting material to an esterification reaction, to obtain a first esterified product containing carboxyl group; subjecting the carboxyl group of the first esterified product and the second starting material to an epoxyethyl ring-opening reaction, to obtain a fifth intermediate product containing hydroxyl group; and reacting the hydroxyl group of the fifth intermediate product with a fifth end-capping compound, to obtain the epoxy-modified acrylic resin, wherein the fifth end-capping compound is selected from anhydrides, acyl halides, compounds containing epoxyethyl, compounds containing oxetanyl, or compounds containing a double bond,

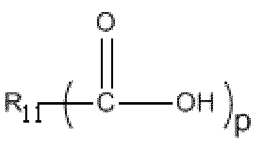 (A), 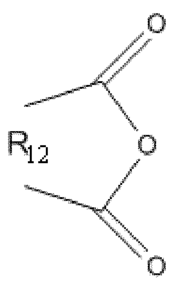 (B),

wherein $R_{11}$ is selected from $C_1$-$C_{20}$ linear or branched alkyl, $C_3$-$C_{20}$ cycloalkyl or substituted cycloalkyl, or $C_6$-$C_{24}$ aryl, and one or more hydrogen atoms in $R_{11}$ each may be independently substituted by a group selected from alkyl, halogen, or nitro; p is an integer of 2 to 6;
$R_{12}$ is selected from $C_1$-$C_{20}$ linear or branched alkyl, $C_3$-$C_{20}$ cycloalkyl or substituted cycloalkyl, or $C_6$-$C_{24}$ aryl, and one or more hydrogen atoms in $R_{12}$ each may be independently substituted by a group selected from carboxyl, alkyl, halogen, or nitro;

Process 6: using an olefinic monomer represented by formula (X) as a fifth starting material, using a polycarboxylic acid or cyclic anhydride represented by formula (A) or formula (B) as a sixth starting material, and using a compound containing oxetanyl and epoxyethyl represented by formula (V) or formula (VI) as a second starting material, the preparation process including:
subjecting the hydroxyl group of the fifth starting material and the sixth starting material to an esterification reaction, to obtain a second esterified product containing carboxyl group; subjecting the carboxyl group of the second esterified product and the second starting material to an epoxyethyl ring-opening reaction, to obtain a sixth intermediate product containing hydroxyl group; reacting the hydroxyl group of the sixth intermediate product with a sixth end-capping compound, to obtain the epoxy-modified acrylic resin, reacting with the sixth end-capping group to obtain a sixth end-capped product, wherein the sixth end-capping compound is selected from anhydrides, acyl halides, compounds containing epoxyethyl, compounds containing oxetanyl, or compounds containing a double bond; and copolymerizing the sixth end-capped product with a third olefinic monomer, to obtain the epoxy-modified acrylic resin;
Process 7: using 3-hydroxymethyl-3-ethyloxetanyl as a seventh starting material, and using methyl methacrylate as an eighth starting material, the preparation process including:
subjecting 3-hydroxymethyl-3-ethyloxetanyl as the seventh starting material and methyl methacrylate as the eighth starting material to a transesterification reaction, to obtain a transesterified product; and copolymerizing the transesterified product with a fourth olefinic monomer;
wherein in Processes 1 to 7, the first olefinic monomer, the second olefinic monomer, the third olefinic monomer, and the fourth olefinic monomer are the same or different.

**11.** The preparation method according to claim 10, **characterized in that** the second starting material is selected from one or more of the group consisting of:

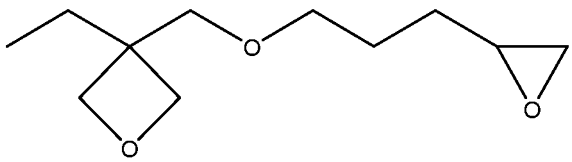

,

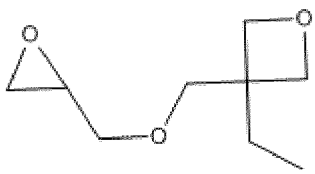

,

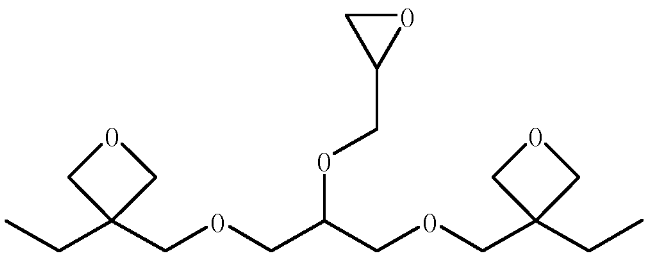

,

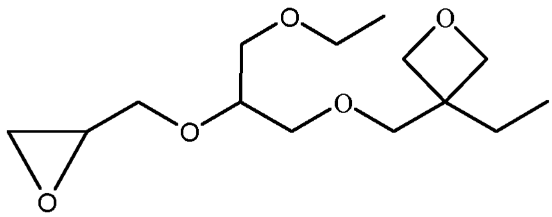

,

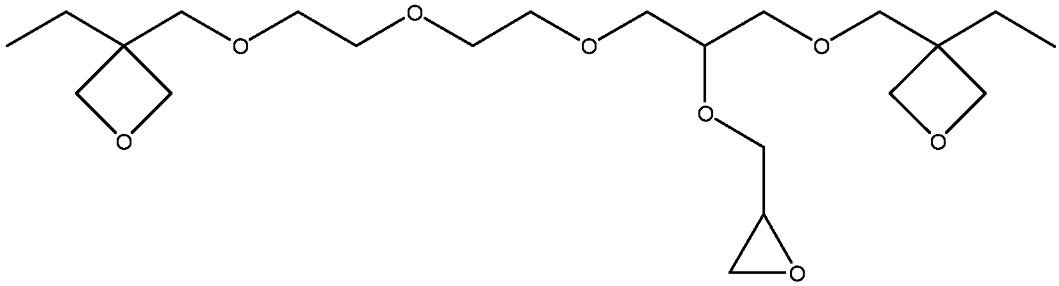

,

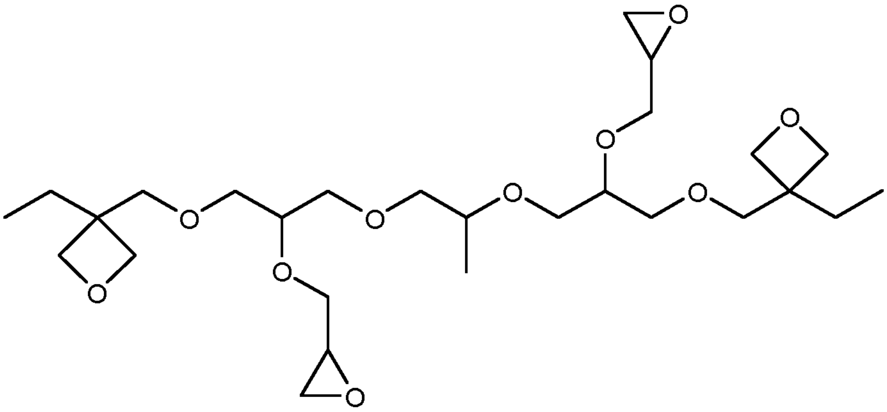

,

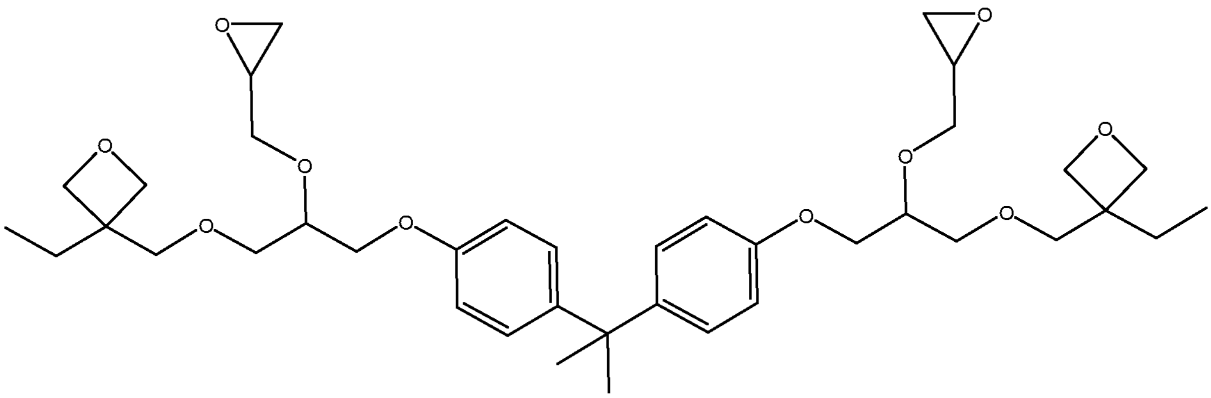

,

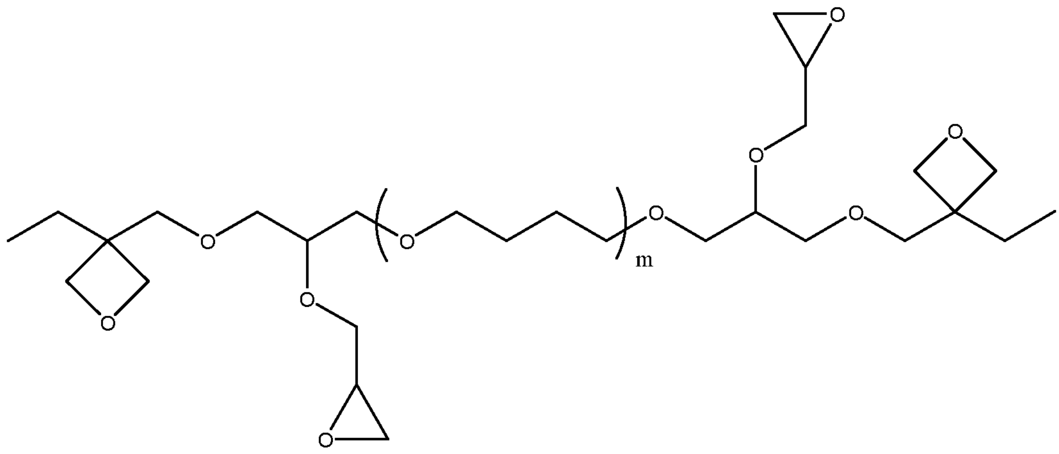

,

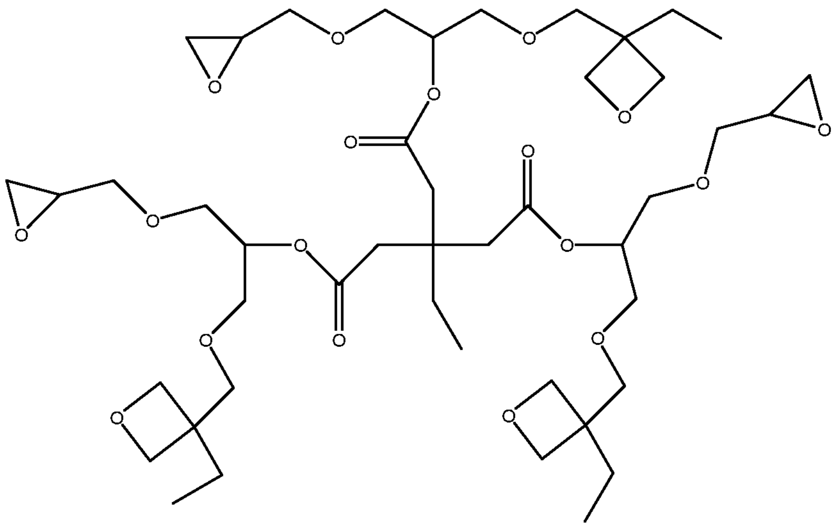

,

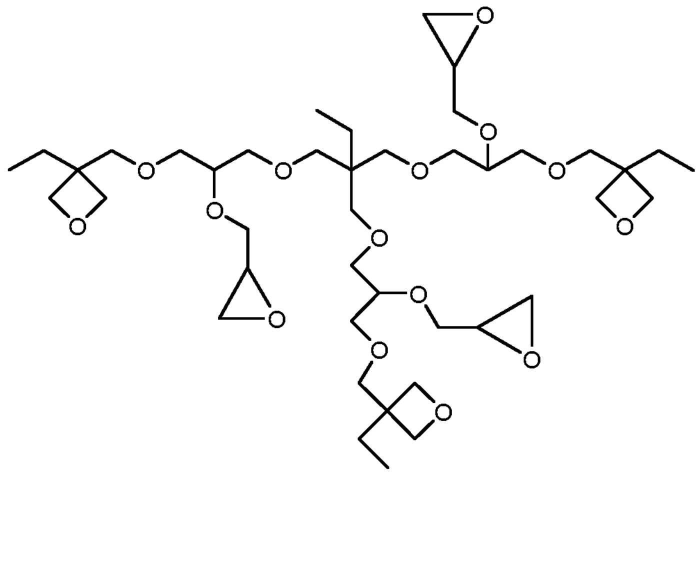

,

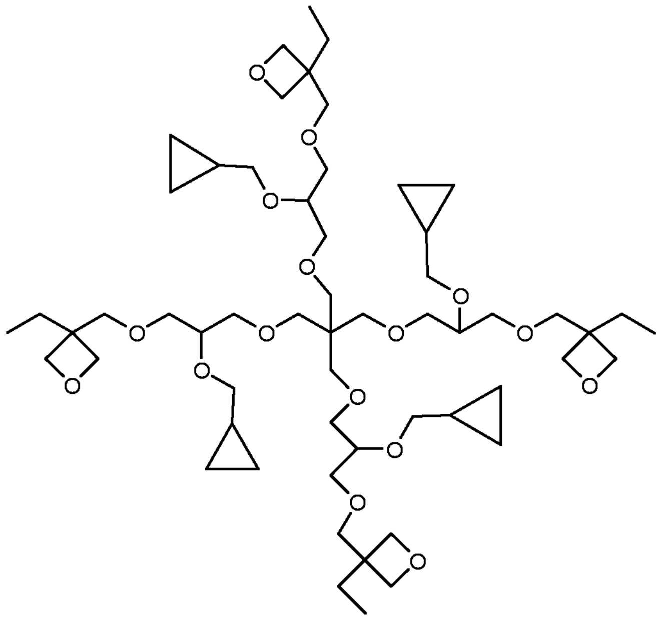

,

and

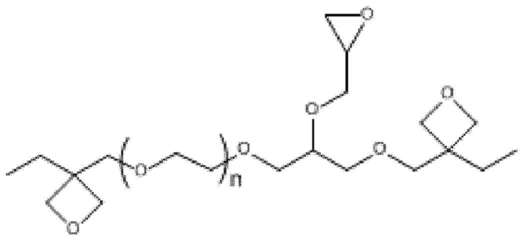

,

wherein m is an integer of 1 to 30, preferably m is an integer of 1 to 10; n is an integer of 1 to 10, preferably n is an integer of 1 to 5.

**12.** The preparation method according to claim 10, **characterized in that** the first end-capping compound, the second end-capping compound, the third end-capping compound, the fourth end-capping compound, the fifth end-capping compound, and the sixth end-capping compound are independently selected from compounds represented by formula (C) to formula (G), respectively

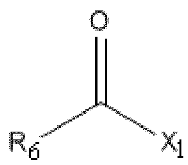

(C),

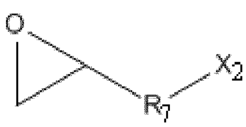

(D),

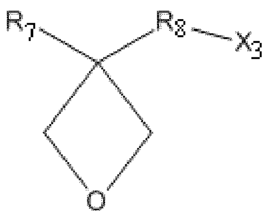

(E),

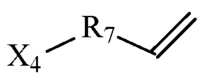

(F),

and

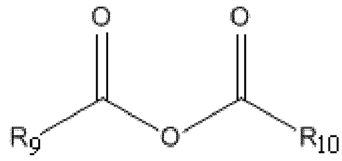

(G),

wherein $R_6$, $R_7$, and $R_8$ have the same meanings as in claims 1 to 8, $R_9$ and $R_{10}$ are independently selected from $C_1$-$C_6$ linear or branched alkyl, respectively, and $X_1$, $X_2$, $X_3$, and $X_4$ are independently selected from an halogen atom, respectively.

**13.** An energy-curable composition containing an epoxy-modified acrylic resin, **characterized in that** the composition comprises a resin, an initiator, and a curable epoxy-modified acrylic resin, and the epoxy-modified acrylic resin is the epoxy-modified acrylic resin of claims 1 to 9 or prepared by the preparation method of any one of claims 10 to 12.

**14.** The composition according to claim 13, **characterized in that** the content of the epoxy-modified acrylic resin is 10-80 wt%;
preferably, the content of the epoxy-modified acrylic resin is 30-75 wt%,
by the weight percentage of the energy-curable composition containing the epoxy-modified acrylic resin.

**15.** Use of the energy-curable composition containing the epoxy-modified acrylic resin of anyone of claims 13 or 14 in the field of energy-curing.

**16.** The use of the energy-curable composition containing epoxy-modified acrylic resin in the field of energy-curing according to claim 15, **characterized in that** the field of energy-curing is the field of inks, paints and adhesives.

## INTERNATIONAL SEARCH REPORT

International application No. **PCT/CN2019/094344**

**A. CLASSIFICATION OF SUBJECT MATTER**

C07D 305/00(2006.01)i; C08G 59/02(2006.01)i; C07D 305/06(2006.01)i; C08L 33/08(2006.01)i

According to International Patent Classification (IPC) or to both national classification and IPC

**B. FIELDS SEARCHED**

Minimum documentation searched (classification system followed by classification symbols)

C07D; C08G; C08L

Documentation searched other than minimum documentation to the extent that such documents are included in the fields searched

Electronic data base consulted during the international search (name of data base and, where practicable, search terms used)

WPI; CNABS; CNTXT; CNKI; STN; CNPAT: oxetane, acrylic resin, acrylate resin, graft+, trimethylene oxide, 接枝, 环氧氯丙烷, 氧杂环丁烷,丙烯酸, 三苯基膦, 氧杂环丁烷, 环氧改性, 支链, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 105938299 A (DONGWOO FINE-CHEM CO., LTD.) 14 September 2016 (2016-09-14) description, paragraphs 17-64 | 1, 13-16 |
| X | KR 101491975 B1 (HUNET PLUS CO.LTD) 11 February 2015 (2015-02-11) claims 1 and 10 | 1, 13-16 |
| X | KR 20170112012 A (DONGWOO FINE-CHEM CO.LTD) 12 October 2017 (2017-10-12) claims 1-5 | 1, 13-16 |
| A | US 2013164679 A1 (SAN EI KAGAKU CO ET AL.) 27 June 2013 (2013-06-27) description, pp. 2 and 3 | 1-16 |
| A | CN 109400551 A (CHANGZHOU TRONLY ADVANCED ELECTRONIC MAT CO LTD ET AL.) 01 March 2019 (2019-03-01) claim 1, and embodiments 1-11 | 1-16 |
| A | JP 2000168000 A (MITSUBISHI RAYON CO) 20 June 2000 (2000-06-20) embodiments 1-10 | 1-16 |
| A | US 2007015845 A1 (PENTRON CLINICAL TECHNOLOGIES) 18 January 2007 (2007-01-18) description, pp. 2 and 3 | 1-16 |

☐ Further documents are listed in the continuation of Box C.　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 September 2019** | **22 October 2019** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT
**Information on patent family members**

International application No.
**PCT/CN2019/094344**

| Patent document cited in search report | Publication date (day/month/year) | Patent family member(s) | Publication date (day/month/year) |
|---|---|---|---|
| CN 105938299 A | 14 September 2016 | JP 2016164662 A | 08 September 2016 |
| | | TW 201632991 A | 16 September 2016 |
| | | KR 20160107951 A | 19 September 2016 |
| KR 101491975 B1 | 11 February 2015 | None | |
| KR 20170112012 A | 12 October 2017 | None | |
| US 2013164679 A1 | 27 June 2013 | US 9201299 B2 | 01 December 2015 |
| CN 109400551 A | 01 March 2019 | WO 2019033982 A1 | 21 February 2019 |
| JP 2000168000 A | 20 June 2000 | None | |
| US 2007015845 A1 | 18 January 2007 | None | |

Form PCT/ISA/210 (patent family annex) (January 2015)